(19) Europäisches Patentamt
European Patent Office
Office européen des brevets

(11) EP 4 219 685 A1

(12) EUROPEAN PATENT APPLICATION

(43) Date of publication:
**02.08.2023 Bulletin 2023/31**

(21) Application number: **22305110.3**

(22) Date of filing: **31.01.2022**

(51) International Patent Classification (IPC):
***C12N 5/0735*** *(2010.01)* *C12M 3/00* *(2006.01)*

(52) Cooperative Patent Classification (CPC):
**C12N 5/0606; C12M 23/16; C12M 25/02;**
C12N 2501/235; C12N 2501/40; C12N 2501/70;
C12N 2501/72; C12N 2513/00; C12N 2535/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **INSTITUT PASTEUR**
  **75724 Paris Cedex 15 (FR)**
- **Ecole Polytechnique**
  **91128 Palaiseau Cedex (FR)**
- **INSTITUT NATIONAL DE LA SANTE ET DE LA RECHERCHE MEDICALE**
  **75654 Paris Cedex 13 (FR)**

(72) Inventors:
- **BAROUD, Charles**
  **75724 PARIS CEDEX 15 (FR)**
- **COSSEC, Jack Christophe**
  **75724 PARIS CEDEX 15 (FR)**
- **DEJEAN, Anne**
  **75724 PARIS CEDEX 15 (FR)**
- **SART, Sébastien**
  **75724 PARIS CEDEX 15 (FR)**
- **TRABOULSI, Tatiana**
  **75724 PARIS CEDEX 15 (FR)**

(74) Representative: **Ernest Gutmann - Yves Plasseraud S.A.S.**
**66, rue de la Chaussée d'Antin**
**75009 Paris (FR)**

# (54) IN VITRO GENERATION OF ORGANIZED 3D CELL STRUCTURES INCLUDING HEAD-TRUNK EMBRYO-LIKE STRUCTURES, USING EPIGENETIC REMODELING FACTORS - MICROFLUIDIC PLATFORM SUITABLE FOR THEIR GENERATION

(57) The invention relates to *in vitro* generation of organized 3D cell structures recapitulating various degrees of early organogenesis, including head-trunk embryo-like structures, using epigenetic remodeling factors. The invention relates in particular to methods of obtaining such organized 3D cell structures from mammalian cells, and to devices, in particular microfluidic platform, to perform such methods.

The invention also concerns the use of the thus obtained 3D cell structures in applications of molecule screening, developmental testing, production of physiologically active substances and models for therapeutic investigation or use.

EP 4 219 685 A1

## Description

The invention relates to *in vitro* generation of organized 3D cell structures recapitulating various degrees of early organogenesis, including head-trunk embryo-like structures, using epigenetic remodeling factors. The invention relates in particular to methods of obtaining such organized 3D cell structures from mammalian cells, in particular non-human mammalian cells and to devices, in particular microfluidic platform, to perform such methods.

The invention also concerns the use of the thus obtained 3D cell structures in applications of molecule screening, developmental testing, production of physiologically active substances and models for therapeutic investigation or use.

*In vitro* recapitulation of early mammalian embryogenesis is a very active field of research[1-13]. Two main strategies have been developed to build mouse or human embryo-like structures (ELS), either by combining embryonic and extraembryonic stem cells[1-3], which spontaneously self-organize to mimic post-implantation embryos, or by challenging embryonic stem cells (ESCs) with a pulse of Wingless-type integration site protein (WNT) agonist to recapitulate early organogenesis[4-8]. However, a large portion of the central nervous system is missing from these structures, particularly brain derivatives.

By contrast, the inventors have been able to design methods suitable to prepare 3D cell structures that may enable to obtain various degrees of early organogenesis, until elongated ELS characterized by a head-to-tail body axis with mid-hindbrain neuronal cell types as well as Schwann cell precursors, concomitant with somitogenesis.

The inventors have also shown that morphogen-free protocols can be designed in order to prepare organized 3D cell structures and the designed methods provide a step forward in exploring neural networks in embryos, showing that *in vitro* multicellular self-organization recapitulating early developmental organogenesis can be achieved using epigenetic remodeling factors. Among epigenetic remodeling factors, the inventors have addressed the effects of modulating the small ubiquitin-like modifier (SUMO) signaling pathway on pluripotent or multipotent vertebrates cell fate, in particular mammalian cell fate.

As is known in the art, the dynamic post-translational modification by small ubiquitin-like modifier (SUMO) predominantly targets chromatin-associated proteins[14-16], granting SUMOylation the ability to broadly affect the chromatin landscape, and modulate large transcriptional programs[17-20]. Recently, SUMO has been implicated in safeguarding cell fate, both *in vivo* and *in vitro,* by stabilizing substrate complexes on key cell identity genes[21-25]. Notably, suppressing SUMOylation is sufficient to promote conversion of mouse ESCs to two-cell-stage-like (2C-L) cells[23]. The inventors hypothesized that sequential waves of hypoSUMOylation in ESCs would further expand cell diversity.

The methods of the invention stem from the determination by the inventors that repeated transient inhibition of small ubiquitin-like modifier (SUMO) conjugation (so-called hypoSUMOylation) in mouse ESCs was suitable and sufficient to form organized 3D cell structures encompassing characterized epiblast-containing spheroids. These epiblast-like cells of spheroids were then capable of undergoing lineage-specific differentiation into all three embryonic germ layers when cultured in different culture formats including 2D plates or 3D microfluidics, especially in an optimized droplet-microfluidic platform. Mechanistically, the steps (i.e. rounds) of hypoSUMOylation gradually increased the global level of DNA methylation, which in turn repressed transcription of *Nanog* and other pluripotency-associated genes, facilitating cell fate determination.

The invention accordingly relates to methods of *in vitro* preparing organized 3D cell structures of vertebrate cells, advantageously mammalian cells wherein the organized 3D cell structures achieve various levels of early stage developmental events of mammalian embryos, in particular development stages taking place during gastrulation or early organogenesis. In an aspect of the invention, the prepared organized 3D cell structures recapitulate development until a spheroid structure is obtained. In an aspect of the invention, the prepared organized 3D cell structures recapitulate development until a gastruloid structure is obtained. In another particular aspect of the invention, diversification of cell types recapitulates early organogenesis.

In a further aspect the invention relates to a specific microfluidic platform, including a device which has been uniquely designed for pluripotent stem cells culture. This device and format of culture have proved to promote the generation of the organized 3D cell structures.

Beside reducing the volume of culture, the microfluidic platform of the invention provides a unique complex microenvironment generated by a controlled level of biochemical and mechanical confinement (due to accumulation of secreted molecules and unique cell/interface interactions), e.g. for the generation of late gastruloids and embryoids. These microenvironmental conditions may be also favorable for the maturation of other type of organoids derived from pluripotent stem cells (PSCs).

In a particular aspect, the invention relates to methods of *in vitro* preparing organized 3D cell structures of non-human mammalian cells, in particular rodents, especially mice, in particular laboratory mammalian animals. In a particular aspect, the invention relates to methods of *in vitro* preparing organized 3D cell structures of mammalian cells that are human cells,

The invention accordingly relates to a method of *in vitro* preparing organized 3D cell structures of mammalian cells wherein the method comprises:

i. Providing a population of pluripotent or multipotent vertebrate cells, in particular mammalian cells, in particular rodent cells, especially mouse cells in a first culture medium suitable for ESC culture and maintenance of pluripotent state,
ii. On the cells in the first culture medium of i., performing two steps of hypoSUMOylation treatment with an agent inhibiting small ubiquitin-like modifier (SUMO) conjugation (*SUMO inhibitor*),

wherein the second step of hypoSUMOylation treatment is separated in time from the first step of hypoSUMOylation treatment by at least 3 days, in particular by less than 20 days, in particular by 3 to 15 days or 5 to 15 days and more particularly by 3 to 5 days and
wherein each step of hypoSUMOylation treatment with the SUMO inhibitor is conducted for not more than 48h, preferably is conducted for 48h, and optionally recovering cells obtained after one or two steps of hypoSUMOylation treatment,

iii. Culturing the cells obtained after the second hypoSUMOylation step of treatment with the SUMO inhibitor according to ii. in a second culture medium suitable for ESC culture and differentiation of cells, wherein the first and the second culture medium have different composition,
iv. Optionally repeating at least once the step of hypoSUMOylation treatment with the SUMO inhibitor, wherein each repeat step of hypoSUMOylation treatment is carried out as in ii. and is performed separated in time from the immediately previous one as in ii.,
v. Recovering spheroids, wherein the spheroids are composed of at least three 3D self-assembled cell types encompassing from the center to the periphery of the spheroids embryonic stem-like cells (ES-like cells), forming the core of the spheroid on a monolayer of epiblast-like cells (EPI-L cells) and surrounded by extraembryonic endoderm cells (XEN-like cells) and in particular wherein the cell types in the spheroids lack pluripotency.

In a particular embodiment, the method is carried out when in step i. a homogeneous population of pluripotent or multipotent cells is provided. In an embodiment, the method is carried out when in step i. a homogeneous population of pluripotent or multipotent non-human mammalian cells, especially non-human embryonic stem cells is provided. In an embodiment, the method is carried out when in step i. a homogeneous population of pluripotent or multipotent human mammalian cells, especially human embryonic stem cells is provided

The expression *"organized 3D cell structure"* relates to cells that self-organize as 3D aggregates and recapitulate early development stages of animal tissues. Developmental stages may include differentiation and maturation of the pluripotent or multipotent cells provided to perform the methods of the invention. The organized 3D cell structures reflect dynamic spatiotemporal development *in vitro* of the originally provided population of pluripotent or multipotent cells while performing the methods of the invention. In a particular embodiment, the cell population initially provided is a homogenous population of cells such as a single population of pluripotent or multipotent cells while performing the methods of the invention. The term « homogenous » has the common meaning in the art and especially refers to a population of cells having similarities in their proliferation (also disclosed as division progression or self-renewal capacity) and differentiation outcome. The organized 3D cell structures in particular exhibit multiple cell types providing cell clusters that may be organized as discrete layers, in particular multiple germ layers. A cell cluster is a set of cells sharing a similar transcriptomic profile and that may be detected together in scRNA seq analysis.

The organized 3D cell structure may be said to be self-assembled. The expression "self-assembled" as disclosed herein relates to how the organized 3D cell structure is made when carrying out the methods of the invention.

According to various embodiments of the invention, organized 3D cell structures according to the invention may accordingly have achieved various stages of development and may be recovered as spheroids, or as polarized structures showing anterior-posterior polarization, or elongated axed structures such as anterior-posterior body axed structures (also designated as head-to-tail body axis), including in particular elongating-multilineages-organized (EMLO) structures such as gastruloids or embryoids. According to a particular embodiment, these organized 3D cell structures are non-human mammalian structures. According to a particular embodiment, these organized 3D cell structures have been obtained without the use of embryo. According to a particular embodiment, these organized 3D cell structures lack cell totipotency or cell pluripotency. According to a particular embodiment, these organized 3D cell structures lack certain types of cells such as 2C-like cells. According to an embodiment, when the organized 3D cell structures are gastruloids according to the invention, they lack Primordial Germ Cells and/or trophectoderm. According to an embodiment, when the organized 3D cell structures are embryoids according to the invention, they lack at least one of the following types of cells: notochord, forebrain, sclerotome, floor plate organizer, extra-embryonic annexes, blood cells and/or allantois.

The expressions *"pluripotent cells" or "multipotent cells"* relate to unspecialized cells with self-renewal capacity and potential to differentiate as all cell types of the body (pluripotent cells) or as multiple cell types of the body (multipotent cells). Examples of such cells are Embryonic Stem Cells (ESC) or Adult Stem Cells. The expression *"homogenous population of pluripotent or multipotent cells"* relates to such cells originating essentially from a single source. In a

particular embodiment, the term « homogenous » has the common meaning in the art and refers to a population of cells having similarities in their proliferation (also disclosed as division progression or self-renewal capacity) and differentiation outcome

The population of pluripotent or multipotent cells or the homogeneous population of pluripotent or multipotent cells may accordingly be Embryonic stem cells, in particular mammalian embryonic stem cells. In a particular embodiment, the ESCs are non-human mammalian embryonic stem cells, for example rodent embryonic stem cells such as mouse or rat embryonic stem cells. Alternatively, the homogeneous population of pluripotent or multipotent cells may be induced pluripotent stem cells (iPSC). In a particular embodiment, when the starting population of cells is human cells, the cells are induced pluripotent stem cells (iPSC).

In a particular embodiment, the ESCs or the PSCs are human ESCs. In a particular embodiment, when a human embryo is used to provide human ESCs, the method for obtaining ESCs from the embryo does not comprise destructing the embryo. Methods for obtaining ESCs without destruction of embryos are known in the art and for example disclosed in Klimanskaya I. et al[52].

In a particular embodiment of the invention, the organized 3D cell structures of mammalian cells are prepared using rodent homogeneous population of pluripotent or multipotent cells, in particular mouse ESC (mESC).

Otherwise stated, the disclosure provided herein by reference to ESCs also applies to PSCs, in particular iPSCs. Accordingly, in the disclosed embodiments ESC(s) and PSC(s) terms may be interchangeably used.

The "hypoSUMOylation treatment" according to the invention, designates a treatment applied to the cell culture that targets the molecular signaling pathway of the Epigenetic remodeling factor that is small ubiquitin-like modifier enzyme (SUMO enzyme), more particularly targets the molecular signaling pathway of the SUMO E1 activating enzyme. More precisely, the hypoSUMOylation treatment prevents small ubiquitin-like modifier (SUMO) conjugation to target proteins, predominantly to chromatin-associated proteins and leads to global hypermethylation of DNA (that can be in particular detected on enhancers heterochromatin, intergenic regions...). According to the invention, hypoSUMOylation treatment is achieved using an agent known to inhibit small ubiquitin-like modifier (SUMO) (*SUMO inhibitor*), in particular a small-molecule inhibitor of SUMO E1 enzyme. Increase in DNA methylation due to hypoSUMOylation treatment may be detected by genome-wide methylome, bisulfite-sequencing or other known techniques. Other phenomena associated with the hypoSUMOylation treatment according to the invention are highlighted in the Examples, especially in relation to Figures 10f, 10g.

In a particular embodiment of the methods of the invention, the inhibitor of SUMOylation is a selective small-molecule inhibitor of SUMO E1 enzyme wherein the effect of hypoSUMOylation on DNA hypermethylation is partially mediated by Sall4 transcription factor known to regulate DNA methyltransferase Dnmt3a (Zhang . J. et al).

In a particular embodiment of the methods of the invention, the inhibitor of SUMOylation is a selective small-molecule inhibitor of SUMO E1 enzyme, in particular is ML-792. ML-792 (CAS No. : 1644342-14-2) is a molecule of the following formula (provided by Takeda) that is a selective inhibitor of SUMO-activating enzyme that interferes with the first step of the SUMOylation of proteins cascade catalyzed by the SUMO activating enzyme (SAE), SUMO E1. ML-792 belongs to adenosine sulfamate (AdoS) inhibitors of E1 enzymes and inhibits SUMO1 conjugation to target proteins that are known in the art as chromatin-associated proteins.

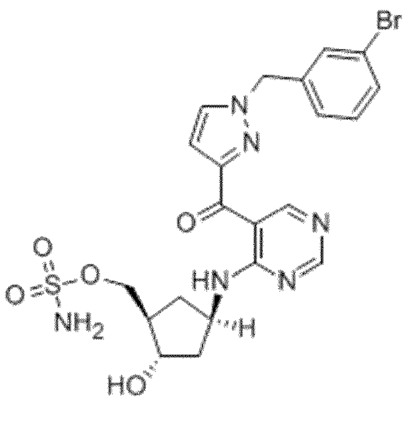

Chemical structure of ML-792

In a particular embodiment, ML-792 is used at a concentration of 1 to 2.5μM for 48h, more particularly a concentration of 2.5μM.

According to another embodiment, the inhibitor of SUMOylation is a selective small-molecule inhibitor of SUMO E1 enzyme that is Tak-981 (2',3',4'-trihydroxy-flavone,2-2,3,4-Trihydroxyphenyl)-4H-1-Benzopyran-4-one; CAS number 144707-18-6). In a particular embodiment 0.1 μM of Tak-981 may be used for treatment for 48h.

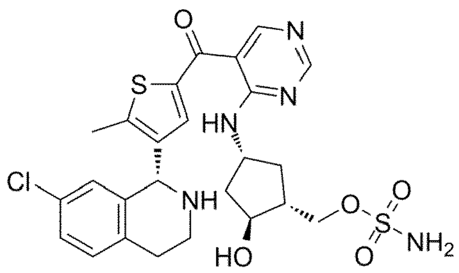

Chemical structure of Tak-981

A culture medium used in the methods of the invention, in particular the designated first culture medium and second culture medium, is a medium suitable for culture of ESC. A culture medium suitable for use according to the invention accordingly sustains growth, proliferation and/or differentiation of cells. Such mediums are well known in the art and are commercially available (Gibco, Milteyni Biotec etc). Their composition may comprise Serum (Knockout™ DMEM, ES Cell qualified FBS, GlutaMAX™ Supplement, MEM Non-Essential Amino Acids, Penicillin -Streptomycin 2-Mercaptoethanol (Gibco)) and additional substances such as Lif (Miltenyi Biotec 130-099-895) (Serum + Lif is suitable for use as first culture medium) or may comprise N2B27 (Neurobasal™ medium, advanced DMEM/F-12, N-2 supplement, B-27™ supplement, Bovine Albumin Fraction V, GlutaMAX™ Supplement (Gibco)), and additional substances such as antibiotics (penicillin -streptomycin) 2-mercaptoethanol and Lif (Miltenyi Biotec 130-099-895) (N2B27 + Lif is suitable for use as second culture medium). The first culture medium for use according to the methods of the invention enables maintenance of the pluripotent state of the ESCs. The second culture medium for use according to the methods of the invention enables cell differentiation.

In the methods of the invention, the hypoSUMOylation treatment comprises transient steps (or rounds or waves) of hypoSUMOylation in the ESCs provided to carry out the methods of the invention. Steps of hypoSUMOylation may be sequential or may be repeated. Accordingly, the methods of the invention comprise at least two steps of hypo-SUMOylation. In a particular embodiment, the methods of the invention comprise 3 steps of hypoSUMOylation. In a particular embodiment, the methods of the invention comprise exclusively (otherwise stated exactly (i.e. not less and not more)) two steps of hypoSUMOylation treatment. Repetition of the steps merely indicates that the number of steps of hypoSUMOylation treatment is more than 1, especially more than 2.

In a particular embodiment, the conditions (duration of the treatment and /or SUMO inhibitor) used to carry out the multiple steps of hypoSUMOylation treatment are independent for each step.

In a particular embodiment, a single SUMO inhibitor is used in at least the two initial steps of hypoSUMOylation treatment, in particular in all steps of hypoSUMOylation treatment. In a particular embodiment, the duration of the steps of hypoSUMOylation treatment is the same in at least the two initial steps of treatment, in particular is the same in all steps. In a particular embodiment, the SUMO inhibitor and the duration of the steps of hypoSUMOylation treatment are the same in the at least two initial steps, in particular are the same in all steps.

As disclosed herein, the steps of hypoSUMOylation must be separated in time in such a way that a subsequent step is separated from the immediately previous step by at least 3 days, in particular by less than 20 days or less than 15 days. The time between two consecutive steps of hypoSUMOylation treatment is calculated from the day after the final day of the treatment in the first of the considered consecutive steps to the day of the initiation of the treatment in the subsequent step. Calculation is schematically illustrated in the examples (Figure 1Aa, Figure 1(B)a). Preferably between two consecutive steps of hypoSUMOylation treatment cells recover ability, especially full ability to SUMOylation. In addition, when 2 steps of hypoSUMOylation treatment are performed, the time between steps and treatment conditions (duration and SUMO inhibitor) are provided to enable the gradual increase of the methylated CG fraction from Day 1 (D1) of the treatment to the last day of the final treatment step, in particular from D1 to D10 of the culture wherein D1 is the first day of culture and first day of the first step of treatment and D10 is the final day of the second step of treatment. The methylated CG fraction may be observed by genome-wide profiling of the cells. The inventors have also observed that SUMOylation recovery between two consecutive steps of hypoSUMOylation treatment does not revert the DNA methylation overload (Figure 10c).

In a particular embodiment, two consecutive steps of hypoSUMOylation treatment are separated in time by at least 5 days, in particular by 5 to 15 days. In a particular embodiment, the duration of each step of hypoSUMOylation treatment is at least 24 hours, in particular is from 24h to 72h more particularly at least 48h and especially is 48h. This applies in particular when two steps of hypoSUMOylation treatment are carried out. In a particular embodiment, exactly (i.e. not less and not more) two steps of hypoSUMOylation treatment are performed in the methods of the invention wherein each step has a duration of 48h and the steps are separated in time by 5 days. According to an embodiment

the duration between two steps of hypoSUMOylation treatment with the selective inhibitor of SUMO E1 enzyme enables full recovery of the SUMOylation capability of the cells between the two consecutive steps of hypoSUMOylation treatment. Full recovery of the SUMOylation capability may be assessed by visualization of conjugated substrates by Western Blot (Fig 1c) or by recovery of the SUMOylation peaks in ChIP-seq (Fig. 10 and Fig 13).

The culture medium used in the methods of the invention are suitable for culture of ESCs. In addition, the first culture medium must enable maintenance of the pluripotency of the cells during the culture step, including during SUMOylation treatment and enables cell expansion and growth. The second culture medium must enable the obtained culture cells to differentiate. Otherwise stated the second medium is a medium permissive for cell differentiation. Such mediums are available in the art and illustrated by examples in the present disclosure.

In a particular embodiment, these mediums are Lif (Leukeamia inhibitory factor) containing medium or medium containing another STAT3 activator.

In a particular embodiment, the basal medium in the second medium is a serum-free medium such as N2B27 medium.

In a particular embodiment of the methods of the invention, the first ESC culture medium is a Serum + Lif culture medium and the second ESC culture medium is a N2B27 + Lif culture medium.

In another embodiment, the first ESC culture medium is serum + Lif medium with KSR (KnockOut™ Serum Replacement; Gibco #10828010) replacing ES Cell qualified FBS and the second culture medium is N2B27 + Lif culture medium.

In another embodiment, the first ESC culture medium is N2B27 + Lif + 2i (PD0325901 and CHIR99021) medium (where 2i designates two inhibitory factors: dual inhibition of extracellular signal-regulated protein kinases 1 / 2 pathway and glycogen synthase kinase 3 beta) and the second medium is N2B27 + Lif medium.

In a particular embodiment of the methods of the invention, especially when using the above specified culture mediums, the inhibitor of SUMOylation is a selective small-molecule inhibitor of SUMO E1 enzyme, in particular is ML-792, in particular treatment with ML-792 is performed for 48h in each hypoSUMOylation treatment step.

In a particular embodiment of the methods of the invention, especially when using the above specified culture mediums, and the inhibitor of SUMOylation ML-792, in particular when treatment with ML-792 is performed for 48h in each hypoSUMOylation treatment step, two consecutive steps of hypoSUMOylation treatment are separated in time by at least 5 days, in particular by less than 15 days, more particularly the time between two consecutive steps of hypo-SUMOylation treatment is 5 days.

In a particular embodiment of the methods of the invention, especially when using the above specified culture mediums, when the inhibitor of SUMOylation is the selective small-molecule inhibitor of SUMO E1 enzyme Tak-981, the treatment with 0.1$\mu$M of Tak-981 is performed for 48h in each hypoSUMOylation treatment step, and two consecutive steps of hypoSUMOylation treatment are performed, said steps being separated in time by 6 days.

According to a particular embodiment of the methods of the invention, the duration of one step of hypoSUMOylation treatment is independent of the duration of the other step(s) of hypoSUMOylation treatment.

According to an embodiment of the invention, when a further step of hypoSUMOylation treatment is performed, the culture medium in the additional step is one of the above disclosed mediums, especially is identical in composition to the first culture medium in particular is Serum + Lif culture medium.

In a particular embodiment of the methods of the invention, especially when performing the above embodiments for the hypoSUMOylation treatment with ML-792, the spheroids are recovered on day 15, preferably on day 18 and up to day 50 of culture calculated from the first day of the first step of hypoSUMOylation treatment.

In a particular embodiment when Tak-981 is used, the spheroids are recovered as soon as on day 14, preferably on day 16 and up to day 50 of culture calculated from the first day of the first step of hypoSUMOylation treatment.

According to a particular embodiment of the methods of the invention, the first step of hypoSUMOylation treatment is started as soon as the cells are provided in the culture medium in step i. in other words, the first day of the culture coincide with the first day of the first hypoSUMOylation treatment step. In another embodiment, the culture in the ESC suitable culture medium is started before the first day of the first hypoSUMOylation treatment step.

According to the invention, increased DNA methylation may be obtained as soon as 8 days (D8) from the first day of first hypoSUMOylation treatment (when compared either to the cells at day 1 (D1) or to cells that remain untreated for hypoSUMOylation and otherwise cultured in the same conditions). Increased DNA methylation is accompanied by transcriptomic changes relating to expression of genetic markers. In these conditions ES-D8 cells are still able to self-renew and to differentiate into the 3 germ layers indicating maintenance of the pluripotency state, stable over cell passages. Organized 3D cell structures at a later stage, in particular after the second step of hypoSUMOylation treatment and as soon as spheroids are obtained lack cells with pluripotency state. The genetic markers of cells at these later stages are disclosed below and in the examples with respect to mammalian, especially mouse cells.

In a particular embodiment, cells may be retrieved at D8 after culture or initiation of the first step of hypo-SUMOylation treatment (i.e. the method may be used to yield cells at this intermediate stage of preparation of organized 3D cell structures) as disclosed in step ii. and in the above conditions and used for DNA methylation study, in particular

for screening of modulators of DNA methylation. In some embodiments, ES-D8 cells may be used to derive hypermethylated cell types such as hypermethylated neurons as cells for screening of modulators of DNA methylation.

ESCs yielded at D8 (D8_Hypermethylated_ESCs) may be those deposited at the CNCM (Collection Nationale de Cultures de Microorganismes, Institut Pasteur, 25-28, rue du Docteur Roux 75724 Paris Cedex 15 - France) under number I-5713 deposited on July 19, 2021. These cells qualify as hypermethylated ES cells. They may be frozen after being provided in a suspension fluid of 10% DMSO in ES Cell qualified FBS (Gibco).

When the method of the invention is used for the preparation of organized 3D cell structures that are spheroids, in particular spheroids of mammalian cells, the recovered spheroids may in particular be adherent spheroids contained on adherent plates. Spheroids may be characterized by their cell diversity, using well known techniques in the art (such as FACS or RT-qPCR), in particular using Single-cell RNA-seq characterization as disclosed in the Examples (Figures 1 and 2).

Spheroids are expected to be obtained from day 15 from the onset of the first step of hypoSUMOylation treatment (that may coincide with the first day or the culture of the cells in the culture medium suitable for ESCs) if at least 2 steps of hypoSUMOylation treatment have been carried out that have enabled increase in DNA methylation of the cells. The spheroids are composed of at least three distinct self-assembled cell types encompassing from the center to the periphery of the spheroids embryonic stem-like cells (ES-like cells or ES-L), forming the core of the spheroid on a monolayer of epiblast-like cells (EPI-L cells) and surrounded by extraembryonic endoderm cells (XEN-like cells). The spheroids may alternatively be described as a core of ES-like cells, resting on epiblast-like cells and surrounded by XEN-like cells. The harvested spheroids are especially adherent spheroids.

In a particular embodiment, the cells in the spheroids lack pluripotency as may be evidenced by their inability to form colonies in a clonogenic assay (such as alkaline-phosphatase positive colonies) and their failure to activate retinoic acid-responsive genes. The identity of the different cell types in the spheroids may be assessed by Single-cell RNA-seq characterization and immunostaining. These spheroids may be obtained as adherent cell culture on plates.

In a particular embodiment, the spheroids are yielded as an adherent organized 3D cell structure that contain over 55% in particular 55% to 65.0% ES-like cells, over 29 %, in particular 29.6 to 40% EPI-L cells and over 4.5 % in particular 5% XEN-like cells. In a particular embodiment, the spheroids may be characterized by cluster markers such as specific genes in the group of *Nanog, Dppa3, Ifitm3* for ESC-like cells, *Sox17, Gata4, Gata6* for XEN-like cells and *Pou3f1, Fgf5, Wnt3* for EPI-like cells, in particular *Dppa3, Sox17* and *Pou3f1* respectively. These markers may be detected by RT-qPCR. It is noted that ESC-like cells, by contrast to ESC express specific primordial germ cell markers including *Dppa3, Ifitm1* and *Ifitm3.*

In a particular embodiment, the spheroids may be additionally characterized by cluster markers expressed on cell membranes such as *Cd24a* for EPI-like cells, *Pdgfra (Cd140)* for XEN-like cells and *Cd31* (*Pecam1*) for ES-like cells. These markers may be detected by FACS.

In a particular embodiment the spheroids of the invention are an adherent organized 3D cell structure yielded at day 18 (D18) that contains three types of cells i.e., EPI-like cells related to the epiblast of the mouse embryo development (E4.5-E5.5), XEN-like cells related to the extraembryonic endoderm (E4.5-E6.5) and ESC-like expressing markers of ESC and markers of pluripotent state of PGC cells (Primordial Germ Cells). Such adherent organized 3D cell structure yielded at day 18 (designated D18_Spheroid_cells) has been deposited at the CNCM (Paris- France) under number I-5754 on September 28, 2021. These deposited D18_Spheroid_cells express the membrane markers *Cd24a* (for EPI-like cells), *Pdgfra* (*Cd140*) (for XEN-like cells ) and *Cd31* (*Pecam1*) (ESC-like) and the markers of specific sub-types *Pou3f1, Fgf5, Wnt3, Sox17, Gata4, Gata6,* and *Nanog, Dppa3, Ifitm3.* They may be stored in suspension fluid such as 10% DMSO in ES cell Qualified FBS (Gibco 16141-079).

In a particular embodiment of the invention, the spheroids may be used to model cancer progression and metastasis as cell adhesion molecules that may be expressed by the spheroids also significantly influence cancer progression and metastasis. In a particular use, the spheroids may accordingly be suitable for screening (in particular high throughput screening) of molecules capable of modulating, in particular inhibiting 3D shape establishment of cells.

The methods disclosed above are in particular carried out without steps of providing morphogen substance to the cells.

In a further aspect, the invention relates to a method as disclosed above that may give rise to spheroids, wherein the method comprises additional steps after recovering the spheroids wherein the steps comprise:

a) Transferring spheroid cells to a non-adherent microwell structure wherein the transfer is carried out after at least 14 days or after at least 18 days from initiation of the first hypoSUMOylation treatment step, and

b) culturing said cells to enable lineage-specific differentiation into embryonic germ layers,

wherein the culturing step is performed in the second culture medium and is continued until cell populations are obtained that comprise cell clusters of at least one, preferably all population(s) in the group of: primitive streak (cluster 4), definitive endoderm (cluster 5), neuromesodermal progenitors (NMPs cluster 6) and neuroepithelium (cluster 7), in particular wherein the culture is continued for at least 3 days to achieve elongated structures, in

particular is continued for 3 to 4 days,

c) recovering self-organized grown structures that are elongating-multilineages-organized (EMLO) gastruloids with an anterior-posterior body axis comprising discrete ES-L and EPI-L derived compartments that comprise anteriorly neural ectoderm lineages, posteriorly definitive endoderm and mesoderm lineages, and a primitive streak, wherein the neuroectoderm cell lineages are opposite to the primitive streak.

The non-adherent microwell plate may be an AggreWell™ plate (StemCells Technologies) or another system that enables aggregation of cells in controlled conditions allowing medium exchange during cell culture such as InSphero GravityPLUS Technology or a microfluidic device (microfluidic chip). In a particular embodiment, each microwell contains 70 to 130 cells, in particular 100 cells and the culture is carried out for 3 to 4 days, in particular 3 days,

The transfer of cells may be carried out as a seeding step of dissociated cells. The dissociated cells may be provided as a homogenous suspension of these cells. In a particular embodiment, the transfer of cells includes a step of passage of the spheroid culture and a step of obtaining a cell suspension used to seed dissociated cells in the wells of a plate.

In a particular embodiment, the transfer of spheroid cells is performed on day 18 and the recovery of self-organized grown elongated structures is performed on day 21 after the first step of hypoSUMOylation treatment has been initiated on the homogeneous population of pluripotent or multipotent mammalian cells.

As the recovered 3D- self-organized grown structures exhibit the 3 primary germ layers (ectoderm, endoderm, mesoderm) of the organism development they may be designated as gastruloids, i.e. a multicellular *in vitro* model of a gastrulating embryo obtained without using embryo.

According to a particular embodiment, gastruloids obtained according to the invention may be characterized by specific markers such as T for mesodermal transcription factor brachyury, *Cer1, Hoxb9, En1,* and additional markers such as *Nanog* and *Pou3f1.* These markers may be detected by RT-qPCR or immunostaining.

In a particular embodiment, the obtained gastruloids lack some types of cells such as primordial germ cell precursors, the notochord and they lack the extra-embryonic tissues that are found in the gastrulating embryo.

It is pointed out that the obtained gastruloids may exhibit different phenotypic outcome depending on the culture plate, in particular microwell plate or microfluidic chip used. In a particular embodiment of the invention, the gastruloids may be used to differentiate into multiple cell types with therapeutic potential. In another embodiment, the gastruloids may be used as an *in vitro* model to study or screen molecules or agents influencing development, in particular to study developmental toxicity of molecules. In another embodiment, the gastruloids may be used *in vitro* to produce molecules of therapeutic interests including growth factors, cytokines, morphogens, chemokines. Such molecules may in particular act as immunomodulating agents, rejuvenating agents, anti-aging agents...

In a further aspect, the invention relates to a method as disclosed above that may give rise to spheroids, wherein the method comprises additional steps after recovering the spheroids wherein the steps comprise:

a) Seeding dissociated cells obtained from the spheroid in drops such as drops of 4-10$\mu$l, in particular 6-7$\mu$l, in particular in a microfluidic device, in particular in a droplet microfluidics platform, wherein the transfer is carried out after at least 14 days or at least 18 days from initiation of the first hypoSUMOylation treatment, and

b) culturing said cells to enable lineage-specific differentiation into embryonic germ layers, wherein the culturing step is performed in the second culture medium and is continued until axial elongation of the grown structure is reached, in particular wherein the culture is continued for at least 4 days to achieve elongated structures, in particular is continued for 4 to 5 days until recovery of the self-organized grown structures showing elongation with an anterior-posterior body axis,

c) embedding the recovered self-organized grown structures showing elongation with an anterior-posterior body axis in Matrigel or in a matrix equivalent, in particular in 20% Matrigel and culturing to enable increasing elongation of the structure, in particular carrying out the culture in the second culture medium and Matrigel for at least 2 days, in particular 2 to 3 days,

d) recovering self-organized grown elongated structures that are elongating-multilineages-organized (EMLO) embryoids with an anterior-posterior body axis, wherein cell populations are obtained that comprise cell clusters of at least one, preferably all population(s) are obtained in the group of: endoderm in particular gut endoderm, mesoderm, neuroectoderm, in particular cells of at least one, said elongated structures comprising preferably all cell type(s) in the group of: ES-L, EPI-L, primitive streak, NMPs, presomitic mesoderm, somitic mesoderm, pharyngeal mesoderm, definitive endoderm, radial glia, dermomyotome, mesenchyme, craniofacial mesenchyme, endothelium, cardiomyocytes, spinal cord, midbrain-hindbrain, Schwann cell precursors and neurons, in particular recovering self-organized grown elongated structures comprising Schwann cell precursors.

The step of seeding dissociated cells may be performed after a step of passage of the spheroid culture and a step of obtaining a cell suspension used to seed dissociated cells in the microfluidic device. In particular, the transfer of

the cells in the microfluidic device is carried out using droplets containing the cells (e.g. one droplet may comprise 60 to 300 cells, in particular 120 or 200 cells).

After the self-organized grown structures showing elongation with an anterior-posterior body axis such as gastruloid structures have been obtained in the microfluidic device, these structures may be recovered for seeding into a plate suitable for embedding the structures in a solution of Matrigel (e.g. 20% Matrigel) or an equivalent matrix as disclosed herein contained into cell culture medium (such as N2B27 + Lif medium) for incubation to promote further elongation of the structures, especially to achieve embryo-like structures.

Alternatively, after the self-organized grown structures showing elongation with an anterior-posterior body axis such as gastruloid structures have been obtained in the microfluidic device, these structures contained in first droplets may be brought into contact with second droplets for fusion of the first and second droplets, wherein the second droplets contain Matrigel (e.g. 40% Matrigel) or an equivalent matrix as disclosed herein to yield fused drops, such as drops containing 20% Matrigel, that are allowed to gelify. These drops are suitable for further culture of the self-organized grown elongated structures. In some embodiments, the Matrigel or equivalent matrix is in a proportion of 10% to 40% of the contents of the drop resulting from the fusion. This method according to the invention enables the preparation using a microfluidic device, advantageously a droplet microfluidics platform as disclosed herein, and using a Matrigel matrix or an equivalent matrix of poly(ethylene glycol) (PEG) hydrogel, Geltrex (Thermo Fisher) GrowDex (UPM biomedicals) HyStem® hydrogels to obtain embryoid structures derived from ES or PS cells i.e., obtained without using embryo. The terms "drop(s)" or "droplet(s)" are used interchangeably in the present description. These terms may designate structures that may have a different size such as to disclose that fusion of droplets result in a larger structure of droplet that may be designated as a drop.

The steps of culture in the Matrigel or equivalent matrix as disclosed herein may be carried out for at least 2 days, in particular for 7 to 14 days.

Using the microfluidic platform, the inventors have found that after mechanical immobilization into anchors using hydrogel and performing oil-to-medium phase change, the device allows the application of controlled feeding/perfusion strategies (e.g. to allow periodic stimulation etc.) for further embryoid maturation (i.e. controlling fluid dynamics), in particular at a low Reynolds number, combined with *in situ* imaging which would not be currently achievable in regular 96-well plates or rotating bioreactor. In a particular embodiment, the embryoids are remarkable in that they comprise Schwann cell precursors, dermomyotome, spinal cord cell types.

In a particular embodiment, the obtained embryoids lack some types of cells such as cells or primordial germ cell precursors, forebrain tissue and/or the notochord and they lack the extra-embryonic tissues that is found in the embryo.

In a particular embodiment of the method of obtaining such embryoids, the transfer of spheroid cells is performed on day 18 (D18) and the recovery of self-organized grown elongated structures is performed on day 25 (D25) after the first step of hypoSUMOylation treatment has been initiated on the homogeneous population of pluripotent or multipotent mammalian cells.

According to a particular embodiment, embryoids obtained according to the invention may be characterized by specific markers such as at least one and up to all of *Uncx, En1, Tuj1, Pax2, Map2 Dmrt2, Cd93, Tnnt2, Rfx6, Sox10, Pax1, Pax9, Noto, Shh, Foxg1, Emx1* and *Vsx2.* According to a particular embodiment, at least cardiomyocytes marker *Tnnt2* is expressed. According to a particular embodiment, embryoids obtained according to the invention may be characterized by specific markers such as at least one and up to all of *Uncx, En1, Tuj1, Pax2, Map2 Dmrt2, Cd93, Tnnt2, Rfx6* and *Sox10*According to a particular embodiment, embryoids obtained according to the invention may be characterized by specific markers such as *Tnnt2, Noto, Shh, Foxg1 and Emx1.* According to a particular embodiment, embryoids obtained according to the invention may be characterized by specific markers such as *Tnnt2, Pax1, Pax9* and *Vsx2.*

In a particular embodiment of the invention, the embryoids may be used to differentiate into multiple cell types with therapeutic potential. In another embodiment, the embryoids may be used as an *in vitro* model to study or screen molecules or agents influencing development, in particular to study developmental toxicity of molecules. In another embodiment, the embryoids may be used *in vitro* to produce molecules of therapeutic interests including growth factors, cytokines, morphogens, chemokines. Such molecules may in particular act as immunomodulating agents, epigenetic drugs, rejuvenating agents, anti-aging agents...

The invention also relates to an organized 3D structure which is self-assembled into spheroid wherein the spheroid comprises mammalian cell clusters encompassing 3D-organized ES-like cells as a core resting on EPI-like cells and surrounded by XEN-like cells, in particular wherein the cell types lack pluripotency. In a particular embodiment, the mammalian cells in the organized 3D structure are not human cells or are not exclusively human cells. In a particular embodiment, the mammalian cells in the organized 3D structure comprise or consist of human cells or are not exclusively human cells.

In another embodiment, the invention relates to an organized 3D structure which is self-assembled into a structure of mammalian cells with Head-to-tail body axis which is:

- either an elongating-multilineages-organized (EMLO) gastruloid comprising discrete ES-L and EPI-L derived compartments that comprise anteriorly neural ectoderm lineages, posteriorly definitive endoderm and mesoderm lineages, and a primitive streak wherein the neuroectoderm cell lineages are opposite to the primitive streak or,

- an elongating-multilineages-organized (EMLO) embryoid comprising cell clusters of at least one, preferably all population(s) obtained from the group of: endoderm in particular gut endoderm, mesoderm and neuroectoderm, , said elongated structures comprising preferably all cell type(s) in the group of ES-L, EPI-L, primitive streak, NMPs, presomitic mesoderm, somitic mesoderm, pharyngeal mesoderm, definitive endoderm, radial glia, dermomyotome, mesenchyme, craniofacial mesenchyme, endothelium, cardiomyocytes, spinal cord, midbrain-hindbrain, Schwann cell precursors and neurons, in particular comprising Schwann cell precursors, wherein in a particular embodiment the mammalian cells are not human cells or are not exclusively human cells, in particular wherein the mammalian cells lack pluripotency. It is especially noted that in this structure the markers of emerging tissues (brain, heart, gut, dermomyotome, Schwann cell precursors, endothelium, spinal cord neuronal precursor subtypes) were expressed in spatially organized manner along the 3D structure's anterior-posterior axis.

In a particular embodiment these elongating-multilineages-organized (EMLO) embryoids have been obtained in a culture device that is suitable for drop adjustment and culture or in a microfluidic platform, as described herein.

In a particular aspect, the invention concerns also the use of a spheroid structure obtained according to the method of the invention, for screening of molecules, in particular for high throughput screening of molecules for assessing their activity on 2D to 3D transition of cell culture, in particular for assessing their capability to interfere with 2D to 3D transition. The screened molecules may be anti-cancer drug candidates.

The invention also relates to the use of elongating-multilineages-organized (EMLO) gastruloids obtained according to the method of the invention, or elongating-multilineages-organized (EMLO) embryoids obtained according to the method of the invention, in particular of non-human mammalian EMLO gastruloids or embryoids, (i) for screening of molecules, in particular for high throughput screening of molecules for assessing their activity on development in particular on developmental toxicity or (ii) for the production of growth factors, cytokines, morphogens, chemokines of interest, in particular for interest for therapeutic use or (iii) for the production of cells for therapeutic potential.

When the elongating-multilineages-organized (EMLO) gastruloids or the elongating-multilineages-organized (EMLO) embryoids are obtained according to the method of the invention using the microfluidic platform, the platform allows controlled droplet fusion for combinatorial screening or for temporally controlling hydrogel encapsulation.

Besides, elongating-multilineages-organized (EMLO) gastruloids or embryoids obtained according to the method of the invention provide structures with cellular complexity that is generally not achieved with specialized organoids. The structures obtained according to the invention, thus provide improvements in making available 3D-cell structures for therapeutic applications, in particular for those requiring long-term viability and extended maturation potential including elements of the angiogenesis and immune cell maturation. Accordingly, elongating-multilineages-organized (EMLO) gastruloids or embryoids obtained according to the method of the invention provides structures for cell-based therapy. In particular these structures may be of interest in providing transplantation material for neural tissue repair or therapy of autoimmune, cardiovascular, skeletal or liver diseases.

Elongating-multilineages-organized (EMLO) gastruloids may exhibit particularly interesting potential in regeneration therapy of neural cells damage or injury, in particular leading to neurodegenerative situation in animals or humans, such as Spinal Cord Injury (SCI), due to their capability to generate neural and endothelial lineages from ectodermal and mesodermal lineages respectively, without interference of more specialized cells.

Accordingly, elongating-multilineages-organized (EMLO) gastruloids or embryoids obtained according to the method of the invention provide structures for grafting or transplanting in an organism in need thereof, suitable to provide a combinatorial therapeutic strategy bringing both cell replacement and sourcing growth promoting molecules and more generally trophic support.

In a particular embodiment of the invention, use of the spheroid structure or use of the elongating-multilineages-organized (EMLO) structures obtained according to the methods of the invention may involve manipulation of the structures within immobilized droplets as disclosed herein. According to this aspect of the manipulation protocol, a drop containing the cell aggregates of the invention may be provided in the culture chamber and fused with a drop containing molecules to be assayed or more generally biomaterial (including agarose for immobilization, dyes). After the structures of the invention have been enabled to react with the molecules or biomaterial provided, they can be recovered for analysis, in particular by cytometry, RT-qPCR, single cell RNA-sequencing, immunostaining...

Culture of spheroid structure or of elongating-multilineages-organized (EMLO) structures in immobilized drops according to the invention provides the advantageous property that culture volumes may be reduced up to about 15 times without impacting aggregation kinetics, expansion degree and level of marker expression showing unaltered cell function with respect to culture on low adhesion 96-well plates. Volume reduction provides in particular the advantage of improving study of the role of para/autocrine signaling.

The invention also relates to a plurality of organized 3D cell structures as defined herein, wherein each of the plurality of organized 3D structures comprises cells having the same nuclear genome.

The invention also relates to a plurality of organized 3D cell structures as defined in the invention, wherein each of the plurality of organized 3D structures is provided in a culture medium.

The invention also relates to a culture or a storage means chosen from a plate of microwells, a collection of droplets, or a microfluidic device comprising cell traps wherein said each of said microwells, droplets or respectively cell traps is loaded with an organized 3D cell structure and the plate of microwells, collection of droplets, and respectively microfluidic device comprise a plurality of organized 3D cell structures according to the invention.

The invention also relates to a combination product comprising A) an organized 3D cell structure which is self-assembled into a spheroid according to the herein disclosed embodiments, and B) a homogeneous population of pluripotent or multipotent vertebrate cells, wherein the organized 3D cell structure of A) and the homogeneous population of B) both comprise cells having the same nuclear genome

The invention also concerns a method of providing a cellular therapy to a patient in need thereof, comprising (i) providing an organized 3D cell structure as defined herein, (ii) obtaining cells of one or more cell types from the organized 3D cell structure, and (iii) administering the cells of one or more cell types to the patient.

In a particular embodiment of this method the cells of one or more cell types of (ii) are cultured, passaged and/or differentiated before the administration to the patient.

The invention also relates to a method of providing a cellular therapy to a patient in need thereof, comprising (i) providing an organized 3D cell structure which is self-assembled into a structure of mammalian cells with Head-to-tail body axis according to the invention, (ii) obtaining cells of one or more cell types from the organized 3D structure, and (iii) administering the cells of one or more cell types to the patient.

In an embodiment of this method, the cells of one or more cell types of (ii) are cultured, passaged and/or differentiated before the administration to the patient.

In a further aspect, the invention relates to microfluidic device comprising a body having a thickness and comprising a bottom side and a top side facing each other, said bottom side being arranged at distance with a plate so as to define a channel for the flow of a fluid between at least one inlet and at least one outlet, said body comprising at least one trap extending along an axis of revolution with said trap comprising a first part and a second part extending along said axis of revolution, the first part being arranged, along said axis of revolution, between the second part and an opening of the trap that opens out at the bottom side in the channel, wherein the surface of a cross-section of the first part at the opening is greater than the surface of a cross-section of the second part and wherein the diameter of the opening is greater than or equal to twice the distance between the plate and said opening.

In the present invention, the axis of revolution is an axis around which the internal surface of the cavity is formed and forms in cross-section a closed contour.

The microfluidic device allows the trapping of a first droplet by capillarity in the first part of the cavity of a trap. The first droplet migrates to the second part of the cavity of said trap by buoyancy after a given time, thus liberating the first part of the cavity of the trap and allowing possibly but not necessarily the trapping of a second droplet in the same trap.

This proposed device is unique for immobilizing and manipulating liquid droplets with a volume comprised between 4 and 10 $\mu$L, while promoting the long-term culture and differentiation of pluripotent stem cells (PSCs). Another important aspect is this volume range has been characterized as the minimal volume enabling the aggregation and culture of PSCs in 3D.

According to the invention, the fact that the surface of a cross-section of the first part at the opening is greater than the surface of a cross-section of the second part, facilitates trapping of a droplet by gravity and allows the droplet to adopt a convex shape at its bottom tip when trapped in the second part. Compared to bottom of the convex shape at the bottom tip of the first droplet eases the sedimentation of the cells at the droplet bottom and a faster aggregation of the cells.

The diameter of the opening being greater than or equal to twice the distance between the plate and said opening, allow a trapping by capillarity by facilitating the release of the surface energy of a droplet.

The first part may comprise a convex annular wall having a peripheral free edge defining said opening of each cavity.

The channel may comprise a diffusion area for diffusion of the droplets connected to said at least one inlet and a collect area connected to said at least one outlet. Those area may have a triangular shape. Said at least one inlet opens out into said diffusion area and said at least one outlet opens out into said collecting area.

Said body and said plate may be single piece made or made of two distinct pieces. Said plate may be made of a transparent material allowing an observation of the at least one cavity through said plate. It should be noted that the microfluidic device may comprise a plurality of traps. Also, said traps may be arranged in rows, which are staggered along a direction extending between said at least one inlet and said at least one outlet.

According to the invention, when brought into the cavity of the traps, the first or the second droplets comprise cells, in particular cells recovered from culture and treatment according to the methods of the invention, especially mammalian cells as disclosed herein that have achieved a degree of 3D-structure organization such as gastruloids and embryoids disclosed herein. Besides, cells that have been brought into the cavity of the traps are allowed to grow in the

drop as further organized 3D-structures such as gastruloids or embryoids according to the invention when the other droplets brought to the cavity provides in particular cell culture medium and optionally Matrigel or equivalent matrix as disclosed herein.

In a particular embodiment, the traps may all be identical in the microfluidic device. The cavity of each trap is a blind hole and as such also serves as a chamber for the culture and growth of the provided cells in the drop. In other words, each cavity comprises a bottom that may be concave.

In a particular embodiment, the body may be parallelepipedic. Alternatively, the body may present a circular cross-section.

In a particular embodiment, the axis of revolution may be perpendicular to the top and/or bottom side of the body and/or the plate. The configuration within which the axis of revolution is substantially perpendicular to the bottom side and the plate may be considered as a preferred embodiment since it allows the maximum gravity forces to apply so as to promote the full droplets capture into the traps, when the microfluidic device rests horizontally on the plate when in operation.

The top and bottom sides may be parallel. In another embodiment, the axis of revolution may be tilted with regards to the top and/or bottom side of the body.

The annular wall of the first part of the cavity, of at least one or each trap, may be convex with regards to the axis of revolution.

For at least one, or for each trap, the cavity may be delimited in its second part by a cylindrical wall having a hexagonal cross-section. Accordingly, the cross section of the second part of the cavity of said trap may be constant along the axis of revolution. Such cross section allows the formation or the growth of organized 3D-structures of cells, such as spheroids as disclosed herein, and their growth as elongated structures to form gastruloids or embryoids according to the invention, when one or more droplets are trapped in the trap and provided with suitable culture medium.

For at least one, or for each trap, the cross section of the first part of the cavity of said trap, respectively the cross section of the second part of the cavity of said trap, may be comprised in a plane perpendicular to the axis of revolution.

In a particular embodiment, the dimension of the second part of the cavity of at least one or each trap along the axis of revolution (i.e. the height of the second part), may be at least five times the dimension of the first part of the cavity of at least one or each trap along the axis of revolution (i.e. the height of the first part).

In a particular embodiment, the dimension of the first part of the cavity of at least one or each trap, along the axis of revolution may constitute from 2% to 20% of the total dimension of the trap along the axis of revolution (i.e. the total height of the trap).

In a particular embodiment, the dimension of the second part of the cavity of at least one or each trap, along the axis of revolution may constitute from 80% to 95% of the total dimension of the trap along the axis of revolution.

In a particular embodiment, the opening, of at least one or each trap, may be circular. The diameter of the opening may be comprised from 2 to 3 mm, in particular from 2.2 to 2.6 mm, in particular equal to 2.4 mm. This arrangement promotes the trapping by capillarity of an initial droplet in the trap. These particular dimensions are well adapted for the manipulation of the first droplets of 7 $\mu$l.

In a particular embodiment, the diameter of the cross-section of the second part of the cavity, of at least one or each trap, may be comprised from 1 to 2 mm, in particular from 1.1 to 1.3 mm, in particular equal to 1.2 mm. The diameter of the cross-section of the second part of the cavity, of at least one or each trap, may be the diameter of an inscribed circle of the cross section of said second part of the cavity. This arrangement allows full trapping of a droplet inside the trap, by buoyancy and allows promoting a moderate level of said droplet confinement in the trap which prevents damaging the droplets inside the trap.

In an embodiment, each trap opens out in a channel formed by a recess arranged in the body on the side containing the opening of the traps, i.e. the bottom side of the body. In an embodiment, the bottom side may comprise an annular rim surrounding an internal bottom face and the traps that open out onto said internal bottom face. The microfluidic device may comprise a unique channel. The channel may extend in a plane parallel to the bottom side of the body and may have a hexagonal shape and may cover all the openings of the traps.

In an embodiment, the diameter of the opening, of at least one or each trap, may be greater than two times the dimension of the channel along the axis of revolution of the cavity. This arrangement promotes the trapping by capillarity of at least a droplet in the trap.

In a particular embodiment, the dimension of the channel along the axis of revolution (i.e. the height of the channel) may be comprised from 0.5 to 2 mm, in particular equal to 1mm. The channel allows a moderate level of confinement of droplets circulating in the channel.

In an embodiment, the curvature of the convex annular wall of the first part of the cavity, of at least one or each trap, may increase towards the opening along the axis of revolution. In another embodiment, the radius of curvature may be constant on the annular wall and may be around 0,5 mm.

In a particular embodiment, the dimension of each trap along the axis of revolution may be comprised from 2

to 6 mm, in particular from 3 to 5 mm, in particular may be equal to 4 mm. Such arrangement promotes full trapping of a droplet inside the second part of the cavity of the trap comprising said droplet and thus leaving an empty space at the first part of the cavity of the trap to anchor another droplet in the same trap. Besides, this arrangement promotes the contact between the two droplets in said trap. In a particular embodiment, the dimension of each trap along the axis of revolution (i.e. the total height of the trap) may be proportional to the volume of the droplets intended to be introduced in the microfluidic device.

In a particular embodiment, the distance between two adjacent traps may be comprised from 5 to 10 mm, in particular 7 to 9 mm, to in particular equal to 8 mm. This design limits the contact between the droplets. This distance between two adjacent traps may be measured between the axis of revolution of the respective traps.

In a particular embodiment, the microfluidic device may comprise a number of traps from 2 to 100, in particular may comprise 81 traps arranged along 9 columns. This arrangement provides a convenient number of biological replicates.

In an embodiment, the cavity of each trap may comprise a third part arranged at an end of the cavity opposite to the opening. The third part may be delimited by a concave wall forming a dome.

The microfluidic device may further comprise at least one duct forming an inlet of the microfluidic device into the channel. Said at least one duct is formed in the body and opens out at a first one end at the top side of the body and at an opposite second end into the channel. More particularly, the duct opens out in the diffusion area.

In a particular embodiment, the duct is tilted, with respect to the bottom side of the body, by an angle comprised from 30° to 60°, in particular equal to 45°. The orientation of the duct avoids droplet breaking at the entrance in the channel and also avoids loose liquid when injecting liquid into the microfluidic device.

The second end of said at least one duct may comprise an annular convex wall that prevents the droplets from breaking when droplets are introduced into the channel.

In a particular embodiment, the microfluidic device may comprise at least two rails arranged in the channel, suitable for guiding the droplets, outputted from the duct, to the traps. The rails guide the droplets evenly inside the channel. Each rail may be formed by a groove formed in the bottom side of the body or in the plate and having a depth smaller than two times the dimension of the channel along the axis of revolution. Each rail may have a depth of 0.5 mm.

In a particular embodiment, the body may comprise at least two microfluidic outlets that are formed by outlet conduits arranged at an end of said body that is opposite to said at least one inlet. Each outlet conduit is connected to the channel and may extend substantially vertically. The body may comprise three or four microfluidic outlet conduits. The outlet conduits open out in the top side of the microfluidic device. Those outlets limit the hydrostatic pressure inside the microfluidic device.

In another embodiment, the microfluidic device may comprise a plurality of inlets and a plurality of outlets. These inlets and outlets are formed in the body of the microfluidic device.

In a particular embodiment, the microfluidic device may comprise at least two inlets and at least two outlets. The microfluidic device may comprise the same number of inlets and outlets. Each inlet may be associated to one single outlet. Moreover, the microfluidic device is configured such that fluid entering by one specific inlet, flows through the channel and to one specific outlet. In another embodiment, the microfluidic device comprises three inlets and/or three outlets.

The body of the microfluidic device may have a substantially parallelepiped shape that comprises a first longitudinal axis corresponding to the direction of fluid flow, a second transverse axis and third axis corresponding to the thickness of the body. The inlets may be spaced, in the second direction, by a distance substantially equal to the distance spacing the outlets in the second direction. In this manner, the liquid entering one inlet tends to flow towards the outlet that is aligned with the inlet according to the first longitudinal direction. In this manner, should the microfluidic device comprise N inlets and N outlets, the fluid entering through one inlet k (k being comprised in the range 1 to N) flows through the channel and to the outlet k without mixing with the first adjacent fluid flowing from the inlet k-1 through the channel to the outlet k-1 and without mixing with the second adjacent fluid flowing from the inlet k+1 through the channel to the outlet k+1.

In a particular embodiment, the diameter of the opening, of each trap, may be comprised from 2.2 to 2.6 mm, and the diameter of the cross-section of the second part of the cavity, of each trap, may be comprised from 1.1 to 1.3 mm.

In a particular embodiment, the diameter of the opening, of each trap, may be comprised from 2.2 to 2.6 mm, and the diameter of the cross-section of the second part of the cavity, of each trap, may be comprised from 1.1 to 1.3 mm, and the dimension of each trap along the axis of revolution may be comprised from 3 to 5 mm. In a particular embodiment, the diameter of the opening, of each trap, may be comprised from 2.2 to 2.6 mm, and the diameter of the cross-section of the second part of the cavity, of each trap, may be comprised from 1.1 to 1.3 mm, and the dimension of the channel along the axis of revolution may be comprised from 0.5 to 2mm.

In a particular embodiment, the dimension of each trap along the axis of revolution may be comprised from 3 to 5 mm, and the dimension of the channel along the axis of revolution may be comprised from 0.5 to 2 mm.

In a particular embodiment, the microfluidic device may comprise four rails, arranged in the channel, and the

duct connecting the outside of the microfluidic device to the channel may be tilted, with respect to the bottom side of the body, by an angle comprised from 30° to 60.

In a particular embodiment, the body and the plate may be made of polymer such as polydimethylsiloxane (PDMS). Also, they may comprise each a material or any combinations of materials selected from, but not limited to, silicone rubber (i.e. polysiloxane), crystalline silicon, poly(dimethylsiloxane) (PDMS), silica (e.g., quartz and glass), thermoplastics (e.g., poly(methyl methacrylate) (PMMA), polycarbonate (PC), polystyrene (PS), poly(ethylene glycol) diacrylate (PEGDA), polyurethane (PU), perfluorinated compounds (e.g., perfluoroalkoxy (Teflon PFA) and fluorinated ethylenepropylene (Teflon FEP)), and polyolefins (e.g., cyclic olefin copolymer (COC), cyclic olefin polymer (COP), cyclic block copolymer (CBC), and polyvinyl chloride (PVC)), polyimide (PI), poly(lactic-co-glycolic acid) (PLGA), thermoset polyester (TPE), off-stoichiometry thiol-ene (OSTE), transparent ceramics such as aluminum oxide (Al2O3), spinel (MgAl2O4), yttria alumina garnet (YAG), and neodymium-doped yttria alumina garnet (Nd:YAG), and paper (e.g. transparent or translucent paper). In a particular embodiment, the first element and the second element comprise silica (e.g., quartz and glass). In another embodiment, the first element and the second element comprise poly(dimethylsiloxane) (PDMS), thermoplastics (e.g., poly(methyl methacrylate) (PMMA), polycarbonate (PC), polystyrene (PS), polyurethane (PU), perfluorinated compounds (e.g., perfluoroalkoxy (Teflon PFA) and fluorinated ethylenepropylene (Teflon FEP)), and polyolefins (e.g., cyclic olefin copolymer (COC), cyclic olefin polymer (COP), cyclic block copolymer (CBC), and polyvinyl chloride (PVC)), poly(lactic-co-glycolic acid) (PLGA), thermoset polyester (TPE).The body of microfluidic device may be fabricated by molding. The body and/or the plate may be made using additive manufacturing techniques.

In a particular embodiment, the plate may be made of glass.

According to an embodiment, the invention relates to a manufacturing process of the microfluidic device disclosed herein, said manufacturing process may comprise the following steps:

- providing a mold presenting mold imprints of the traps as disclosed herein,
- filling the mold with a mixture of a polymer base and a curing agent,
- placing the mold in an oven set up at least at 65°C for at least 4 hours,
- separating the resulting body from the mold,
- bonding the bottom side of the body to a plate slide,
- coating the microfluidic device, to be rendered fluorophilic, with an appropriate fluorophilic coating.

The polymer may be PDMS and the curing agent may be present at a ratio of 1:10, for example the volume of the curing agent may be for about 50-60 mL. The curing agent may be a chemical mixture sold with the PDMS base elastomer, for which the formulation is proprietary. The base contains a linear chain of siloxane with dimethyl groups and with Si-vinyl end groups, and a Pt catalyst complex. The curing agent is rich in Si-H bonds and may also contains other oligomers such as tetramethyl tetravinyl cyclotetrasiloxane (Venkatachalam et al., Ceramic International, 2019).

The plate slide may be a 75 × 50 mm rectangular. The plate slide may be made of glass or polymer. The plate slide may be bounded by plasma for two rounds of 40 seconds and by heating the resulting microfluidic device at least at 80°C, for at least 2 hours. The glass slide can be replaced by Polycarbonate, Polystyrene etc.

The fluorophilic coating may be fixed to the microfluidic device by heating the microfluidic device at least at 110°C, for three rounds.

In a further aspect, the invention relates to a method of manipulating droplets using the aforementioned microfluidic device, the method comprising:

(M1) introducing a first liquid composition comprising first droplets in the channel of the microfluidic device,
(M2) tilting the body at a first angle with respect to the horizontal axis, the first angle being comprised from 30° to 60°, in particular comprised from 40° to 50°, in particular equal to 45°,
(M3) trapping one of the first droplets by capillarity in the first part of the cavity of one of the traps and standing by until said first droplet migrates to the second part of the cavity of said trap by buoyancy,

According to another feature of the method, it may comprise:

(M4) introducing a second liquid composition comprising second droplets in said microfluidic device,
(M5) tilting the body at a second angle with respect to the horizontal axis, the second angle being comprised from 25° to 35°, in particular equal to 25°,
(M6) trapping one of the second droplet by capillarity in the first part of the cavity of said trap and standing by until the first droplet and the second droplet comprised in said trap merge.

The step (M2) promotes gravity forces in order to allow the droplets' motion inside the microfluidic device. The step (M5) allows to adjust the gravity forces in order to favor a second drop trapping in the anchors by capillary forces.

The first angle promotes gravity forces in order to allow the droplets motion inside the channel.

The second angle helps controlling the gravity forces in order to favor a second droplet trapping in the traps of the first part of the cavity by capillary forces.

In an embodiment, in the step (M2) and/or the step (M5), the microfluidic device may be tilted such as the outlets of the microfluidic device are above the horizontal axis while the inlet of the microfluidic device is bellow of the horizontal axis.

In a particular embodiment of the invention, the first droplets may be created by plugs of aqueous phase having a volume comprised from 5 μL to 10 μL, in particular equal to 7 μL. The first liquid composition may further comprise plugs of a fluorogenic oil having a volume comprised from 5 μL to 10 μL, in particular equal to 6 μL, said plugs of fluorogenic oil separating the first droplets during fabrication of the droplet before injection of the first liquid composition in the microfluidic device. The fluorogenic oil may contain a fluorogenic surfactant at 0.5% of the total weight.

In an embodiment, the method may comprise before step (M4) a step of flushing an immiscible fluorocarbon oil without surfactant in the traps.

In a particular embodiment of the invention, the second droplets may contain soluble molecules such as culture medium, dyes, or a biomaterial.

In some embodiments the second droplets may contain Matrigel or equivalent matrix as disclosed herein to provide gelified drops after fusion of the first and second droplets in step (M6). In such embodiments the Matrigel or equivalent matrix is in a proportion of 10% to 40% of the contents of the drop resulting from the fusion, in particular of about 20% of the contents of the drop resulting from the fusion when the hydrogel is Matrigel.

Besides, the first liquid composition and/or the second liquid composition may be introduced in the microfluidic device at a flow of 1000 μL/min.

In a particular embodiment of the invention, the method may comprise before step (M4) a step of placing the microfluidic device in an incubator set up at 37°C and 5% of $CO_2$ to allow cells culture. The cross section of the second part of the cavity promotes shaping the first droplet in a spheroid shape.

In some embodiments, after step (M6) when oil phase is present surrounding the gelified drops said oil phase is replaced by culture medium. In some embodiments culture medium is then renewed at chosen time intervals such as every 2 days.

In an embodiment, the first or the second droplets, in particular the first droplets, may comprise cells, in particular may comprise a homogenous population of pluripotent or multipotent vertebrate cells, in particular non-human mammalian cells, more particularly rodent cells or alternatively may comprise a homogenous population of pluripotent or multipotent human cells.

The method of using the microfluidic device according to the invention, in particular the method of manipulating droplets as disclosed herein may be implemented in the culture illustrated in the Examples such as in the culture disclosed in relation to Figures 4 and 5.

The invention will be further disclosed in the Examples and the figures that follow which highlight specific non limiting features and embodiments.

**FIGURE LEGENDS - the figures are filed as color figures**

**Fig. 1 Emergence of gastruloids from mouse Embryonic Stem Cells (mESCs) treated twice with SUMO inhibitors (Fig 1a) ML-792. a,** Protocol schematic. **b**, Spheroids obtained at D18; these Spheroids were used to obtain the results in Figures 2, to 4 and 6 to 13. c, Immunoblots of SUMO1 and SUMO2/3. **d,** Uniform Manifold Approximation & Projection (UMAP) plot of 4,707 cells from D1 and D18. Cells are colored by their cluster annotation. Inset shows cells colored by their time point. **e**, Expression levels of cluster markers. **f**, Immunostaining of spheroid cell types. **g**, Gastruloid obtained from dissociated spheroid cells cultured in suspension. **h**, UMAP plot of 3,478 cells isolated from gastruloids. Cells are colored by their cluster annotation. **i**, Expression of gastruloid-specific markers. **j**, Immunostaining, and **k,** HCR staining of gastruloids. Scale bars, 25 μm and **(Fig 1b) TAK-981 a,** Protocol schematic. **b**, Spheroids obtained at D18.

**Fig. 2 Characterization of D18 spheroid cell types. a**, Expression of cluster markers. PGC, primordial germ cells; XEN, extraembryonic endoderm; EPI, epiblast. **b**, Comparison of spheroid clusters to *in vivo* data[27] (p-value determined by two-sided binomial testing). **c**, UMAP plot of 4,974 cells isolated from D1 and D18, with the additional control condition of untreated ESCs cultured in N2B27+Lif. **d**, Expression of key cluster markers measured at D18, following 1 or 2 rounds of ML-792 in serum+Lif medium, with or without a medium switch to N2B27+Lif after the last round. N = 3. **e,** Expression of plasma membrane markers used for isolating the 3 different cell types forming D18 spheroids. **f**, Fractions isolated by FACS. **g,** Expression of key spheroid cell type markers in the isolated FACS fractions. N=3. **h,** *(Top)* Expression of key spheroid cell type markers in the Pecam1+ ES-L cells cultured post-FACS

in N2B27+Lif. N=3. *(Bottom)* Representative image of Pecam1+ ES-L cells cultured for 14 days after FACS isolation. **i**, *(Top)* Summary of cell survival and sphere morphology recovery after culture in N2B27+Lif from different combinations of the isolated FACS fractions. *(Bottom)* Representative images of the cell fraction combinations which recover sphere morphology after FACS. **j,** Alkaline phosphatase colony formation assay comparing control cells (D1) with cells generated at D18 of our protocol. Graph shows the number of colonies from 2 independent biological replicates (N1, N2), each having 3 technical replicates. **k**, Expression of differentiation markers in control cells (D1) and D18 cells treated with 1 $\mu$M of retinoic acid (RA) over 5 days in media without Lif. N=4. Scale bars, 50 $\mu$m.

**Fig. 3 Characterization of gastruloids. a**, Shape and size of gastruloids. ****p-value < 0.0001 (two-tailed unpaired t-test). **b**, Expression of cluster markers. NMP, neuromesodermal progenitors. **c**, Comparison of gastruloid clusters to *in vivo* data[28] (p-value determined by two-sided binomial testing). **d**, UMAP plot of 4,187 gastruloid cells and untreated ESCs in N2B27+Lif cultured for 3 days in AggreWell plates. **e**, Quantification of **T** signal along the minor (anterior-posterior) axis in immunofluorescence images (Fig. 1j). n=14. **f,** Expression of signaling pathway genes associated with primitive streak establishment. **g**, Expression of primitive streak marker *T* in structures obtained after culture of dissociated spheroid cells in AggreWell plates for 3 days with or without 500 nM of the BMP inhibitor DMH1. N=3. *p-value < 0.05 (two-tailed unpaired t-test).

**Fig. 4 Droplet-microfluidic culture and Matrigel embedding generate Embryo-Like Structures (ELS), a,** Microfluidic device. **b**, Protocol for generation of late gastruloids or ELS from D18 spheroids. **c**, Representative images of obtained structures. **d**, UMAP plot of 7,123 cells isolated after 2 or 5 days of culture in the microfluidic device (F2, F5), or after 4 days in the device and 3 days in Matrigel (M7). Cells are colored by their cluster annotation. Inset shows cells colored by their time point. **e**, Immunostaining of F5 late gastruloids. Graph shows quantification of T, Pax6 and Foxa2 signals along the major axis of F5 structures. Representative of N=3 independent experiments, n=16 gastruloids. **f**, Late gastruloid obtained from a Sox1::eGFP-T::mCherry ES cell line. Graph shows the evolution of the size of the structure and the area expressing T or Sox1 over time. n=11. Scale bars, 100 $\mu$m.

**Fig. 5 | Microfluidic device design and validation for pluripotent stem cell culture. a**, Microfluidic device characteristics. **b**, Microfluidic device mold. **c**, Distribution of the diameter of mESC aggregates formed in the microfluidic device and their evolution over time. n=337 aggregates. **d**, Aggregation kinetics (n=37 drops, n=25 wells), and **e,** growth kinetics (n=22 drops, n=16 wells) of mESCs in the microfluidic device compared to a 96-wells plate. Scale bars, 200 $\mu$m. **f**, Quantification of Ssea1-expressing mESCs grown in the microfluidic device compared to a 96-wells plate. **g**, *In situ* immunostaining of mESCs performed in the droplet-microfluidic platform. Scale bar, 25 $\mu$m.

**Fig. 6 | Characterization of ELS. a,** Growth kinetics of dissociated D18 spheroid cells in the microfluidic device or the AggreWell. Scale bar, 200 $\mu$m. Graphs show the evolution of the major axis length and the eccentricity of these structures over time. n=81 drops, n=122 microwells. ****p-value < 0.0001; *p-value < 0.05 (two-sided Mann-Whitney-Wilcoxon test with Bonferroni correction). **b**, Proportion of elongated structures obtained after Matrigel embedding. n=60. x, major axis length. Scale bar, 500 $\mu$m. **c** and **d,** Comparison of gastruloid (F2, F5) and embryo-like (M7) clusters to *in vivo* data[28,34] (p-value determined by two-sided binomial testing). **e**, Distance of Sox1 and T peak signal from the center of ::eGFP-T::mCherry gastruloids over time. n=11 gastruloids acquired every 3 hours. **f**, *(Top)* Evolution of the eccentricity of Sox1::eGFP-T::mCherry gastruloids over time. *(Bottom)* Slope of the eccentricity evolution for the time periods before and after 30 h. n= 11 gastruloids acquired every 3 hours. **p-value < 0.01 (two-sided Mann-Whitney-Wilcoxon test with Bonferroni correction). **g**, Proportion of cells from each time point in each cluster (cf. Fig. 2d).

**Fig. 7 | Embryo-like cell type markers.** Expression of cluster markers. NMP, neuromesodermal progenitors.

**Fig. 8 | Cell type determination. a**, Opposing Fgf and RA signaling in presomitic mesoderm (PSM) specify posterior (pPSM) and anterior (aPSM) regions respectively. **b**, Neural and mesodermal cell fate in NMPs are associated with Irx3/Sox1 and Nkx1-2 gradients. **c**, Expression levels of markers from different regions of the brain. **d**, Wnt1/3a/9a expression in the Mid-Hindbrain cluster. **e**, *(Top)* UMAP plot showing the cell cycle phase for the fluidic and Matrigel conditions (cf. Fig. 2d). *(Bottom)* Proportion of cells in each cell cycle phase for each cluster. **f**, Gene expression specifying progenitor and post-mitotic interneurons of the spinal cord in cluster 18.

**Fig. 9 | Anterior neuronal cell types and somites confer a head - trunk organization for the ELS. a,** HCR double staining of ELS. **b,** Sox2 immunostaining in a Sox1::eGFP-T::mCherry embryoid. **c**, and **d,** Immunostaining of ELS. Scale bars, 200 $\mu$m **(a, c, d),** 100 $\mu$m **(b).** Each image is representative of at least 3 biological replicates.

**Fig. 10 | HypoSUMOylation triggers global DNA hypermethylation. a**, Gene expression, and b, protein levels of components of the DNA methylation machinery. **c**, Evolution of the whole genome methylated CG fraction. **d**, Level of DNA methylation at super-enhancers related to the expression of the closest gene at D10 vs. D1. **e,** *Nanog* expression in cells at D8 after a round of hypoSUMOylation, followed or not by a 5-azacytidine treatment. N=4. *p-value < 0.05 (two-tailed unpaired t-test). **f**, Differentially marked SUMO peaks at D8 vs. D1. **g,** DNA motif enrichment identified in SUMO peaks UP and SUMO peaks DOWN. **h,** Browser view of the *Nanog* locus. SE, super-enhancer. **i**, Local ChIP for the indicated proteins at the *Nanog* super-enhancer. N=3. *p-value < 0.05; **p-value < 0.005 (two-tailed unpaired t-test).

**Fig. 11** | **Characterization of cell types between D1 and D10 of the hypoSUMOylation protocol. a**, *(Left)* UMAP plot of 6,120 cells isolated at D1, D3, D8 and D10 of the protocol (cf. Fig. 1a). Cells are colored by their cluster annotation. *(Right)* Cells from each time point are highlighted. **b**, Proportion of cells from each time point in clusters 4, 5 and 6. c, Expression of cluster markers. **d**, Expression of cluster 6 markers (XEN-L cells) from D1 to D10 in bulk-RNA-seq. **e**, Volcano plot showing the log2 fold change of gene expression and the statistical significance of the differential expression analysis performed between D8 and D1 in bulk-RNA-seq. Cluster 6 markers are in green, and cluster 4 markers in yellow. **f**, Expression of cluster 4 markers (2C-L cells) from D1 to D10 in bulk-RNA-seq. **g**, *(Top)* Representative image of spheroids obtained by performing 2 rounds of hypoSUMOylation in Dppa2/4 double knock-out mESCs. Scale bar, 50 μm. *(Bottom) Zscan4* expression from D1 to D10 in the Dppa2/4 DKO mESCs. N=2.

**Fig.12** | **Deregulation of the DNA methylation machinery following hypoSUMOylation. a**, Expression of components of the DNA methylation machinery. **b**, H3K9me3 and H3K27me3 signals from D1 to D10 centered around the H3K9me3 and H3K27me3 ChIP peaks respectively. **c**, Gene set enrichment categories for cluster 3 markers. **d**, Immunoblot of Sall4 and SUMO2/3 after transfection of siRNAs targeting Sall4 for 72 h, with or without concurrent ML-792 treatment for the last 48 h. e, Gene expression for the siSall4 experiment. N=4. *p-value < 0.05; **p-value < 0.005 (two-tailed unpaired t-test). **f**, Methylation changes between D1 and D10 at categories of chromatin. **g**, Immunoblot of Nanog. **h**, Alkaline phosphatase colony formation assay comparing D1 with D8. Graph shows the number of colonies from 3 independent biological replicates (N1, N2, N3), each having 3 technical replicates. *p-value < 0.05 (two-tailed nested t-test). **i**, Expression of differentiation markers in D1 and D8 cells treated with 1 μM of retinoic acid (RA) over 5 days in media without Lif. N=3. **j,** Treatment protocol and representative images of spheroids obtained by allowing a 5- or 15-day recovery period between the ML-792 rounds. Scale bars, 50 μm. Expression of key spheroid cell type markers for each protocol. N=3. **q,** Gene set enrichment categories for cluster 5 markers.

**Fig.13** | **Divergence of the SUMO chromatin landscapes at D8 vs. D1. a,** Proportion of SUMO peaks assigned to TSS, exon, intron and intergenic regions. **b**, Top 6 classes of transposable elements (TE) enriched in the SUMO peaks UP and SUMO peaks DOWN. c, MA plot displaying differentially marked SUMO peaks at D8 in comparison to D1. SUMO peaks overlapping L1Md_F and L1MD_F2 sequences are highlighted. **d**, Gene set enrichment categories for SUMO peaks UP. **e,** Changes in the level of DNA methylation at SUMO peaks UP *(left)* and all other SUMO peaks *(right)* according to the number of Zfp57 motifs. **f**, Expression of genes with a SUMO peak at their TSS. **g,** Correlation between the level of DNA methylation and the SUMO signal at SUMO peaks between D8 and D1. **h,** Local ChIP for the indicated proteins at the *Nanog* promoter. N=3. **i,** Three-step model of the impact of hypoSUMOylation on the regulation of the DNA methylation machinery and the expression of *Nanog.* 1/ SUMOylation suppression at D3 modifies the balance between TET and DNMT enzymes, in part through Sall4, leading to an excess of methylation across the genome. 2/ Hypermethylated regions containing Zfp57 binding motifs recruit Zfp57/Kap1-SUMO/Setdb1 complexes that deposit the chromatin-condensing mark H3K9me3. 3/ The expression of neighboring genes is dysregulated during the waves of hypoSUMOylation as exemplified by the strong reduction of *Nanog* at D8.

**Fig.14** is a top view of an example of the microfluidic device according to the invention.

**Fig.15** is a perspective view of the example of the microfluidic device.

**Fig.16** is a cross view according to line A100-A100 of the example of the body of the microfluidic device of figure 14.

**Fig.17** is an enlargement view of the area B100 of figure 16.

**Fig.18** is an enlargement view of the area C100 of figure 15.

**Fig.19** is a schematic view of trapping droplets in a trap of the example of the microfluidic device of figures 14-18.

**Fig.20** is a lateral view of the microfluidic device in a tilted position.

**Fig. 21** Protocol for droplet fusion on chip. A first drop was trapped in the top part of the anchors **(A).** Then a second drop was trapped in the bottom part of the anchors **(B).** The two drops were allowed to fuse and to generate large drops that contained the mix of the two initial droplets **(C).**

**Fig. 22** Selective perfusion of ELS on chips. Several inlets (122b and 122c on the left side of the schematic) and outlets (124b and 124c on the right side of the schematic) were added in front of the middle of a group of traps **(A).** Then the inlets were connected to syringes containing different chemical compositions. Streams of various chemical compositions could be flown in different areas of the chip without mixing under continuous perfusion **(B).**

**Fig 23** Embryoids encapsulated into Matrigel on chip.

## Examples

## METHODS

### Cell culture

Mouse ES-R1 cells[14] were used for most experiments and were maintained in serum+Lif medium (KnockOut DMEM supplemented with 15% ES cell qualified FBS, 1% GlutaMAX, 1% MEM non-essential amino acids, 1% penicillin-streptomycin, 0.1 mM 2-mercaptoethanol, 10 ng/mL Lif (Miltenyi Biotec #130-099-895)) on gelatin-coated plates in a humidified incubator (37°C, 5% $CO_2$). The Sox1 ::eGFP-T::mCherry double reporter CGR8 mouse ES cells (gift from David M. Suter, Swiss Federal Institute of Technology, Lausanne, Switzerland, ref.56) were maintained on Mitomycin C-treated mouse embryonic fibroblast feeder cells in serum+Lif medium, and the MERVL::tdTomato-Dppa2/4 DKO mouse ES-E14 cells (gift from Wolf Reik, Babraham Institute, Cambridge, UK[57]) were cultured in serum+Lif medium on gelatin-coated plates. Mouse ES-D3 cells (from ATCC) were used for droplet-microfluidic platform validation experiments and were cultured in ESLIF medium as previously described[10]. For 2i culture, ES-R1 cells were maintained in N2B27+Lif medium (1:1 Neurobasal medium and Advanced DMEM/F-12, 1 % N-2 supplement, 2% B-27 supplement, 0.05% Bovine albumin fraction V, 1% GlutaMAX, 1% penicillin-streptomycin, 0.1 mM 2-mercaptoethanol, 10 ng/mL Lif) supplemented with 1 μm PD0325901 (Miltenyi Biotec #130-103-923) and 3 μm CHIR99021 (Miltenyi Biotec #130-103-926).

For retinoic acid differentiation assays, cells were seeded in 6-well plates in medium without Lif (serum medium for D1and D8, N2B27 medium for D18) and treated with 1 μM of Retinoic Acid (Sigma #R2625). Medium was refreshed every day for a total of 5 days of treatment. Cells were collected every 24 h for RNA extractions.

For 5-azacytidine experiments, cells were seeded in 6-well plates in serum+Lif medium and treated with 0.1 μM of 5-azacytidine (Abcam #ab142744). Medium was refreshed every day for a total of 5 days of treatment.

### Clonogenic assay

500 cells (D1 and D8 in serum+Lif medium; D18 in N2B27+Lif) were seeded in 6-well plates. After 6 days, cells were fixed with 4% paraformaldehyde for 5 min and washed twice with PBS. Colonies were stained using Alkaline Phosphatase Blue Membrane Solution Kit (Sigma #AB0300) for 15 min in the dark. Wells were washed once with PBS and left to dry. Plates were scanned and colonies were counted using ImageJ.

### Generating adherent spheroids

- Using ML-792 as hypoSUMOylation agent (SUMO E1 enzyme inhibitor): ESCs (ES R1 cells - 129X1 × 129S1 - ATCC) were dissociated with Trypsin-EDTA and plated (2.5 million cells in 100 mm dish) in serum+Lif medium supplemented with 2.5 μM ML-792 (Takeda Pharmaceuticals International Co.). Medium was replaced the next day to refresh treatment. After 48 h of treatment, plates were rinsed twice with PBS then cells were allowed to recover in serum+Lif. 5 days after the end of the first round, cells were similarly counted and plated for a 2nd round of ML-792. After 48 h of treatment, plates were rinsed twice with PBS then cells were allowed to recover in N2B27+Lif medium. 6 to 8 days later, cells form three-dimensional adherent spheroids, which can be maintained in culture or frozen for subsequent use (cell stocks in ES cell qualified FBS with 10% DMSO).
- Using TAK-981 as hypoSUMOylation agent (SUMO E1 enzyme inhibitor): The protocol used is similar to the protocol disclosed herein when using ML-792 as a hypoSUMOylation agent in so far as cell line used (ES R1 cells - 129X1 × 129S1: the R1 cell line was established in August 1991, from a 3.5 day blastocyst produced by crossing two 129 substrains (129S1/SvImJ and 129X1/SvJ).), steps of thawing and cell culture procedure (thawing performed on feeders, culture performed on gelatin and passaging) incubator settings (37°C, 5% $CO_2$). By contrast the following specific parameters are taken into consideration with TAK-981:
  500 000 cells are plated in a well of a 6-well plate in serum + Lif medium, supplemented with 0.1 μM of TAK-981. Medium was replaced the next day to refresh treatment. After 48 h of treatment, wells were rinsed twice with PBS then cells were allowed to recover in serum+Lif. 6 days after the end of the first round, cells were similarly counted and plated for a 2nd round of TAK-981. After 48 h of treatment, wells were rinsed twice with PBS then cells were allowed to recover in N2B27+Lif medium. 3 to 5 days later, cells form three-dimensional adherent spheroids, which can be maintained in culture or frozen for subsequent use (cell stocks in ES cell qualified FBS with 10% DMSO).

**Cell sorting of the 3 cell types of D18 spheroids**

Cell surface markers specific to each spheroid cell type were extracted from the list of scRNA-seq cluster markers. The markers chosen for XEN-L and EPI-L cells were previously validated0[58].

Spheroids were briefly dissociated with StemPro Accutase (Gibco #A111-05-01) and cells were resuspended in PBS with 3% ES cell qualified FBS. Cells were incubated for 30 min at 4°C with the following antibodies: Cd31(Pecam1)-FITC (1:100, Invitrogen #11-0311-81), Pdgfra(Cd140)-PE (1:100, Invitrogen #12-1401-81), Cd24a-APCeFluor780 (1:100, Invitrogen #47-0242-82). Cells were washed twice with PBS-3% ES cell qualified FBS then resuspended in PBS-3% ES cell qualified FBS. Propidium iodide (1 ug/mL, Invitrogen #P3566) was added to cell suspension before transferring sample to a cell strainer cap tube. Cells were analyzed on a BD FACSAria III Cell Sorter (BD Biosciences). Cell fractions were collected in PBS-3% ES cell qualified FBS then divided for RNA extraction and resuspension in N2B27+Lif medium for re-plating.

**Culturing AggreWell gastruloids**

1 mL of anti-adherence rinsing solution (StemCell Technologies #07010) was added to each well of an AggreWell™800 plate (StemCell Technologies #34815). Plate was centrifuged for 5 min at 2,000 rpm then incubated 30 min at room temperature in a tissue culture hood. Wells were washed twice with 2 mL of PBS, then 500 μL of N2B27+Lif medium were added and plate was stored in a humidified incubator (37°C, 5% $CO_2$) until use.

Spheroids were briefly dissociated with Trypsin-EDTA and 30,000 cells in 1 mL of N2B27+Lif were seeded in each well (~ 100 cells per microwell). Plate was incubated (37°C, 5% $CO_2$) for 3 days to obtain gastruloids.

For BMP inhibitor experiments, 500 nM of BMP inhibitor II DMH1 (Sigma #203646) was added at seeding and gastruloids were collected after 3 days for RNA extraction.

**Droplet-microfluidic device design and fabrication**

The molds to fabricate the chips were designed using Fusion 360 (Autodesk). The molds were patterned with 81 traps on the top of the culture chamber (Fig.5a for the dimensions). The traps were equipped with two trapping areas: the bottom part of the traps allows droplet trapping by capillarity, while the top enables the full droplet anchoring by gravity (Fig.5a for the dimensions). The molds were also equipped with rails in order to guide the drops evenly within the culture chamber. The molds were 3D printed using a ClearV4 resin (Formlabs) and an SLA 3D printer (Form3, Formlabs). The molds were filled with a mixture of PDMS (SYLGARD®, Dow) base and a curing agent at a ratio of 1:10 (about 50-60 mL per chip). The molds were placed in an oven set at 65°C for at least 4 hours. After curing, the PDMS imprinted with the top of the chip were separated from the molds. The tops of the chip were then plasma bound (Cute, Femto Science Inc.) to a 75 × 50 mm glass slide (Corning #2947) for two rounds of 40 seconds, and placed in an oven set at 80°C for at least 2 hours. Finally, the chips were rendered fluorophilic by treating them with Novec™ 1720 (3M) and heating them at 110°C for three rounds of 30 minutes each.

**Pluripotent stem cell loading and manipulation within immobilized droplets**

An Upchurch cross-junction (PEEK, low pressure, 1/16 compression size) was used to form 7 μL plugs flowed at 1,000 μL/mL using syringe pumps (neMESYS, Cetoni) and containing 120-300 pluripotent stem cells (PSCs) in their medium. The aqueous plugs were separated by 6 μL plugs of fluorogenic oil (FC-40, 3M) containing a fluorogenic surfactant (RAN biotechnologies) at a concentration of 0.5% v/v, and were also flowed at 1,000 μL/min. The chips were placed at an angle of 45° from the horizontal to allow gravity to act as a driving force for droplet motion. The drops were then spontaneously captured by capillarity in the bottom part of the traps, thus preventing other drops from being anchored in the same traps at this stage. Next, the drops spontaneously moved by gravity to the top part of the traps after 3-5 minutes, leaving empty the bottom part of the traps (the capillary anchoring zone). The chips were then placed in a humidified incubator (37°C, 5% $CO_2$) to allow PSC culture for long time periods.

The performance of the droplet-microfluidic platform to promote PSC aggregation and expansion, and maintain expression of pluripotency markers was compared to standard 96-well plates. Briefly, about 300 mES-D3 were encapsulated into drops containing ESLIF medium, while the same cell number was seeded into 96-well plates in 100 μL of ESLIF medium per well. The kinetics of cell aggregation and proliferation were monitored by imaging.

The level of expression of pluripotency marker Ssea1 was quantified by flow cytometry, using an LSR-Fortessa (BD Biosciences), and by labelling the cells with mouse AlexaFluor647-conjugated anti-Ssea1 antibody (1:100, BD Biosciences # 560120). The level of expression of pluripotency marker Oct4 was analyzed by imaging after methanol fixation and *in situ* immunolabelling using a mouse anti-Oct4 antibody (1:100, Millipore #MAB 4419), which was revealed using an AlexaFluor488 conjugated goat anti-mouse IgG1 (1:100, Invitrogen #A21121).

### Culturing droplet-microfluidic late gastruloids and Matrigel embryo-like structures

Spheroids were briefly dissociated with Trypsin-EDTA and a suspension of 168,000 cells in 10 mL of N2B27+Lif was prepared (~ 120 cells per 7 μL droplet). Cells were loaded into the droplet-microfluidic device as described above and incubated for 5 days (37°C, 5% $CO_2$) to obtain late gastruloids.

Structures were recovered from the droplet-microfluidic device after 4 days by flipping the chip at a 90° angle while flushing pure FC-40. The oil was separated from the aqueous phase containing the gastruloids by filtration on a PTFE membrane (Thermo-Fisher #F2517-9). 1-4 gastruloids were seeded in each well flat-bottom of low adhesion 96-well plates (Corning #3474) in 100 μL of N2B27+Lif medium containing 20% Matrigel (Corning #354234). Plates were incubated for 2 to 3 days (37°C, 5% $CO_2$) to promote elongation of the embryo-like structures.

### Immunofluorescence

Spheroids were seeded in 8-well chamber slides (Ibidi #80826) at a density of 40,000 cells / 300 μL. 48 h later, cells were fixed in 4% paraformaldehyde for 8 min, permeabilized in 0.2% Triton-PBS for 20 min and incubated in blocking buffer (10% BSA, 5% serum, 0.1% Triton-PBS) for 2 h at room temperature. For gastruloids, the structures were collected from AggreWells and fixed in 4% paraformaldehyde for 15 min. Then, the structures were permeabilized in 0.5% Triton-PBS for 20 min and incubated in blocking buffer (10% BSA, 5% serum, 0.1% Triton-PBS) for 3 h at room temperature. Primary antibodies were diluted in 1% BSA, 0.1% Triton-PBS and incubated overnight at 4°C with the spheroids or the gastruloids. After 3 washes of 10 min in PBS, the structures were incubated with the secondary antibodies diluted at 1:400 for at least 1 h at room temperature then washed with PBS 3 times for 10 min. To immunolabel the late gastruloids and the embryo-like structures, the samples were first fixed with 4% paraformaldehyde for 2 hours at 4°C. The structures were then incubated overnight at 4°C in PBSFT (5% FBS and 0.5% Triton-X100 in PBS). The primary antibodies were diluted in PBSFT and incubated with the samples overnight at 4°C on an orbital rocker. After 3 washes with PBSFT, the samples were incubated overnight with a solution of 1:100 diluted secondary conjugated antibody containing 0.2 mM DAPI (Thermo-Fisher #R37606) at 4°C on an orbital rocker. After washing with PBS, the samples were cleared using RapiClear 1.52 (Sunjin lab), following the manufacturer's instructions. The specificity of the primary antibodies was verified by incubating the samples with the secondary antibody alone. Under these conditions, an absence of fluorescent signal validated the specificity of the primary antibodies.

The antibodies used were rat anti-Nanog (1:300, eBioscience #14-5761-80), goat anti-Sox17 (1:100, R&D Systems #AF1924), rabbit anti-Pou3f1 (1:100, Sigma #HPA073824), goat anti-T (1:100, R&D Systems #AF2085) or rabbit anti-T (1:100, Abcam #ab209655), mouse anti-Pax6 (1:100, Abcam #ab78545), rabbit anti-Foxa2 (1:100, Cell Signaling # D56D6), mouse anti-Sox2 (1:100, Millipore #17-656), mouse anti-Tuj1 (1:100, Biolegend #801201), rabbit anti-Pax2 (1:100, Invitrogen #71-600), mouse anti-Map2 (1:100, Sigma-Aldrich # M4403), rabbit anti-Sox10 (1:100, Abcam #ab264405), AlexaFluor488 donkey anti-rat (Invitrogen #A21208), AlexaFluor546 donkey anti-goat (Invitrogen #A11056), AlexaFluor647 donkey anti-rabbit (Invitrogen #A31573), AlexaFluor488 goat-anti-mouse (Invitrogen # A11001).

### *In situ* hybridization

Gastruloids were collected 4 days after cell seeding in AggreWells and fixed for 7 h in 4% paraformaldehyde at 4°C before dehydration in methanol. No proteinase K incubation was performed after rehydration. Embryo-like structures were collected 2 days after Matrigel embedding and fixed overnight in 4% paraformaldehyde at 4°C before dehydration in methanol. Structures were incubated for 10 min with proteinase K (10 μg/mL) after rehydration. *In situ* whole mount HCR V3 was performed as previously described[31] using reagents from Molecular Instruments. Briefly, each condition (up to 100 gastruloids or 20 embryo-like structures) was incubated in 1 mL of probe hybridization buffer for 5 min at room temperature and 30 min at 37°C before incubation with 2 pmol of each probe in 500 μL of probe hybridization buffer overnight at 37°C. The next day, samples were washed 4 × 15 min with 1 mL probe wash buffer at 37°C, and 2 × 5 min with 1mL 5X SSC-Tween at room temperature, then incubated in 1 mL amplification buffer for 5 min at room temperature. A mixture of 30 pmol of each hairpin (individually snap cooled beforehand) in 500 μL of amplification buffer was added to samples for an overnight incubation at room temperature in the dark. The next day, samples were washed 2 × 5 min, 2 × 30 min, 1 × 5 min with 1 mL 5X SSC-Tween at room temperature in the dark then stored at 4°C before imaging. Accession numbers for HCR probes used were *Nanog* (NM_001289828.1, hairpin B1), *T* (NM_009309.2, hairpin B3), *En1* (NM_010133.2 hairpin B4), *Uncx* (NM_013702.3, hairpin B1). Hairpins B1 were labeled with AlexaFluor488 or AlexaFluor546, hairpin B3 with AlexaFluor647 and hairpin B4 with AlexaFluor488 or AlexaFluor647.

**Microscopy**

The images were acquired using a motorized microscope (Ti or Ti 2, Eclipse, Nikon), equipped with a CMOS (complementary metal-oxide semiconductor) camera (ORCA-Flash4.0, Hamamatsu). Widefield imaging was performed by illuminating the samples with a fluorescence light-emitting diode source (Spectra X, Lumencor), while for spinning disc confocal imaging the samples were illuminated with lasers (W1, Yokogawa). The images were taken with a 10× objective with a 4-mm working distance (extra-long working distance) and a 0.45 numerical aperture (NA) (Plan Apo λ, Nikon). For widefield live imaging, the samples were imaged using a Muvicyte (Perkin-Elmer) equipped with a 10x objective with a 10-mm working distance and 0.30 NA (UPlanFL N, Olympus), which was placed in a humidified incubator (37°C, 5% $CO_2$). Images were acquired in brightfield every 30 min for the embryo-like structures in Matrigel. For the fluorescent reporter cell line Sox1 ::eGFP-T::mCherry, cells were cultured in phenol red-free N2B27+Lif medium and images were acquired every 3 hours.

**Image analysis**

The brightfield and fluorescent images were analyzed with a Python custom image analysis algorithm. Briefly, the aggregates were first detected from the brightfield images by edges detection. They were then centered and aligned along their major axis, which enabled to measure their major (a) and minor (b) axis length. The eccentricity was calculated as follows:

$$Eccentricity = \sqrt{(1 - \frac{a^2}{b^2})}$$

The fluorescent images were segmented using an automatically calculated threshold (Otsu's method). Then, the segments corresponding to gastruloids were oriented along their major axis, according to their red fluorescent signal (i.e. mCherry, TRITC). To quantify the time evolution of the structural organization within Sox1::eGFP-T::mCherry fluorescent reporter gastruloids, the distance of the maximum intensity of the mCherry and eGPF signals from the structure's center was calculated for every time point. In addition, the area of the mCherry and eGFP signals was measured for every time point.

For immunofluorescence and *in situ* HCR samples, the length of the major or minor axis was normalized for each gastruloid. Then, the images were segmented along the selected axis into a specific number of bins (ranging from -0.5 to 0.5, with 0 being the center of the gastruloid), for which the average fluorescent signal of each channel was measured.

**Single-cell RNA-seq**

Cells were dissociated with Trypsin-EDTA and resuspended in PBS. Propidium iodide (1 ug/mL, Invitrogen #P3566) and Calcein AM (1.5 ug/mL, Invitrogen #C31 00MP) were added to cell suspension before transferring sample to a cell strainer cap tube. Cells were sorted into 384-well cell capture plates using a BD FACSAria III Cell Sorter (BD Biosciences) to collect live cells and sort only singlets. Plates were snap frozen on dry ice and stored at -80°C until further processing. All single cell libraries were prepared with the same conditions and reagents using the MARS-seq protocol as previously described[59]. Briefly, a Bravo Automated Liquid Handling Platform (Agilent) was used to reverse transcribe (Invitrogen #18080085) mRNA into cDNA with an oligonucleotide containing both the unique molecule identifiers (UMIs) and cell barcodes. Unused oligonucleotides were removed by Exonuclease I (New England Biolabs #M0293S) treatment. cDNAs were pooled (each pool containing half of a 384-well plate) for second strand synthesis (New England Biolabs #E6111S) and in vitro transcription amplification (New England Biolabs #E2040S). DNA template was removed (Invitrogen #AM2238) before fragmenting (Invitrogen #AM8740) and ligating (New England Biolabs #M0204S) resulting RNA to an oligo containing the pool barcode and Illumina sequences. Finally, RNA was reverse transcribed (Agilent Technologies #600107) and libraries were amplified (Roche #7958935001). Libraries were quantified with a Qubit 2.0 (Invitrogen) and their size distribution was determined by a 4200 TapeStation System (Agilent Technologies). Finally, libraries were pooled at equimolar concentration and sequenced on an Illumina NextSeq500, in 8 sequencing runs, using high-output 75 cycles v2.5 kits (Illumina #20024906).

**Processing single-cell data**

The mouse genome GRCm38.p6 (mm10) with the gencode annotation M23 was used for all sequencing analyses (https://www.gencodegenes.org/mouse/release M23.html).

The MARS-seq2.0 pipeline[60] was used to produce count tables. The Seurat 4 R package[61] was used for normalization, dimension reduction and clustering. A manual iterative strategy was used to exclude cell libraries with low complexities. Briefly, all libraries (cells and empty control wells) in the count matrix were run through a standard Seurat workflow from count data to cluster computation (50 PCA dimensions to generate the neighbors' graph and UMAP computation). Empty wells and poor-quality cells usually clustered together and manual inspection allowed removal of clusters with low UMIs (inferior in mean ~1000 UMIs). This process was repeated until no low UMIs cluster remained. Cells with mitochondrial gene expression fractions greater than 2.5% were also excluded. Batch effects due to sequencing runs performed on different days were removed using the Harmony package[62] in Seurat.

Cluster markers were computed with the 'FindAllMarkers' Seurat function using the default parameters (except for the only.pos argument set to True, to only list genes upregulated in each cluster). Markers were considered significant if their adjusted p-value was inferior to 0.05.

The cell cycle score was computed with the 'CellCyclingScoring' Seurat function using the provided gene list.

### Comparison between scRNA-seq clusters and *in vivo* datasets

For the D18 spheroid clusters comparison to *in vivo* data, the 'FindMarkers' Seurat function was used to compute genes differentially expressed between the XEN-L (#2) and EPI-L (#3) clusters. The list of genes upregulated in each cluster was compared to the lists of differentially expressed genes between the epiblast and the primitive endoderm / visceral endoderm at E4.5, E5.5, E6.5[27]. For the AggreWell gastruloids, droplet-microfluidic device gastruloids and Matrigel embryo-like structures, the lists of cluster markers computed with the 'FindAllMarkers' Seurat function were compared to the markers identified for the different embryonic cell types defined in previously published mouse embryo scRNA-seq datasets[28,35]. Common genes, with a $\log_2$-transformed fold change superior to 1.01 and an adjusted p-value inferior to 0.01, were found and significance was assigned using a binomial test as previously described[10].

### Methyl-seq

DNA was extracted and purified from 2 million cells for each condition with the *Quick*-DNA Midiprep Plus Kit (Zymo Research D4075) following manufacturer's instructions. DNA was quantified with a NanoDrop ND-1000 (ThermoFisher Scientific). The NEBNext Enzymatic Methyl-seq Kit (New England Biolabs #E7120S) was used to prepare libraries for detection of 5-mC and 5-hmC. 200 ng of DNA from each sample were sheared to 275 bp fragments with an E220 Focused-ultrasonicator (Covaris) with the following settings: Duty Factor, 10% - Peak Incident Power, 175 W-Cycles per burst, 200 - Duration, 100 sec. Fragment size was validated by a 4200 TapeStation System (Agilent Technologies). The NEBNext Enzymatic Methyl-seq Kit workflow was then followed, using the sodium hydroxide option for the denaturation step. The size distribution and concentration of the libraries was determined by TapeStation. The libraries were sequenced on an Illumina HiSeq4000 sequencer as paired-end 100 base reads following Illumina's instructions. Image analysis and base calling were performed using RTA 2.7.3 and bcl2fastq 2.17.1.14.

### Processing methyl-seq data

Methyl-seq data were processed with the Bismark pipeline[63] using bowtie2 aligner[64] with the default parameters. Biological triplicates were merged and CG sites with at least 5 reads were kept for downstream analyses. Methylated and unmethylated CG sites were counted within predetermined windows (bin or interval) and a binomial test was used to compare different timepoints or regions. ESC super-enhancers genome coordinates were taken from Whyte et *al*[65]. The liftOver webtool from the UCSC website (https://genome.ucsc.edu/cgi-bin/hgLiftOver) was used to convert the mm9 track bed file to a mm10 bed file. The chromHMM genome annotation for ESCs produced by Pintacuda *et al*[66] was also used (https://qithub.com/guifengwei/ChromHMM mESC mm10).

### ChIP-seq and local ChIP

Cells at D1, D3, D8 and D10 were fixed for 10 min at room temperature in culture medium with 1% formaldehyde (Thermo Scientific #28908). Formaldehyde was then quenched with glycine (125 mM final). Cells were washed in ice cold PBS. The extracted chromatin was sonicated with a Bioruptor Pico (Diagenode) until chromatin fragments reached a size of 200-400 base pairs (30 sec ON, 30 sec OFF, 6 cycles), as assayed by electrophoresis through agarose gels. Immunoprecipitation, reversal of cross-linking and DNA purification were performed using ChIP-IT kit (Active Motif #53040). Polyclonal antibodies against SUMO1 (Abcam #ab32058), H3K4me3 (Active Motif #39159), H3K9me3 (Abcam #ab8898), H3K27me3 (Millipore #07-449) were used for ChIP-seq. For local ChIP experiments, a similar approach was performed using the following antibodies: SUMO2 (Abcam #ab3742), Zfp57 (Abcam #ab45341), Kap1 (Abcam #ab10483), Setdb1 (Proteintech #11231-1-AP), H3K9me3(Abcam #ab8898) and IgG (Cell Signaling #2729S). 50 ng of

spike-in chromatin (Active Motif #53083) and 2 μg of spike-in antibody (Active Motif #61686) were added to normalize the signal between ChIP-seq experimental samples.

ChIP-seq libraries were prepared using Microplex Library Preparation kit V2 (Diagenode #C05010014) following the manufacturer's protocol (V2 02.15) with some modifications. Briefly, in the first step, 10 ng of double-stranded ChIP enriched DNA or input DNA was repaired to yield molecules with blunt ends. In the next step, stem-loop adaptors with blocked 5' ends were ligated to the 5' end of the genomic DNA, leaving a nick at the 3' end. In the third step, the 3' ends of the genomic DNA were extended to complete library synthesis and Illumina -compatible indexes were added through a high-fidelity amplification. In an additional step, the libraries were size selected (200-400bp) and cleaned-up using AMPure XP beads (Beckman Coulter #A63881). Prior to analyses, DNA libraries were checked for quality and quantified using a 2100 Bioanalyzer (Agilent). The libraries were sequenced on an Illumina HiSeq4000 sequencer as paired-end 100 base reads following Illumina's instructions. Image analysis and base calling were performed using RTA 2.7.3 and bcl2fastq 2.17.1.14.

**Processing ChIP-seq data**

Libraries were aligned using bowtie2[64] with default parameters on mouse and fly genomes together. All alignments were filtered on MAPQ (mapping quality value) 30 with SAMtools[67] Libraries were deduplicated with the Picard toolkit[68]. The number of reads mapped on the fly genome was used as a spike-in value to downsample libraries as previously described[69]. Peak calling was performed with MACS2[70] with default parameters.

For the SUMO1 ChIP-seq data, low coverage peaks were filtered out. The pileup values were extracted from the MACS2 output, transformed using $\log_{10}$ and scaled. Peaks with a scaled $\log_{10}$ value inferior to -0.5 were filtered out from each replicate. This corresponded approximately to the 33% quantile (-0.52, -0.53, -0.48, -0.55, for D1 rep1, D1 rep2, D8 rep1, D8 rep2, respectively).

An irreproducible discovery rate (IDR)[71] of 0.1 was used to filter out irreproducible peaks. For each histone mark or SUMO1 ChIP-seq dataset, the IDR validated peaks from all time points were merged using the bedtools merge function[72].

A differential analysis was performed for the SUMO1 ChIP-seq data by counting the number of reads for each peak in each downsampled replicate with the featureCounts program[73]. The produced matrix was analyzed with the DESeq2 R package[74], using a size factor of 1 for the 4 libraries (2 rep D1, 2 rep D8). Changes of SUMO1 levels between D1 and D8 were considered significant if the adjusted p-value was inferior to 0.05.

For motif enrichment analysis, a 400 bp window centered on the local maximum coverage for each peak was first identified. The MEME-ChIP webtool[75] was used with default parameters. H3K4me3 and H3K27me3 ChIP-seq data generated in this study were used to classify the transcription start sites (TSSs). TSSs were classified as "inactive" in the absence of both peaks, "active" when marked only by H3K4me3, "repressed" when marked only by H3K27me3 and "bivalent" when having both H3K4me3 and H3K27me3. The class of TSSs was attributed for SUMO peaks overlapping the 1kb neighborhood centered in any TSS. SUMO peaks were annotated with the following priority: TSS, exon, intron, intergenic with respect to the UCSC mm10 transcript annotations.

**Gene enrichment analysis**

The EGSEA R package[76] was used for gene list enrichment with Gene Ontology term (GO term), pathways (KEGG, Biocarta) or curated gene list (mSigDB) with the egsea.ora function (Over-representation Analysis).

**Bulk RNA-seq**

Total RNA was purified by Trizol extraction and RNA was analyzed on a BioAnalyzer Nano chip (Agilent). If the RNA integrity number was superior to 8, samples were used for subsequent analyses. RNA concentration was quantified with a Qubit (Invitrogen). Total RNA-seq libraries were generated from 500 ng of total RNA using TruSeq Stranded Total RNA Library Prep Gold kit and TruSeq RNA Single Indexes kits A and B (Illumina), according to manufacturer's instructions. Briefly, cytoplasmic and mitochondrial ribosomal RNA (rRNA) were removed using biotinylated, target-specific oligos combined with Ribo-Zero rRNA removal beads. Following purification, the depleted RNA was fragmented into small pieces using divalent cations at 94°C for 2 minutes. Cleaved RNA fragments were then copied into first strand cDNA using reverse transcriptase and random primers followed by second strand cDNA synthesis using DNA Polymerase I and RNase H. Strand specificity was achieved by replacing dTTP with dUTP during second strand synthesis. The double stranded cDNA fragments were blunted using T4 DNA polymerase, Klenow DNA polymerase and T4 PNK. A single 'A' nucleotide was added to the 3' ends of the blunt DNA fragments using a Klenow fragment (3' to 5'exo minus) enzyme. The cDNA fragments were ligated to double stranded adapters using T4 DNA Ligase. The ligated products were enriched by PCR amplification (30 sec at 98°C; [10 sec at 98°C, 30 sec at 60°C, 30 sec at 72°C]

× 12 cycles; 5 min at 72°C). Surplus PCR primers were further removed by purification using AMPure XP beads (Beckman Coulter #A63881) and the final cDNA libraries were checked for quality and quantified using capillary electrophoresis. The libraries were sequenced on an Illumina HiSeq4000 sequencer as paired-end 50 base reads following Illumina's instructions. Image analysis and base calling were performed using RTA 2.7.3 and bcl2fastq 2.17.1.14.

**Processing bulk RNA-seq data**

FastQC (Version 0.11.2) was run using the following arguments --nogroup --casava to produce base quality, base sequence content and duplicated reads. FastQ-Screen (Version 0.5.1) was run using the following arguments: --subset 10000000 --aligner bowtie --bowtie '-p 2'x. In order to avoid PCR amplification biases in read quantification, duplicated reads were removed using the MarkDuplicates tool of Picard. The differential expression analysis of DESeq2 was applied on the filtered replicates[77].

**Immunoblots**

Cells were collected and directly lysed in Laemmli buffer (Bio-Rad #161-0747). Proteins were quantified using Pierce 660 nm Protein Assay (ThermoFisher Scientific #22662) according to manufacturer's instructions. Equal amounts of proteins were loaded on gels and good equilibration of the different samples was assessed by Ponceau staining after membrane transfer. Antibodies against SUMO1 (1:1000, Abcam #ab32058), Actin (1:4000, Sigma #A1978), SUMO2/3 (1:1000, Abcam #ab81371), Gapdh (1:1000, Cell Signaling #2118), Dnmt1 (1:1000, Abcam #ab13537), Dnmt3a (1:1000, Abcam #ab2850), Dnmt3b (1:1000, Abcam #ab2851), Tet2 (1:1000, Cell Signaling #36449S), Histone H3 (1:5000, Abcam #ab24834) were used according to standard protocols and suppliers' recommendations.

**Quantitative PCR**

cDNA was generated with a High-Capacity cDNA Reverse Transcription Kit (Applied Biosystems #4368814) from 500 ng to 2 μg of total RNA purified by Trizol extraction. Quantitative real-time PCR analysis was performed with SYBR Green PCR master mix (Applied Biosystems #4309155) and the primer sets indicated in Supplementary Table 7 using cDNA or genomic DNA (local ChIP). Quantitative real-time PCR analysis was performed on a CFX96 Touch Real-Time PCR Detection System (Bio-Rad) or a QuantStudio 6 Flex Real-Time PCR System (Applied Biosystems).

**Preparation of Gastruloids from hypoSUMOylated mouse Embryonic Stem Cells (mESCs)**

We used ML-792, a highly selective inhibitor of the small ubiquitin-like modifier (SUMO) E1 enzyme[26], to decrease the global level of SUMOylation in mouse ESCs, and found that two rounds of 48 h treatments, followed by a medium switch, yielded large spheroid structures on adherent plates (Fig. 1a, b). We confirmed the drop of SUMO conjugates at day 3 (D3) and D10, corresponding to the first and second treatment respectively, while inhibitor withdrawal fully restored the level of SUMOylation between the two rounds at D8 (Fig. 1c). Single-cell RNA sequencing (scRNA-seq) revealed that D18 spheroids are composed of three distinct cell types, whereas D1 ESCs form a homogeneous population (Fig. 1d). Using known marker genes and comparisons to *in vivo* peri-implantation data[27], we annotated cluster 3 as epiblast-like (EPI-L) cells and cluster 2 as extraembryonic endoderm (XEN-L) cells, and found a strong similarity with E5.5 embryos (Fig. 1e, Fig. 2a, b, Table 1). Cluster 1 was enriched for pluripotency markers and overlapped with untreated ESCs grown in N2B27+Lif medium, hence the name ESC-like (ES-L) for this cluster (Fig. 1e, Fig. 2a, c). Note that ES-L cells strongly expressed specific primordial germ cell (PGC) markers (*Dppa3, Ifitm1, Ifitm3*) in comparison to ESCs, indicating an atypical transcriptional profile (Fig. 1e, Fig. 2a). Importantly, two rounds of hypoSUMOylation and the medium switch were essential for both the formation of spheroids and the high expression of cluster-specific markers (Fig. 2d). Immunofluorescent labeling for the three cell types revealed a 3-D organization, with an ES-L core resting on EPI-L cells and surrounded by XEN-L cells (Fig. 1f). We thus investigated their interdependence to form spheroids by isolating them by flow cytometry (Fig. 2e-g). Whereas ES-L cells alone were able to grow and divide, sorted fractions of XEN-L and EPI-L cells did not survive independently, demonstrating reciprocal communication to maintain spheroid homeostasis (2 Fig. 2h, i). Interestingly, mixing ES-L and EPI-L in the same proportions was sufficient to recover spheroid morphology, indicating that XEN-L cells were not essential for this phenotype (Fig. 2i). The presence of ES-L cells prompted us to evaluate self-renewal capacity and differentiation potential of the spheroids. Cells at D18 were unable to form alkaline-phosphatase positive colonies and failed to activate retinoic acid-responsive genes (Fig. 2j, k), demonstrating the lack of pluripotency of all spheroid cell types. Together, these results show that sequential waves of hypo-SUMOylation in ESCs give rise to 3 cell types capable of self-assembly into spheroids.

Recent studies demonstrated that combining ESCs, XENs and trophoblast stem cells (TSCs) generates Embryo-Like Structures (ELS) closely recapitulating gastrulation[2,3]. We thus hypothesized that crosstalk between spheroid

cell types in specific culture conditions might mimic morphogenetic events akin to early embryogenesis. Transferring 100 spheroid cells to a non-adherent microwell (AggreWell) resulted in elongated structures after 3 days, with 80% efficacy (Fig. 1g, Fig. 3a,). In addition to the cell types detected in spheroids, scRNA-seq analysis of AggreWell structures revealed new cell populations derived from EPI-L cells (Fig. 1h, Fig. 3b). Comparing their transcriptional signatures to a dataset[28] of early embryogenesis allowed us to annotate them as primitive streak (cluster 4), definitive endoderm (cluster 5), neuromesodermal progenitors (NMPs, cluster 6) and neuroepithelium (cluster 7) (Fig. 1h, i, Fig. 3b, c, Table 2). These cell types could not emerge from untreated ESCs cultured in similar conditions (Fig. 3d). Immunostaining of the elongated structures revealed a spontaneous organization with discrete ES-L- and EPI-L-derived compartments echoing the embryonic/abembryonic axis of the mouse blastocyst[29] (Fig. 1j). Moreover, the polarized positioning of Brachyury (T)-positive cells between EPI-L and ES-L cells recapitulated the symmetry breaking event that establishes the anterior-posterior (A/P) body axis around E6 in mice[29] (Fig. 1j, k, Fig. 3e). Blocking Bone morphogenetic protein (BMP) signaling prevented the full expression of T, suggesting that ES-L cells partly mimic the TSC-derived extraembryonic ectoderm by secreting Bmp4 to activate *Wnt3* expression in the EPI-L cells, which is required for *T* expression and primitive streak establishment[30] (Fig. 3f, g). Strikingly, the mid-hindbrain marker *En1* was prematurely expressed on the opposite side of the primitive streak indicating that our structures were partially desynchronized while maintaining strict regional identities (Fig. 1k).

Together, these results show that self-organized structures generated from spheroids largely recapitulate architecture typical of the post-implantation mouse embryo, and will henceforth be referred to as "gastruloids".

**Preparation of Droplet-microfluidic-derived embryo-like structures**

Microfluidic systems have recently been used to improve multicellular self-organization in controlled environments[13,31-33]. To expand the developmental potential of gastruloids, we seeded 120 dissociated cells from D18 spheroids in a new custommade droplet-microfluidics platform optimized for ESC culture (Fig. 1a, 4a, b, Fig. 5). Structures grown in this device showed a higher degree of axial elongation after a 5-day culture period in comparison to AggreWells (Fig. 4c, Fig. 6a). Collecting gastruloids after 4 days in droplets and embedding them in 20% Matrigel further increased elongation to reach over 1 mm in length in 3 days (Fig. 4b, c, Fig. 64b,). We performed scRNA-seq to characterize the cellular content of these structures over time in drops (F2 and F5) and in Matrigel (M7) (Fig. 4b-d). Comparing our clusters to *in vivo* mouse transcriptomic datasets[28,34] from gastrulation to early organogenesis revealed the emergence of a wide diversity of cell types derived from the three germ layers, including ectodermal lineages (clusters 9, 15, 16, 17, 18), mesodermal subtypes (clusters 5, 6, 7, 10, 11, 12, 13, 14) and the gut endoderm (cluster 8) (Fig. 4d, Fig.6c, d). Immunostaining for Pax6 (neurectoderm), Foxa2 (definitive endoderm) and T (mesoderm) revealed tissue-specific patterning along the A/P axis, with Pax6- and T-positive cells located on opposite F5 gastruloid poles (Fig. 4e). Cells expressing Foxa2 were positioned in the central part of the structure and formed an epithelium surrounding a lumen, reminiscent of an embryonic digestive tract (Fig. 4e). To get dynamic insight into the A/P polarization, we used a reporter cell line to measure spatial and temporal evolution of *Sox1* (neuroectoderm) and *T* expression. Interestingly, the spatial separation of these markers was associated with a significant increase in the eccentricity of the structures (Fig. 6e, f,), while a correlation was found between the area occupied by the Sox1-positive cells and the length of the gastruloids (Fig. 4f). These observations suggest that the elongation of the structures results from a coordinated sequence of events starting with the segregation of the mesoderm and ectoderm. In addition, scRNA-seq analysis revealed that cell fate determination was linked to temporal progression (Inset Fig. 4d, Fig. 6g). For instance, mesoderm derivatives of NMPs gave rise to presomitic, somitic and pharyngeal mesoderms in F5, that further differentiate into dermomyotome, mesenchyme and craniofacial mesenchyme in M7 (Fig. 4d, Fig. 6c, 7). These observations were supported by the antagonistic expression of *Fgf8*/*Fgf17* and *Aldh1a2* in the presomitic mesoderm (Fig. 8a), reflecting the opposing FGF and RA signaling that determine the wavefront where cells begin to form somitomeres[35]. Accordingly, whole-mount *in situ* hybridization for *Uncx* confirmed somite segregation in ELS (Fig. 9a). Besides trunk mesodermal tissues (*Nkx1-2* positive cells), neural progenitor cells of the spinal cord emerged from NMPs by expressing *Sox1* and *Irx3*[36] (Fig. 8b), as previously described in gastruloids generated with a pulse of Wingless-type integration site protein (WNT) agonist8. However, our structures contained additional anterior neural cell types such as mid-hindbrain progenitors (cluster 16) recapitulating Wnt1/3a/9a expression patterns of roof plate organizers[37] (Fig. 8c, d). We also identified a cluster corresponding to radial glia cells which formed neural tube-like rosettes in Matrigel structures (Fig 9b, Fig. 64d, 7) similar to the neuroepithelium organization found in embryos[38]. *In vivo,* progenitors are located in the ventricular zone surrounding the lumen of the neural tube, whereas post-mitotic neurons migrate radially towards the cortical surface[39]. In our system, post-mitotic neurons were identified (cluster 18) by their strong enrichment in the $G_1/G_0$ phase of the cell cycle and the robust expression of post-mitotic neuronal markers (Fig. 6d, 7, 8e). Strikingly, post-mitotic neurons showed numerous Tuj1-positive neurites at the periphery of the ELS, whereas the midbrain marker Pax2 stained only the center along the A/P axis (Fig. 9c). Next, to investigate whether post-mitotic maturation was associated with neuronal diversity in cluster 18, we studied the neuronal cell fate determination occurring for class B dl4 progenitors in the spinal cord[40]. We characterized

two single-cell subclusters based on combinatorial expression of transcription factors and identified specific markers for GABAergic inhibitory interneurons on one hand, and glutamatergic excitatory interneurons on the other (Fig. 8f), suggesting the emergence of functionally distinct neuronal subtypes. Finally, Schwann cell precursors were identified (cluster 17) based on specific transcripts involved in neural crest development (Fig. 4d, Extended Data Fig. 6c, d, 7, Table 3). Remarkably, Sox10 staining in ELS revealed a sharp dorsoventral polarity along the A/P axis (Fig. 9d), reminiscent of the localization of dorsal root ganglia containing Sox10 positive-neural crest cells[41].

Collectively, our data show that ELS develop in space and time to specify regionalized tissues from the three germ layers recapitulating dynamic morphogen gradients to drive cell fate determination.

**Cumulative repressive marks alter *Nanog* expression**

To gain mechanistic insight into how transient hypoSUMOylations in ESCs generate 3 cell types, we performed scRNA-seq at D1, D3, D8 and D10 (Fig.1a). The XEN-L population identified in D18 spheroids appeared as soon as D8 (cluster 6), indicating that SUMOylation recovery was essential for XEN lineage commitment (Fig. 11a-e, Table 4). The first round of hypoSUMOylation (D3) yielded a large number of two-cell-stage-like (2C-L) cells (cluster 4), while the second (D10) resulted in a lesser induction, suggesting that the first round established an epigenetic memory impeding further emergence of 2C-L cells (Fig. 11a-c, e, f). Importantly, the totipotent-like state was dispensable for the formation of spheroids, since Dppa2/4 double knockout ESCs that are unable to convert into 2C-L cells[42] were still prone to generate spheroids (Fig. 11g). We thus focused on the other main group at D3/D10 named Hypo-ESCs (cluster 3), and found that the expression of components of the DNA methylation machinery was highly sensitive to the levels of SUMOylation (Fig. 10a, b, Fig. 11a, 12a). We hypothesized that the excess of Dnmt3a/3b/3l at D3/D10 in Hypo-ESCs paired with the decrease of the Tet1/2 enzymes would change the global pattern of DNA methylation. Genome-wide profiling revealed a gradual increase of the methylated CG fraction from D1 to D10 (Fig. 10c), indicating that SUMO in control ESCs is essential to prevent runaway methylation. Moreover, SUMOylation recovery between D3 and D8 was unable to revert the DNA methylation overload, in contrast to the full restoration observed for the repressive mark H3K9me3 (Fig. 12b). We sought a potential SUMO substrate controlling Dnmt3 expression at the chromatin level and identified Sall4, an important transcription factor for pluripotency maintenance[43], as a strong candidate (Fig. 12c, d). Knockdown of Sall4 reduced the induction of Dnmt3a upon ML-792 treatment, indicating that the effect of hypoSUMOylation on Dnmt3a expression is largely mediated by Sall4 (Fig. 12d, e). Of note, cells depleted for Sall4 showed a greater capacity to convert into 2C-L cells, suggesting that the high level of Dnmt3a under hypoSUMOylation prevents the establishment of the totipotent-like state (Fig. 12e).

The increase of DNA methylation was more pronounced in ESC enhancer regions, and correlated with a decreased transcription of neighboring genes including *Nanog, Esrrb* and *Tbx3* (Fig. 10d, Fig. 12f). Although *Nanog* was significantly diminished at both transcript and protein levels at D8, partly explaining why D1 and D8 cells did not fully cluster together, D8 cells still maintained their stemness (Fig. 11a, 12g-i, Table 5). To formally prove that the surplus of DNA methylation was responsible for the decrease of *Nanog* at D8, we treated ESCs with the hypomethylating drug 5-azacytidine for 5 days after the first wave of hypoSUMOylation, and confirmed that these cells were able to maintain a high level of *Nanog* (Fig. 10e). Importantly, spheroids were formed with a similar efficacy when the interval between hypoSUMOylation rounds was prolonged, highlighting that transcriptomic and epigenetic changes were stable over time (Fig. 12j).

Collectively, these data suggest that sequential waves of hypoSUMOylation progressively increase DNA methylation at pluripotency-associated genes, leading to their repression and favoring the expression of genes involved in tissue and embryo development (cluster 5, Fig. 11a, 12k).

As DNA methylation increases from D1 to D8, we hypothesized that the SUMO landscape may be altered after recovery from hypoSUMOylation. ChIP-seq profiling of SUMO1 identified 31,312 peaks, 924 of which were increased, and 403 decreased, at D8 compared to D1 (Fig. 10f, Table 6). SUMO peaks DOWN were enriched in intergenic regions, particularly at the transposable element L1Md repeat family (Fig. 10g, Fig. 13a-c). Further investigation is required to identify the SUMO substrate bound to these loci. For the SUMO peaks UP, the motif for the zinc finger protein Zfp57 was the highest scoring predicted site (Fig. 10g). Zfp57 plays a key role in maintaining DNA methylation imprints that silence genes depending on parental origin[44]. Accordingly, imprinted genes were found over-represented in genes associated with the SUMO peaks UP (Fig. 10f, Fig. 13d). SUMO peaks UP with more than two Zfp57 binding sites correlated with a higher level of DNA methylation when compared to all other SUMO peaks, suggesting that Zfp57 was recruited to these loci through its methylationsensitive binding (Fig. 13e). Moreover, genes associated with these peaks showed strong expression oscillations depending on SUMO levels (Fig. 13f). Although DNA methylation levels at SUMO peaks did not correlate with the variation of the SUMO signal, we identified a genomic region upstream of the *Nanog* promoter that was both hyperSUMOylated and hypermethylated at D8 (Fig. 10h, Fig. 13g). This locus contains a series of Zfp57 motifs, and we confirmed increased Zfp57 binding and the recruitment of Kap1/Setdb1 which deposit H3K9me3 at the *Nanog* locus at D8 (Fig. 10i, Fig. 13h). Of note, SUMOylation of Kap1 was reported to trigger recruitment of Setdb1

in ESCs[45] and may therefore be considered the best SUMO substrate responsible for the SUMO peaks UP. According to our model (Fig. 13i), the waves of hypoSUMOylation destabilize the core pluripotency network, promoting a shift towards an EPI-L state in the heterogenous ESC metastable population[46,47].

| **Spheroids** | **% cells** | **Fluidic gastruloid** | **% cells** | **Flu-Mg Embryo-like** | **% cells** |
|---|---|---|---|---|---|
| ES-L | 65.9 | ES-L | 51.2 | ES-L | 7.3 |
| EPI-L | 29.6 | EPI-L | 0.3 | EPI-L | 0 |
| XEN-L | 4.5 | Primitive streak | 2.2 | Primitive streak | 0 |
| **AggreWell gastruloid** | **% cells** | NMPs | 13.3 | NMPs | 0.4 |
| ES-L | 49.5 | Presomitic meso. | 5.7 | Presomitic meso. | 0 |
| EPI-L | 37.3 | Somitic meso. | 10.4 | Somitic meso. | 0.15 |
| XEN-L | 0.3 | Pharyngeal meso. | 5.9 | Pharyngeal meso. | 0.15 |
| Primitive streak | 6.7 | Def. Endo / Gut | 1.1 | Def. Endo / Gut | 0.4 |
| Definitive endoderm | 0.5 | Radial Glia | 6.2 | Radial Glia | 0.6 |
| NMPs | 2.8 | Demomyotome | 0.1 | Demomyotome | 10.5 |
| Neuroepithelium | 2.9 | Mesenchyme | 1.4 | Mesenchyme | 25.7 |
| | | Craniofacial mes. | 0.06 | Craniofacial mes. | 13.7 |
| | | Endothelium | 0.09 | Endothelium | 1.1 |
| | | Cardiomyocytes | 0 | Cardiomyocytes | 1 |
| | | Spinal cord | 0.06 | Spinal cord | 7.9 |
| | | Mid-Hindbrain | 0 | Mid-Hindbrain | 21.1 |
| | | Schwann cell prec. | 0 | Schwann cell prec. | 3.7 |
| | | Neurons | 2 | Neurons | 6.3 |

It is noted that when spheroids were prepared using TAK-981 as the SUMO E1 inhibitor they exhibited the same properties as those obtained with ML-792 treatment which are disclosed in the present examples:

- Decrease of pluripotency markers *Nanog, Essrb*
- Emergence of an ESC-L cell type expressing *Dppa3*
- Emergence of a XEN-L cell type expressing *Sox17, Gata4, Snai1*
- Emergence of an EPI-L cell type expressing *Pou3f1, Fgf5, Wnt3*

**DISCUSSION**

Here, we describe a new strategy to mimic mouse embryo development in absence of exogenous morphogens, that relies on transient waves of hypoSUMOylation to generate, from sole ESCs, self-organized ELS with all three germ layers. Crosstalk between the spheroid cell types is sufficient to trigger a series of morphogenetic events characteristic of natural gastrulation and early organogenesis that enable the emergence of additional cell populations regionalized in complex structures. However, important cell types from various stages of development are missing, including PGCs and forebrain tissue. Future work is needed to optimize culture conditions, such as adding a WNT agonist or using rotating culture platforms, to improve embryo-like morphology[4,48]. Importantly, D18 spheroids are stable after freeze/thaw cycles, allowing generation of ELS in only 7 days with one culture medium. Our protocol is thus highly scalable and adaptable. The added value of the droplet-microfluidic platform in boosting lineage diversity requires further investigation to determine whether this system modifies the physicochemical microenvironment (e.g. pH gradient) to favor axial elongation of gastruloids similar to natural embryos[49].

Brief suppression of SUMOylation both decreased the heterogeneous expression of *Nanog* and increased the global level of DNA methylation, consistent with events of the peri-implantation stage[50,51]. This could suggest that a physiological wave of hypoSUMOylation may occur at gastrulation to facilitate cell fate determination, as demonstrated at the 2C-stage[24,25].

Finally, adjusting this protocol for human ESCs may provide new insights into early stages of development and the role of SUMO as a barrier to cell fate change. Overall, our work lays foundations for exploring epigenetic drugs as tools to control the balance between cell fate robustness and lineage commitment, with the ultimate goal of reconstructing complex multicellular architecture.

**Gastruloid structure for therapy of spinal cord injury**

Traumatic spinal cord injury (SCI) is a devastating condition that often leads to significant life-long functional impairments, increased death rates, and huge costs in social and financial terms for patients and their families. The estimated annual global incidence is 40 to 80 cases per million population, meaning that approximatively three million people live with SCI, with 250,000 new cases each year. Disabilities may include partial or complete loss of sensory function or motor control of arms, legs and/or body and affect bowel or bladder control, breathing, heart rate, and blood pressure. Thus, SCI may render a person dependent on caregivers and assistive technology is often required to facilitate mobility, communication and selfcare. Depression, related to SCI, has a negative impact on improvements in functioning and overall health. Children with SCI are less likely than their peers to start school while adults with SCI face similar barriers to economic participation, with a global unemployment rate >60%. To date, the only available treatment options include surgical stabilization and decompression of the spinal cord, and rehabilitative care, whereas the only approved pharmacological approach is the administration of high-dosed methylprednizolone, despite serious concerns.

Although specialized medical and surgical care have reduced mortality, novel and effective therapies that would confer long-term functional improvement/recovery represent an unmet clinical need. Cell transplantation is among the most promising strategies to promote repair and several early phase clinical trials have shown its feasibility. Candidate cell types may exert neuroprotective and/or neurodegenerative roles. For example, neural stem cells engraftment provides cell replacement of lost neurons, astrocytes, oligodendrocytes and growth factor support; Schwann cells and their precursors can support axon regeneration and remyelination after injury and also produce a variety of growth factors, mesenchymal stem cells may play an immunomodulatory and neuroprotecting role. Evidently, the application of a singledimensional approach has failed to lead to recovery and co-transplantation of different cell types has presented with significant added therapeutic value.

Here, the inventors propose to graft embryo-like structures obtained using the methods and device of the invention that contain all three embryonic germ layer lineages in a mouse model for SCI. the above reported data show that these structures have the potential of forming distinct spinal cord neuronal precursors, Schwann cell precursors as well as other important elements including cells of the vasculature. The designed approach is expected to present several advantages over current protocols for SCI therapy, by combining the ameliorating effects of multiple cell types and the positive association of growth factors released.

The inventors' main objective is to evaluate the therapeutic benefit of embryo-like structures transplantation in a mouse model of dorsal column crush. At early stages after transplantation, the survival, integration and migration of grafted cells should be determined by immunohistochemistry, followed by assessing motor function recovery and tissue repair at later stages. In addition, the cutting-edge strategy of single-nucleus transcriptome analysis of grafted structures and host spinal cord tissue to identify lineage commitment of the grafted cells and also determine paracrine factors with potential therapeutic value should then be used.

Within this context evaluating whether and how the transplantation of the cellular structures developed may promote neural tissue repair and functional improvement in a mouse model of SCI, would allow reaching the ultimate goal of transposing this approach for translation purposes. The novel type of embryo-like structures of the invention comprises (embryoids) and/or can give rise (gastruloids) to a variety of cell populations essential for recovery after SCI, e.g., neural precursors, neurons (ectoderm), Schwann cells (neural crest), endothelial cells (mesoderm), etc. In addition, these structures are expected to secrete a unique ECM template as well as a broad range of soluble signaling molecules that may favor SCI recovery. Embryoids, when compared to gastruloids, contain a number of more mature cell types that have lost their full cell plasticity. Therefore, implanting gastruloids may be preferred since they have the potential to generate neural and endothelial lineages, albeit devoid of undesirable cell types such as cardiomyocytes or gut progenitors. The inventors hypothesize that the direct transplantation of mESC-derived gastruloids will provide various types of precursor cells capable of promoting regeneration after SCI. they anticipate that the lesioned CNS microenvironment will supply molecular cues to instruct maturation of the grafted gastruloids into essential cell types that will participate in the repair mechanisms both in terms of cell replacement and of sourcing growth promoting molecules. By leveraging the ameliorating effects of multiple cell types, growth factors, ECM molecules and tissue bridging potential, this approach thus represents an innovative combinatorial strategy expected to remedy some of the shortcomings of current protocols for SCI therapy.

**Microfluidic device**

In reference to figures 14 to 18, the microfluidic device 100 is provided for manipulating droplets and allowing cell culture. The example of a microfluidic device 100 as shown in the figures, comprises a plurality of identical traps 102 arranged in a body 101. The microfluidic device 100 further comprises a plate 103 bounded to the bottom side 116 to close the channel 114.

On the drawings, it can be seen that the microfluidic device comprises an annular rim 105 onto which the plate 103 is bounded.

The traps 102 are distributed on a plurality of parallel columns. Preferably, the columns of traps are arranged in a staggered pattern. The body 101 may be made of polymer such as polydimethylsiloxane (PDMS). The body 101 presents a parallelepiped shape and a thickness comprised from 0.8 to 1.2 mm, in particular equal to 1 mm.

The body comprises a first longitudinal axis L corresponding to the direction of fluid flow, a second transverse axis T and third axis corresponding to the thickness Z of the body.

Each trap 102 comprises a cavity, extending along an axis of revolution X100, the cavity being intended to house at least a droplet introduced in the microfluidic device 100. Each trap 102 comprises an opening 106 opening out in a channel 114. The channel 114 is formed by a recess in a bottom side 116 of the body 101.

Each trap may be substantially perpendicular to the top 117 and bottom 116 sides of the microfluidic device 100.

Each trap 102 comprises a first part 104 of the cavity arranged between the opening 116 and a second part 108 of said trap 102, along the axis of revolution X100. The first part 104 of the cavity presents a dimension d1 along the axis of revolution X100 (i.e. the height of the first part) at least five times smaller than a dimension d2 of the second part 108 of the cavity along the axis of revolution X100 (i.e. the height of the second part). The dimension d1 of the first part 104 of the cavity is comprised from 1 to 1.4 mm, in particular equal to 1.2 mm. The dimension d2 of the second part 108 of the cavity is comprised from 2 to 3.5 mm, in particular equal to 3 mm.

The first part 104 of the cavity is advantageously delimited by an annular curved wall 110 having a curvature R1 = 0.5 mm. The annular wall 110 is convex with regard to the axis of revolution X100. The cross section of the first part 104 of the cavity in a transverse plane perpendicular to the axis of revolution X100 is circular. The curvature R1 of the annular wall 110 increases towards the opening 106.

The opening 106 is also circular and presents a diameter Ø1 comprised from 2 to 3 mm, in particular from 2.2 to 2.6 mm, in particular equal to 2.4 mm.

The second part 108 of the cavity is delimited by a cylindrical wall 112 presenting a hexagonal cross section in the transverse plane. The hexagonal cross section of the second part 108 of the cavity presents an inscribed circle of a diameter Ø2 comprised from 1 to 2 mm, in particular comprised from 1.1 to 1.3 mm, in particular equal to 1.2 mm.

The diameter Ø1 of the opening 106 is greater than the diameter Ø2 of the hexagonal cross section.

Each trap 102 may comprise a third part 118 arranged at an end of the trap 102 opposite to the opening 106. The third part 118 is delimited by concave wall 120 forming a dome.

In an embodiment, each trap presents a dimension h2 along the axis of revolution comprised from 2 to 6 mm, in particular from 3 to 5 mm, in particular equal to 4mm. h2 corresponds to the total height of the trap, i.e. the first, the second and the optional third parts. The dimension h2 is determined such as to allow the contact between the first droplet and the second droplet which leads to the fusion of the droplets thus forming a larger droplet as will be shown in the detailed description.

The channel 114 extends in a plane parallel to the bottom side 116 of the body 101 according to a hexagonal shape and covers all the openings 106 of the traps 102. A dimension h1 of the channel according to the axis of revolution X100 (i.e. the height of the channel) is comprised from 0.5 to 2mm, in particular equal to 1mm.

The diameter Ø1 of the opening 106 of a trap is at least equal or two times greater than the dimension h1 of the channel. The channel 114 connects an inlet 122 to a plurality of outlets 124 of the microfluidic device 100. The inlet 122 is arranged at a first end on a top side 117 of the body 101 opposite to the bottom side 116 in the direction of the axis of revolution X100. The inlet 122 is fluidically connected to the channel 114 by a duct 123 which forms an angle $\delta$, with respect to the bottom side 116, comprised from 30° to 60°, in particular equal to 45°. The opening of the duct 123 in the channel 114 is advantageously delimited by an annular curved wall having a curvature R2. The annular curved wall is convex with regard to the axis along the length of the duct. The cross section of the opening of duct 123 in a transverse plane perpendicular to the axis along the length of the duct is circular. The curvature R2 of the annular wall increases towards the opening in the channel 114.

The fluid flows from the inlet 122 through the channel 124 to the outlets 124. In the direction of fluid flow, the device comprises a first end 122A located at the inlet 122 and a second end 124A located at the outlets 124.

The outlets 124 open out on the top side 117 of the body 101 and are arranged at a second end of the top side 117 opposite to the first end along the length of the body 101. The outlets 124 are connected to the channel 114 through respective vertical ducts.

Besides, the channel 114 is provided with four guiding rails 126 arranged for uniformly distributing the droplets

outputted from the duct 123 in the channel 114. Each rail 126 presents a first end arranged next to the opening of the duct 123 in the channel 114. Each rail 126 presents a second end arranged next to a column of traps. Each rail 126 is a groove in the top surface of the channel 114 presenting a depth h3 smaller than two times the dimension h1 of the channel 114 along the axis of revolution X100, in particular equal to 0.5 mm.

Advantageously, the microfluidic device 100 comprises 81 traps distributed along 9 columns. However, the microfluidic device 100 may comprise any number of traps from 2 to 100.

The distance from the axis of revolution X100 of two adjacent traps 102 is comprised from 5 to 10 mm, in particular from 7 to 9 mm, and in particular equal to 8 mm.

In a particular embodiment illustrated in figure 22A, the microfluidic device 100 comprises a plurality of inlets and a plurality of outlets. In the illustrated embodiment, the body 101 of the microfluidic device 100 comprises three inlets and three outlets. It comprises a primary inlet 122a, two secondary inlets 122b, 122c, a primary outlet 124a aligned longitudinally with the primary inlet 122a and two secondary outlets 124b, 124c each aligned longitudinally with a secondary outlet 122b, 122c. The inlets 122a, 122b, 122c may be spaced, in the first direction, by a distance substantially equal to the distance spacing the outlets 124a, 124b, 124c in the second direction. In this manner, the liquid entering one inlet tends to flow towards the outlet that is aligned with the inlet according to the first longitudinal direction.

The microfluidic device of figure 22A may be used as follows. The inlets are connected to syringes containing the different aqueous solutions, which are then continuously perfused at the same flow rate (1000 μL/min). Under this perfusion regime (high Peclet number, i.e. Peclet number (Pe) higher than 1 in particular Pe is equal or higher than 10), the different solutions are not mixing, which allows the different rows of traps to be exposed to different experimental conditions. This allows to obtain the perfusion diagram illustrated in figure 22B where we observe the presence of three distinct zones Ia, Ib, and Ic. The first zone Ia is obtained by the perfusion of a first liquid into the primary inlet 122a to the primary outlet 124a. The second zone Ib is obtained by the perfusion of a second liquid into the secondary inlet 122b to the secondary outlet 124b. The third zone Ic is obtained by the perfusion of a third liquid into the secondary inlet 122c to the secondary outlet 124c.

**Fabrication of the microfluidic device**

The microfluidic device 100 may be fabricated by molding. The process of fabrication of the microfluidic device 100 may comprise the following steps:

- providing a mold presenting mold imprints of the traps,
- filling the mold with a mixture of PDMS base and a curing agent at a ratio of 1:10, for example for about 50-60 mL,
- placing the mold in an oven set up at 65°C, for at least 4 hours,
- separating the resulting body 101 from the mold,
- bounding, by plasma, the bottom side 116 of the body 101 to a glass slide, for two rounds of 40 seconds,
- placing the resulting microfluidic device 100 into an oven set up at 80°C, for at least 2 hours,
- coating the microfluidic device 100, to be rendered fluorophilic, with an appropriate fluorophilic coating such as NovecTM 1720 (3M) and heating the microfluidic device 100 at 110°C, for three rounds.

The glass slide may be a 75 x 50 mm rectangular.

**Method of manipulating droplets using the microfluidic device**

An example of use of the microfluidic device 100 is shown in figures 19 and 20, for manipulating droplets. In a first step S101, a first liquid composition comprising first droplets is introduced in the channel 114 through inlet 122. The first droplets hold cells. Advantageously, during the introduction of the first droplets, the microfluidic device 100 is tilted at a first angle with respect to a horizontal axis 125, as shown in figure 20, the first angle A1 being comprised from 30° and 60°, in particular comprised from 40° and 50°, in particular equal to 45°. The microfluidic device is tilted such that the outlets 124 of the microfluidic device are above said horizontal axis 125 while the inlet of the microfluidic device is under said horizontal axis. In other words, the manipulation of the microfluidic device 100 is processed so as to maintain the first end 122A in contact with a horizontal support and the second end 124A is separated from the support, the microfluidic device 100 being in a tilted position since the plate 103 forms an angle with a planar surface of the support. The first angle promotes gravity forces in order to allow the droplets' motion inside the channel.

Consequently, the first part 104 of the cavity of a trap 102 traps a first droplet 202 by capillarity.

In a following step S102, the first droplet 202 migrates to the second part 108 of the cavity by buoyancy after a given time.

Following step S102, a second liquid composition comprising second droplets may be introduced in the microfluidic device 100 in a step S103. Advantageously, during the introduction of the second droplets, the microfluidic device

100 is tilted at a second angle with respect to the horizontal axis, comprised from 25° and 35°, in particular equal to 25°. The microfluidic device is tilted such as the outlets of the microfluidic device are above the horizontal axis while the inlet of the microfluidic device is below the horizontal axis. Consequently, the first part 104 of the cavity of the same trap 102 traps a second droplet 204 by capillarity. The second angle helps controlling the gravity forces in order to favor a second drop trapping in the anchors of the first part 104 of the cavity by capillary forces.

The dimension h2 allows the contact from the first droplet 202 and the second droplet 204 which leads to the fusion of the droplets 202 and 204 and forms a larger droplet 206 as shown in step S104.

Each first droplet 202 is introduced as a plug of aqueous phase having a volume comprised from 5 μL and 10 μL, in particular equal to 7 μL. The first liquid composition comprises also plugs of a fluorogenic oil having a volume comprised from 5 μL and 10 μL, in particular equal to 6 μL. The plugs of fluorogenic oil separates the first droplets 202. The fluorogenic oil contains a fluorogenic surfactant at 0.5% of the total weight.

After the migration of the first droplet 202 in the second part 108 of the cavity, the microfluidic device 100 can be placed in an incubator set up at 37°C and 5% of $CO_2$ to allow cell culture. The cross section of the second part 108 of the cavity promotes shaping the first droplet 202 in a spheroid shape.

The second droplets 204 are soluble molecules such as culture medium, dyes, or a biomaterial.

In some embodiments, before the introduction of the second liquid composition, an immiscible fluorocarbon oil without surfactant or containing PFO (Perfluoro-Octanol (PFO), which reduces the emulsion stability) at concentration of 20% may be flushed in the traps. The immiscible fluorocarbon oil allows the fusion of the two droplets 202 and 204.

The first liquid composition and/or the second liquid composition are introduced in the microfluidic device at a flow of 1000 μL/min.

The use of the microfluidic device has been illustrated in the experimental data reported on figures 4 and 5. The inventors have in particular been able to provide that by comparison with conventional 96-well plates the culture of mESC-D3 in immobilized drops yields a faster aggregation kinetic, similar degree of expansion and similar level of expression of pluripotency markers (OCT-4 or SSEA-1), albeit the culture volume has been reduced by 15 folds (i.e. 100 μL vs. 7 μL). Volume reduction without altering cell functions may have important implications to study in particular the role of para/autocrine signalling.

In some embodiments, the first and the second droplets comprise distinct media and are used to provide hydrogel encapsulation of embryo-like structures. For illustration it is described that:

- the first droplets comprise cells, especially dissociated spheroid cells as obtained according to the embodiments herein disclosed, in a liquid medium and such cells are anchored in the traps of the microfluidic device, in particular at the top of the traps,
- the second droplets comprise Matrigel or an equivalent matrix diluted in cell culture medium wherein the second droplets are trapped at the bottom of the traps that contain the first droplets
- the first and the second droplets are allowed to fuse giving rise to a large drop containing cells in Matrigel or an equivalent matrix and the hydrogel is allowed to gelify.

More generally the drop/droplet manipulation according to the invention, and the use of the droplet manipulation device of the invention provides the following advantages and improvements over the art:

- The droplet manipulation device provides the minimal reported volume to promote expansion and differentiation of PSCs, while offering a higher degree of throughput per surface area than conventional 96 wells.
- The invention provides the first reported integrated platform for the culture, differentiation and characterization of 3D ESC/PSC culture into immobilized drops.
- As demonstrated in the results, the immobilized droplets provide a unique microenvironment to regulate the fate decision of pluripotent stem cells in 3D.
- Droplet formation and immobilization can easily be automated, providing an easier handling and/or a reduced need for robotic systems than 96, 384 and 1534 well plates.
- The immobilized droplets in the device are less prone to evaporation than 1584 well plates that make use of similar working volumes.
- The device enables to temporally control the culture microenvironment, by droplet fusion. The device can be applied for the derivation of biomaterials for encapsulation or the screening of small molecules (including teratogenic drugs) to regulate the differentiation of ESCs/PSCs or for teratotoxicity studies.

- Beyond pluripotent stem cells, the device was demonstrated to uniquely support the culture of adult neural progenitor cells, thus it opens the way for the screening of neurogenic/neurotoxic molecules.

- After mechanical immobilization into anchors using hydrogel and performing oil-to-medium phase change, the device allows the application of controlled feeding/perfusion strategies (e.g. to allow periodic stimulation etc.) for further embryoid maturation (i.e. controlling fluid dynamics).

**Method of using droplets for hydrogel encapsulation and long-term selective perfusion of embryo-like structures (ELS) on the microfluidic device**

The purpose was to use the technique of droplet fusion as disclosed herein wherein the second droplet contains liquid Matrigel in order to encapsulate embryo-like structures (ELS) within a hydrogel.

The following protocol was performed.

**On Day-0:** Dissociated spheroid cells (obtained through the hypoSUMOylation protocol of mouse ESCs) were encapsulated in anchored liquid droplets as previously described. The cells aggregated then formed gastruloids over a 4-day period.

**On Day-4:** The chips containing gastruloids in drops were cooled down on ice **(Figure 21.A).** The chips were perfused with about 4 mL FC-40, then with a 4 mL solution of Fluorinert™ HFE-7500 containing 20% PFO (PerFluoroOctanol). Then, second 7 μL droplets containing Matrigel were formed using the Upchurch cross-junction by delivering 7 μL plugs of 40% Matrigel diluted in culture medium flowed at 1000 μL/mL that were separated by a 6 μL plug of Fluorinert™ HFE-7500 containing 20% PFO, also flowed at 1000 μL/mL. The drops were trapped at the bottom of the anchors, which already contained a drop containing the gastruloids that were trapped in the top of the traps **(Figure 21.B).** Thereafter, about 4 mL Fluorinert™ HFE-7500 containing 20% PFO, then FC-40 were flown in the culture chamber. The two drops were allowed to fuse, which resulted in a large drop containing 20% Matrigel, and the hydrogel was left to gelify until Day-5, in a $CO_2$ incubator set up at 37 °C **(Figure 21.C).**

**On Day-5:** The oil phase surrounding the gelified drops was exchanged for culture medium, simply by filling about 4 mL of medium through the chip inlet.

**Day-7** to **Day-19:** The medium was changed every 2 days simply by filling about 4 mL of fresh medium through the chip inlet.

After oil-to-medium phase exchange, at least three different aqueous solutions of various chemical compositions could be perfused on a single chip. For this purpose, one outlet (in red) was placed in front of the middle group of traps (3 lanes in the example), and two other inlets (in green) were placed symmetrically to two other outlets (in blue, **Figure 22.A).** The inlets were connected to syringes containing the different aqueous solutions, which were then continuously perfused at the same flow rate (1000 μL/min). Note that under this perfusion regime (high Peclet number), the different solutions were prevented from mixing, which allowed the different rows of traps to be exposed to different experimental conditions **(Figure 22.B**).

<u>Results</u>

- Embryoids obtained after Matrigel droplet fusion in the device **(Figure 23)** compared to Matrigel embedding in 96-well plates -with culture carried out for 2 days - exhibited:

  - Small increase of cardiomyocytes marker (*Tnnt2*)
  - Expression of *Noto* and *Shh*
  - Expression of forebrain markers (*Foxg1, Emx1*)

- Embryoids obtained after Matrigel droplet fusion in the device - with culture carried out for 7 or 14 days - exhibited:

  - Very strong increase of cardiomyocytes marker (*Tnnt2*)
  - Expression of ventral somites markers (*Pax1, Pax9*)
  - Expression of ventral spinal cord marker (*Vsx2*).

According to the invention, Matrigel droplet fusion directly in the device to prepare Embryoids allows direct perfusion of fresh medium while preserving the Matrigel. As a consequence, the obtained structures can continue growing.

**REFERENCES**

1. Rivron, N. C. et al. Blastocyst-like structures generated solely from stem cells. Nature 557, 106-111 (2018).
2. Sozen, B. et al. Self-assembly of embryonic and two extra-embryonic stem cell types into gastrulating embryo-like structures. Nat Cell Biol 20, 979-989 (2018).
3. Zhang, S. et al. Implantation initiation of self-assembled embryo-like structures generated using three types of mouse blastocyst-derived stem cells. Nat Commun 10, 496 (2019).
4. Turner, D. A. et al. Anteroposterior polarity and elongation in the absence of extraembryonic tissues and spatially localised signalling in Gastruloids , mammalian embryonic organoids. Development dev.150391 (2017) doi:10.1242/dev.150391.
5. Beccari, L. et al. Multi-axial self-organization properties of mouse embryonic stem cells into gastruloids. Nature 562, 272-276 (2018).
6. Moris, N. et al. An in vitro model of early anteroposterior organization during human development. Nature 582, 410-415 (2020).
7. van den Brink, S. C. et al. Single-cell and spatial transcriptomics reveal somitogenesis in gastruloids. Nature 582, 405-409 (2020).
8. Veenvliet, J. V. et al. Mouse embryonic stem cells self-organize into trunk-like structures with neural tube and somites. Science 370, eaba4937 (2020).
9. Li, R. et al. Generation of Blastocyst-like Structures from Mouse Embryonic and Adult Cell Cultures. Cell 179, 687-702.e18 (2019).
10. Yu, L. et al. Blastocyst-like structures generated from human pluripotent stem cells. Nature 591, 620-626 (2021).
11. Liu, X. et al. Modelling human blastocysts by reprogramming fibroblasts into iBlastoids. Nature 591, 627-632 (2021).
12. Girgin, M. U., Broguiere, N., Mattolini, L. & Lutolf, M. P. Gastruloids generated without exogenous Wnt activation develop anterior neural tissues. Stem Cell Reports 16, 1143-1155 (2021).
13. Zheng, Y. et al. Controlled modelling of human epiblast and amnion development using stem cells. Nature 573, 421-425 (2019).
14. Cubenas-Potts, C. & Matunis, M. J. SUMO: a multifaceted modifier of chromatin structure and function. Dev Cell 24, 1-12 (2013).
15. Hendriks, I. A. & Vertegaal, A. C. A comprehensive compilation of SUMO proteomics. Nat Rev Mol Cell Biol (2016) doi:10.1038/nrm.2016.81.
16. Theurillat, I. et al. Extensive SUMO Modification of Repressive Chromatin Factors Distinguishes Pluripotent from Somatic Cells. Cell Reports 32, 108146 (2020).
17. Liu, H. W. et al. Chromatin modification by SUMO-1 stimulates the promoters of translation machinery genes. Nucleic Acids Res 40, 10172-86 (2012).
18. Neyret-Kahn, H. et al. Sumoylation at chromatin governs coordinated repression of a transcriptional program essential for cell growth and proliferation. Genome Res 23, 1563-1579 (2013).
19. Niskanen, E. A. et al. Global SUMOylation on active chromatin is an acute heat stress response restricting transcription. Genome Biol 16, 153 (2015).
20. Seifert, A., Schofield, P., Barton, G. J. & Hay, R. T. Proteotoxic stress reprograms the chromatin landscape of SUMO modification. Sci Signal 8, rs7 (2015).
21. Cheloufi, S. et al. The histone chaperone CAF-1 safeguards somatic cell identity. Nature 528, 218-24 (2015).
22. Borkent, M. et al. A Serial shRNA Screen for Roadblocks to Reprogramming Identifies the Protein Modifier SUMO2. Stem Cell Reports 6, 704-16 (2016).
23. Cossec, J.-C. et al. SUMO Safeguards Somatic and Pluripotent Cell Identities by Enforcing Distinct Chromatin States. Cell Stem Cell 23, 742-757.e8 (2018).
24. Higuchi, C., Yamamoto, M., Shin, S.-W., Miyamoto, K. & Matsumoto, K. Perturbation of maternal PIASy abundance disrupts zygotic genome activation and embryonic development via SUMOylation pathway. Biology Open bio.048652 (2019) doi:10.1242/bio.048652.
25. Yan, Y.-L. et al. DPPA2/4 and SUMO E3 ligase PIAS4 opposingly regulate zygotic transcriptional program. PLoS Biol 17, e3000324 (2019).
26. He, X. et al. Probing the roles of SUMOylation in cancer cell biology by using a selective SAE inhibitor. Nat Chem Biol 13, 1164-1171 (2017).
27. Mohammed, H. et al. Single-Cell Landscape of Transcriptional Heterogeneity and Cell Fate Decisions during Mouse Early Gastrulation. Cell Rep 20, 1215-1228 (2017).
28. Pijuan-Sala, B. et al. A single-cell molecular map of mouse gastrulation and early organogenesis. Nature 566,

490-495 (2019).
29. Graham, S. J. L. & Zernicka-Goetz, M. The Acquisition of Cell Fate in Mouse Development. in Current Topics in Developmental Biology vol. 117 671-695 (Elsevier, 2016).
30. Ben-Haim, N. et al. The Nodal Precursor Acting via Activin Receptors Induces Mesoderm by Maintaining a Source of Its Convertases and BMP4. Developmental Cell 11, 313-323 (2006).
31. Sart, S., Tomasi, R. F.-X., Amselem, G. & Baroud, C. N. Multiscale cytometry and regulation of 3D cell cultures on a chip. Nat Commun 8, 469 (2017).
32. Sart, S. et al. Mapping the structure and biological functions within mesenchymal bodies using microfluidics. Sci. Adv. 6, eaaw7853 (2020).
33. Tomasi, R. F.-X., Sart, S., Champetier, T. & Baroud, C. N. Individual Control and Quantification of 3D Spheroids in a High-Density Microfluidic Droplet Array. Cell Reports 31, 107670 (2020).
34. Cao, J. et al. The single-cell transcriptional landscape of mammalian organogenesis. Nature 566, 496-502 (2019).
35. Chal, J. & Pourquié, O. Making muscle: skeletal myogenesis in vivo and in vitro. Development 144, 2104-2122 (2017).
36. Gouti, M. et al. A Gene Regulatory Network Balances Neural and Mesoderm Specification during Vertebrate Trunk Development. Developmental Cell 41, 243-261.e7 (2017).
37. La Manno, G. et al. Molecular architecture of the developing mouse brain. http://biorxiv.org/lookup/doi/10.1101/2020.07.02.184051 (2020) doi:10.1101/2020.07.02.184051.
38. Shi, Y., Kirwan, P., Smith, J., Robinson, H. P. C. & Livesey, F. J. Human cerebral cortex development from pluripotent stem cells to functional excitatory synapses. Nat Neurosci 15, 477-486 (2012).
39. Penisson, M., Ladewig, J., Belvindrah, R. & Francis, F. Genes and Mechanisms Involved in the Generation and Amplification of Basal Radial Glial Cells. Front. Cell. Neurosci. 13, 381 (2019).
40. Hernandez-Miranda, L. R., Müller, T. & Birchmeier, C. The dorsal spinal cord and hindbrain: From developmental mechanisms to functional circuits. Developmental Biology 432, 34-42 (2017).
41. Britsch, S. The transcription factor Sox10 is a key regulator of peripheral glial development. Genes & Development 15, 66-78 (2001).
42. Eckersley-Maslin, M. et al. Dppa2 and Dppa4 directly regulate the Dux-driven zygotic transcriptional program. Genes Dev. 33, 194-208 (2019).
43. Zhang, J. et al. Sall4 modulates embryonic stem cell pluripotency and early embryonic development by the transcriptional regulation of Pou5f1. Nat Cell Biol 8, 1114-1123 (2006).
44. Li, X. et al. A Maternal-Zygotic Effect Gene, Zfp57, Maintains Both Maternal and Paternal Imprints. Developmental Cell 15, 547-557 (2008).
45. Ivanov, A. V. et al. PHD domain-mediated E3 ligase activity directs intramolecular sumoylation of an adjacent bromodomain required for gene silencing. Mol Cell 28, 823-37 (2007).
46. Hayashi, K., Lopes, S. M. C. de S., Tang, F. & Surani, M. A. Dynamic Equilibrium and Heterogeneity of Mouse Pluripotent Stem Cells with Distinct Functional and Epigenetic States. Cell Stem Cell 3, 391-401 (2008).
47. Torres-Padilla, M.-E. & Chambers, I. Transcription factor heterogeneity in pluripotent stem cells: a stochastic advantage. Development 141, 2173-2181 (2014).
48. Aguilera-Castrejon, A. et al. Ex utero mouse embryogenesis from pre-gastrulation to late organogenesis. Nature 593, 119-124 (2021).
49. Oginuma, M. et al. Intracellular pH controls WNT downstream of glycolysis in amniote embryos. Nature 584, 98-101 (2020).
50. Chambers, I. et al. Functional Expression Cloning of Nanog, a Pluripotency Sustaining Factor in Embryonic Stem Cells. Cell 113, 643-655 (2003).
51. Zhang, Y. et al. Dynamic epigenomic landscapes during early lineage specification in mouse embryos. Nat Genet 50, 96-105 (2018).
52. Klimanskaya, I. et al. Human embryonic stem cell lines derived from single blastomeres Nature 444, 481-485 (2006).
56. Deluz, C.; Friman, E. T.; Strebinger, D.; Benke, A.; Raccaud, M.; Callegari, A.; Leleu, M.; Manley, S.; Suter, D. M. A Role for Mitotic Bookmarking of SOX2 in Pluripotency and Differentiation. Genes Dev 2016, 30 (22), 2538-2550. https://doi.org/10.1101/gad.289256.116.
57. Eckersley-Maslin, M.; Alda-Catalinas, C.; Blotenburg, M.; Kreibich, E.; Krueger, C.; Reik, W. Dppa2 and Dppa4 Directly Regulate the Dux-Driven Zygotic Transcriptional Program. Genes Dev. 2019, 33 (3-4), 194-208. https://doi.org/10.1101/gad.321174.118.
58. Semrau, S.; Goldmann, J. E.; Soumillon, M.; Mikkelsen, T. S.; Jaenisch, R.; van Oudenaarden, A. Dynamics of Lineage Commitment Revealed by Single-Cell Transcriptomics of Differentiating Embryonic Stem Cells. Nat Commun 2017, 8 (1), 1096. https://doi.org/10.1038/s41467-017-01076-4.
59. Jaitin, D. A.; Kenigsberg, E.; Keren-Shaul, H.; Elefant, N.; Paul, F.; Zaretsky, I.; Mildner, A.; Cohen, N.; Jung,

S.; Tanay, A.; Amit, I. Massively Parallel Single-Cell RNA-Seq for Marker-Free Decomposition of Tissues into Cell Types. Science 2014, 343 (6172), 776-779. https://doi.org/10.1126/science.1247651.
60. Keren-Shaul, H.; Kenigsberg, E.; Jaitin, D. A.; David, E.; Paul, F.; Tanay, A.; Amit, I. MARS-Seq2.0: An Experimental and Analytical Pipeline for Indexed Sorting Combined with Single-Cell RNA Sequencing. Nat Protoc 2019, 14 (6), 1841-1862. https://doi.org/10.1038/s41596-019-0164-4.
61. Hao, Y.; Hao, S.; Andersen-Nissen, E.; Mauck, W. M.; Zheng, S.; Butler, A.; Lee, M. J.; Wilk, A. J.; Darby, C.; Zager, M.; Hoffman, P.; Stoeckius, M.; Papalexi, E.; Mimitou, E. P.; Jain, J.; Srivastava, A.; Stuart, T.; Fleming, L. M.; Yeung, B.; Rogers, A. J.; McElrath, J. M.; Blish, C. A.; Gottardo, R.; Smibert, P.; Satija, R. Integrated Analysis of Multimodal Single-Cell Data. Cell 2021. https://doi.org/10.1016/j.cell.2021.04.048.
62. Korsunsky, I.; Millard, N.; Fan, J.; Slowikowski, K.; Zhang, F.; Wei, K.; Baglaenko, Y.; Brenner, M.; Loh, P.; Raychaudhuri, S. Fast, Sensitive and Accurate Integration of Single-Cell Data with Harmony. Nat Methods 2019, 16 (12), 1289-1296. https://doi.org/10.1038/s41592-019-0619-0.
63. Krueger, F.; Andrews, S. R. Bismark: A Flexible Aligner and Methylation Caller for Bisulfite-Seq Applications. Bioinformatics 2011, 27 (11), 1571-1572. https://doi.org/10.1093/bioinformatics/btr167.
64. Langmead, B.; Salzberg, S. L. Fast Gapped-Read Alignment with Bowtie 2. Nat Methods 2012, 9, 357-359. https://doi.org/10.1038/nmeth.1923.
65. Whyte, W. A.; Orlando, D. A.; Hnisz, D.; Abraham, B. J.; Lin, C. Y.; Kagey, M. H.; Rahl, P. B.; Lee, T. I.; Young, R. A. Master Transcription Factors and Mediator Establish Super-Enhancers at Key Cell Identity Genes. Cell 2013, 153 (2), 307-319. https://doi.org/10.1016/j.cell.2013.03.035.
66. Pintacuda, G.; Wei, G.; Roustan, C.; Kirmizitas, B. A.; Solcan, N.; Cerase, A.; Castello, A.; Mohammed, S.; Moindrot, B.; Nesterova, T. B.; Brockdorff, N. HnRNPK Recruits PCGF3/5-PRC1 to the Xist RNA B-Repeat to Establish Polycomb-Mediated Chromosomal Silencing. Molecular Cell 2017, 68 (5), 955-969.e10. https://doi.org/10.1016/j.molcel.2017.11.013.
67. Li, H.; Handsaker, B.; Wysoker, A.; Fennell, T.; Ruan, J.; Homer, N.; Marth, G.; Abecasis, G.; Durbin, R.; 1000 Genome Project Data Processing Subgroup. The Sequence Alignment/Map Format and SAMtools. Bioinformatics 2009, 25 (16), 2078-2079. https://doi.org/10.1093/bioinformatics/btp352.
68. Picard Toolkit. Broad Institute, GitHub repository. Broad Institute. 2019.
69. Orlando, D. A.; Chen, M. W.; Brown, V. E.; Solanki, S.; Choi, Y. J.; Olson, E. R.; Fritz, C. C.; Bradner, J. E.; Guenther, M. G. Quantitative ChIP-Seq Normalization Reveals Global Modulation of the Epigenome. Cell Reports 2014, 9 (3), 1163-1170. https://doi.org/10.1016/j.celrep.2014.10.018.
70. Zhang, Y.; Liu, T.; Meyer, C. A.; Eeckhoute, J.; Johnson, D. S.; Bernstein, B. E.; Nusbaum, C.; Myers, R. M.; Brown, M.; Li, W.; Liu, X. S. Model-Based Analysis of ChIP-Seq (MACS). Genome Biol 2008, 9, R137. https://doi.org/10.1186/gb-2008-9-9-r137.
71. Li, Q.; Brown, J. B.; Huang, H.; Bickel, P. J. Measuring Reproducibility of High-Throughput Experiments. The Annals of Applied Statistics 2011, 5 (3), 1752-1779. https://doi.org/10.1214/11-AOAS466.
72. Quinlan, A. R.; Hall, I. M. BEDTools: A Flexible Suite of Utilities for Comparing Genomic Features. Bioinformatics 2010, 26 (6), 841-842. https://doi.org/10.1093/bioinformatics/btq033.
73. Liao, Y.; Smyth, G. K.; Shi, W. FeatureCounts: An Efficient General Purpose Program for Assigning Sequence Reads to Genomic Features. Bioinformatics 2014, 30 (7), 923-930. https://doi.org/10.1093/bioinformatics/btt656.
74. Love, M. I.; Huber, W.; Anders, S. Moderated Estimation of Fold Change and Dispersion for RNA-Seq Data with DESeq2. Genome Biol 2014, 15, 550. https://doi.org/10.1186/s13059-014-0550-8.
75. Machanick, P.; Bailey, T. L. MEME-ChIP: Motif Analysis of Large DNA Datasets. Bioinformatics 2011, 27, 1696-1697. https://doi.org/10.1093/bioinformatics/btr189.
76. Alhamdoosh, M.; Ng, M.; Wilson, N. J.; Sheridan, J. M.; Huynh, H.; Wilson, M. J.; Ritchie, M. E. Combining Multiple Tools Outperforms Individual Methods in Gene Set Enrichment Analyses. Bioinformatics 2017, 33 (3), 414-424. https://doi.org/10.1093/bioinformatics/btw623.
77. Love, M. I.; Huber, W.; Anders, S. Moderated Estimation of Fold Change and Dispersion for RNA-Seq Data with DESeq2. Genome Biology 2014, 15 (12), 550. https://doi.org/10.1186/s13059-014-0550-8.

## Claims

**1.** A method of *in vitro* preparing organized 3D cell structures of mammalian cells wherein the method comprises:

i. Providing a homogeneous population of pluripotent or multipotent vertebrates cells, in particular non-human mammalian cells, in particular rodent cells, especially mouse cells in a first culture medium suitable for ESC culture and maintenance of pluripotent state,
ii. On the cells in the first culture medium of i., performing two steps of hypoSUMOylation treatment with an

agent inhibiting small ubiquitin-like modifier (SUMO) conjugation *(SUMO inhibitor),*

wherein the second step of hypoSUMOylation treatment is separated in time from the first step of hypoSUMOylation treatment by at least 3 days, in particular by less than 20 days and
wherein each step of hypoSUMOylation treatment with the SUMO inhibitor is conducted for not more than 48h, preferably is conducted for 48h, and optionally recovering cells obtained after one or two steps of hypoSUMOylation treatment,

iii. Culturing the cells obtained after the second hypoSUMOylation step of treatment with the SUMO inhibitor according to ii. in a second culture medium suitable for ESC culture and differentiation of cells, wherein the first and the second culture medium have different composition,
iv. Optionally repeating at least once the step of hypoSUMOylation treatment with the SUMO inhibitor, wherein each repeat step of hypoSUMOylation treatment is carried out as in ii. and is performed separated in time from the immediately previous one as in ii.
v. Recovering spheroids, wherein the spheroids are composed of at least three 3D self-assembled cell types encompassing from the center to the periphery of the spheroids embryonic stem-like cells (ES-like cells), forming the core of the spheroid on a monolayer of epiblast-like cells (EPI-L cells) and surrounded by extraembryonic endoderm cells (XEN-like cells) and in particular wherein the cell types in the spheroids lack pluripotency.

**2.** The method of claim 1 wherein the first ESC culture medium is a Serum + Lif culture medium and the second ESC culture medium is a N2B27 + Lif culture medium.

**3.** The method of claim 1 or 2 wherein the inhibitor of SUMOylation is a selective small-molecule inhibitor of SUMO E1 enzyme, in particular is ML-792, in particular wherein treatment with ML-792 is performed for 48h in each hypoSUMOylation treatment step, or in particular is TAK-981, in particular wherein treatment with TAK-981 is performed for 48h in each hypoSUMOylation treatment step.

**4.** The method according to any one of claims 1 to 3 wherein the time between two consecutive steps of hypoSUMOylation treatment is 5 days, in particular when inhibitor of SUMO E1 enzyme is ML-792, or is 6 days, in particular when inhibitor of SUMO E1 enzyme is TAK-981.

**5.** The method according to any one of claims 1 to 4 wherein 3 steps of hypoSUMOylation treatment are carried out.

**6.** The method of claim 1 to 5 wherein the spheroids are recovered after 14 to 50 days, in particular after 18 to 50 days of culture or more particularly from 14 to 18 days of culture.

**7.** The method of claim 1 to 6 wherein the recovered spheroids contain over 55% in particular 55% to 65.0% ES-like cells, over 29 %, in particular 29.6 to 40% EPI-L cells and over 4.5 % in particular 5% XEN-like cells.

**8.** The method of claim 1 to 7 wherein the SUMO inhibitor is removed at the end of each step of cell treatment with it or the method is performed without providing a morphogen substance to the cells or both.

**9.** The method of claim 1 to 8 wherein the duration between two steps of hypoSUMOylation treatment with the selective inhibitor of SUMO E1 enzyme enables full recovery of the SUMOylation capability of the cells between the two consecutive steps of hypoSUMOylation treatment.

**10.** The method of any one of claims 1 to 9 which comprises additional steps after recovering the spheroids wherein the steps comprise:

a) transferring spheroid cells to a non-adherent microwell structure wherein the transfer is carried out after at least 14 days or after at least 18 days from initiation of the first hypoSUMOylation treatment and
b) culturing said cells to enable lineage-specific differentiation into embryonic germ layers, wherein the culturing step is performed in the second culture medium and is continued until cell populations are obtained that comprise cell clusters of at least one, preferably all population(s) in the group of: primitive streak (cluster 4), definitive endoderm (cluster 5), neuromesodermal progenitors (NMPs cluster 6) and neuroepithelium (cluster 7), in particular wherein the culture is continued for at least 3 days to achieve elongated structures, in particular is continued for 3 to 4 days,
c) recovering self-organized grown structures that are elongating-multilineages-organized (EMLO) gastruloids

with an anterior-posterior body axis comprising discrete ES-L and EPI-L derived compartments that comprise anteriorly neural ectoderm lineages, posteriorly definitive endoderm and mesoderm lineages, and a primitive streak wherein the neuroectoderm cell lineages are opposite to the primitive streak, wherein the recovery is in particular performed on day 21 after the first step of SUMOylation treatment.

**11.** The method of any one of claims 1 to 9 which comprises additional steps after recovering the spheroids wherein the steps comprise:

a) Seeding dissociated cells obtained from the spheroid in drops such as drops of 4-10μl, in particular 6-7μl, in particular in a microfluidic device, in particular in a droplet microfluidics platform, wherein the transfer is carried out after at least 14 days or after at least 18 days from initiation of the first hypoSUMOylation treatment and
b) culturing said cells to enable lineage-specific differentiation into embryonic germ layers, wherein the culturing step is performed in the second culture medium and is continued until axial elongation of the grown structure is reached, in particular wherein the culture is continued for at least 4 days to achieve elongated structures, in particular is continued for 4 to 5 days until recovery of the self-organized grown structures showing elongation with an anterior-posterior body axis,
c) embedding the recovered self-organized grown structures showing elongation with an anterior-posterior body axis in Matrigel or in a matrix equivalent, in particular in 20% Matrigel and culturing to enable increasing elongation of the structure, in particular carrying out the culture in the second culture medium and Matrigel for at least 2 days, in particular 2 to 3 days,
d) recovering self-organized grown elongated structures that are elongating-multilineages-organized (EMLO) embryoids with an anterior-posterior body axis, wherein cell populations are obtained that comprise cell clusters of at least one, preferably all population(s) are obtained in the group of: endoderm in particular gut endoderm, mesoderm, neuroectoderm, in particular cells of at least one, said elongated structures comprising preferably all cell type(s) in the group of ES-L, EPI-L, primitive streak, NMPs, presomitic mesoderm, somitic mesoderm, pharyngeal mesoderm, definitive endoderm, radial glia, dermomyotome, mesenchyme, craniofacial mesenchyme, endothelium, cardiomyocytes, spinal cord, midbrain-hindbrain, Schwann cell precursors and neurons, in particular recovering self-organized grown elongated structures comprising Schwann cell precursors, wherein the recovery is in particular performed on day 25 after the first step of SUMOylation treatment.

**12.** An organized 3D cell structure which is self-assembled into spheroid wherein the spheroid comprises mammalian cell clusters encompassing 3D-organized ES-like cells as a core resting on EPI-like cells and surrounded by XEN-like cells, in particular wherein the cell types lack pluripotency, in particular wherein the mammalian cells are not human cells or are not exclusively human cells.

**13.** An organized 3D cell structure which is self-assembled into a structure of mammalian cells with Head-to-tail body axis which is either an elongating-multilineages-organized (EMLO) gastruloid comprising discrete ES-L and EPI-L derived compartments that comprise anteriorly neural ectoderm lineages, posteriorly definitive endoderm and mesoderm lineages, and a primitive streak wherein the neuroectoderm cell lineages are opposite to the primitive streak or which is an elongating-multilineages-organized (EMLO) embryoids comprising cell clusters of at least one, preferably all population(s) obtained from the group of: endoderm in particular gut endoderm, mesoderm, neuroectoderm, in particular cells of at least one, said elongated structures comprising preferably all cell type(s) in the group of ES-L, EPI-L, primitive streak, NMPs, presomitic mesoderm, somitic mesoderm, pharyngeal mesoderm, definitive endoderm, radial glia, dermomyotome, mesenchyme, craniofacial mesenchyme, endothelium, cardiomyocytes, spinal cord, midbrain-hindbrain, Schwann cell precursors and neurons, in particular comprising Schwann cell precursors, wherein the mammalian cells are not human cells or are not exclusively human cells, in particular wherein the mammalian cells, in particular the human cells lack pluripotency.

**14.** Use of a spheroid structure obtained according to the method of any one of claims 1 to 9, for screening of molecules, in particular for high throughput screening of molecules for assessing their activity on 2D to 3D transition of cell culture, in particular for assessing their capability to interfere with 2D to 3D transition, in particular wherein the molecules are anti-cancer drug candidates.

**15.** Use of elongating-multilineages-organized (EMLO) gastruloids obtained according to the method of claim 10, or elongating-multilineages-organized (EMLO) embryoids obtained according to the method of claim 11, in particular of non-human mammalian EMLO gastruloids or embryoids, (i) for screening of molecules, in particular for high throughput screening of molecules for assessing their activity on development in particular on developmental toxicity or (ii) for the production of growth factors, cytokines, morphogens, chemokines of interest, in particular for interest

for therapeutic use or (iii) for the production of cells for therapeutic potential.

**16.** A microfluidic device (100) comprising a body (101) having a thickness and comprising a bottom side and a top side facing each other, said bottom side being arranged at distance with a plate (103) so as to define a channel (114) for the flow of a fluid between at least one inlet (122) and at least one outlet (124), said body comprising at least one trap (102) extending along an axis of revolution (X100) with said trap comprising a first part (104) and a second part (108) extending along said axis of revolution, the first part being arranged, along said axis of revolution, between the second part (108) and an opening (106) of the trap that opens out at the bottom side in the channel, wherein the surface of a cross-section of the first part at the opening is greater than the surface of a cross-section of the second part and wherein the diameter of the opening (106) is equal to or greater than twice the distance between the plate (103) and said opening (106).

**17.** The microfluidic device (100) according to the claim 16, wherein the first part comprises a convex annular wall (110) having a peripheral free edge defining said opening and/or wherein the cavity is delimited in its second part by a cylindrical wall (112) having a hexagonal cross-section.

**18.** The microfluidic device (100) according to anyone of claims 16 to 17, wherein the dimension (d2) of the second part (108) along said axis of revolution (X100) is at least five times the dimension (d1) of the first part (104) along the axis of revolution.

**19.** The microfluidic device (100) according to anyone of claims 16 to 18, wherein the diameter (Ø1) of the opening (106) is comprised from 2 and 3 mm, in particular from 2.2 and 2.6 mm, in particular is equal to 2.4 mm and/or wherein the diameter (Ø2) of the cross-section of the second part (108) is comprised from 1 and 2 mm, in particular from 1.1 and 1.3 mm, in particular equal to 1.2 mm.

**20.** The microfluidic device (100) according to of claims 16 to 19, wherein each trap (102) opens out in a channel (114) formed by a recess arranged in the body (101), and wherein the diameter (Ø1) of the opening (106) of said trap (102) is greater than two times the dimension (h1) of the channel (114) along the axis of revolution (X100).

**21.** Method of manipulating droplets, using the microfluidic device (100) of anyone of claims 16 to 20, the method comprising:

(M1) introducing (S101) a first liquid composition comprising first droplets (202) in the channel (114) of the microfluidic device (100),
(M2) tilting the body at a first angle with respect to the horizontal axis, the first angle being comprised from 30° and 60°, in particular comprised from 40° and 50°, in particular equal to 45°,
(M3) trapping one of the first droplets (202) by capillarity in the first part (104) of one of the traps (102) and standing by (S102) until said first droplet migrates to the second part (108) of said trap by buoyancy.

**22.** The method according to claim 21 further comprising:

(M4) introducing (S103) a second liquid composition comprising second droplets (204) in said microfluidic device (100),
(M5) tilting the body (101) at a second angle with respect to the horizontal axis, the second angle being comprised from 25° and 35°, in particular equal to 25°,
(M6) trapping one of the second droplet (204) by capillarity in the first part (104) of said trap and standing by (S104) until the first droplet (202) and the second droplet (204) comprised in said trap merge, wherein the first or the second droplets, in particular the first, comprises cells, in particular comprises a homogenous population of pluripotent or multipotent vertebrate cells, in particular non-human mammalian cells, more particularly rodent cells.

**23.** Method according to claim 22, comprising before step (M4) a step of flushing an immiscible fluorocarbon oil without surfactant or containing PFO (Perfluoro-Octanol (PFO), which reduces the emulsion stability) at concentration of 20% in the traps (102) and/or wherein the first droplets comprise dissociated spheroid cells and wherein the second droplets comprise Matrigel or an equivalent matrix.

**24.** Method of *in vitro* preparing organized 3D cell structures of mammalian cells according to any one of claims 1 to 9 or 11 wherein the microfluidic device (100) is as defined in any one of claims 16 to 20.

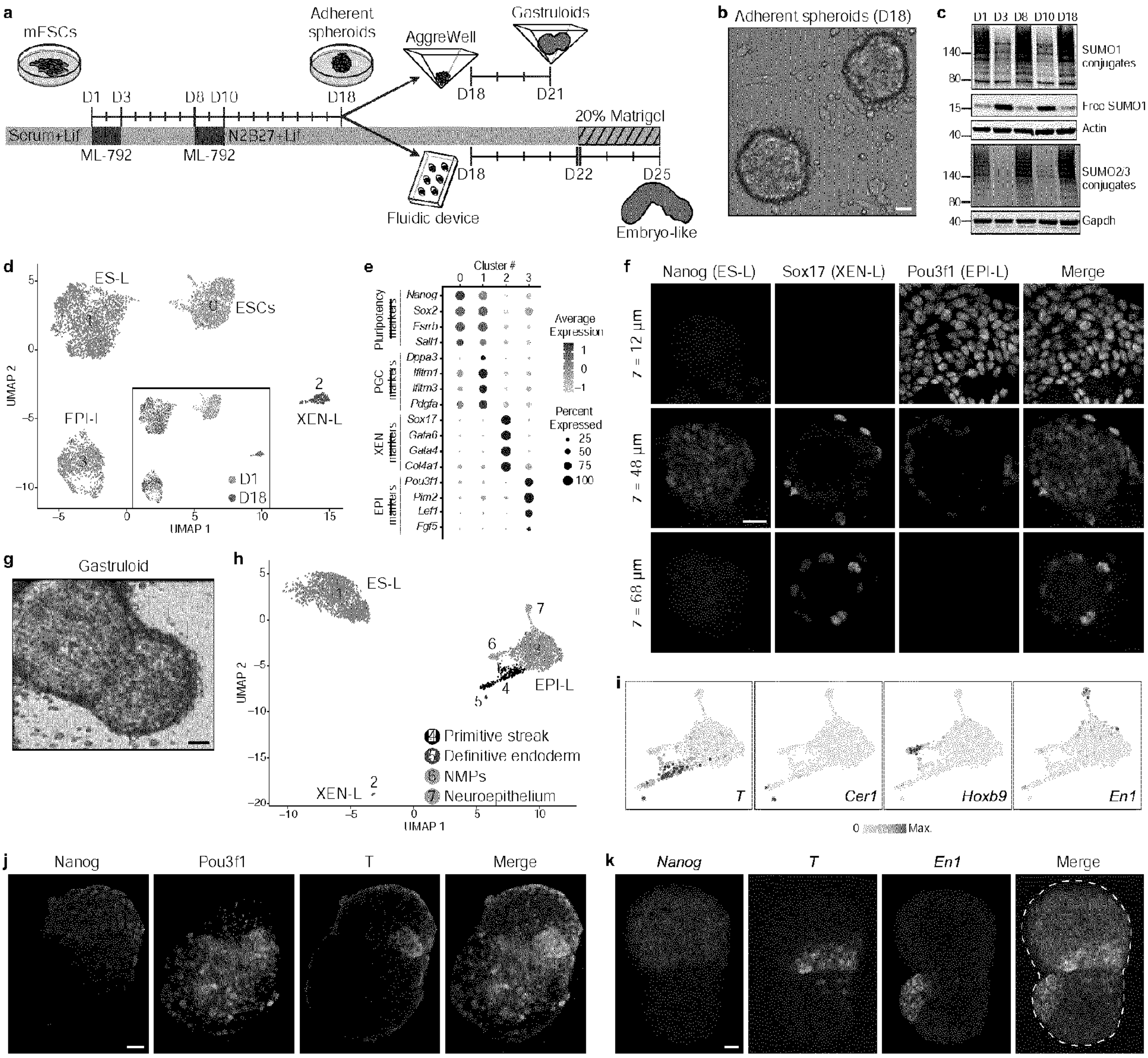

Fig. 1a

a.

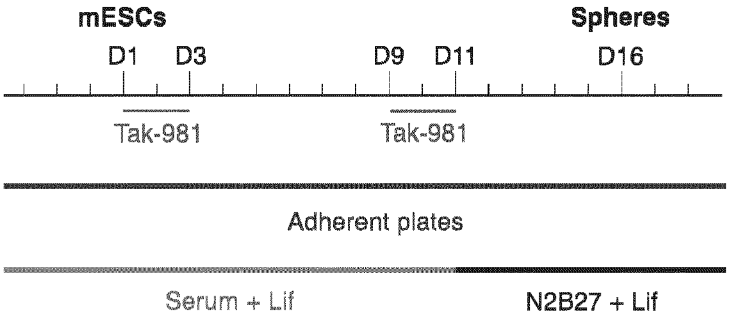
mESCs
Spheres
D1
D3
D9
D11
D16
Tak-981
Tak-981
Adherent plates
Serum + Lif
N2B27 + Lif

b.

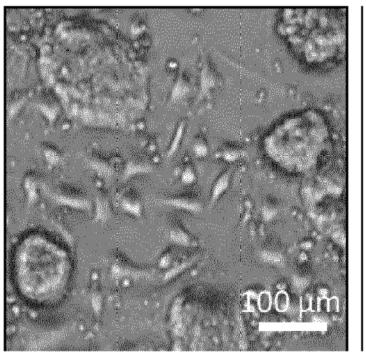
100 µm

FIG. 1b

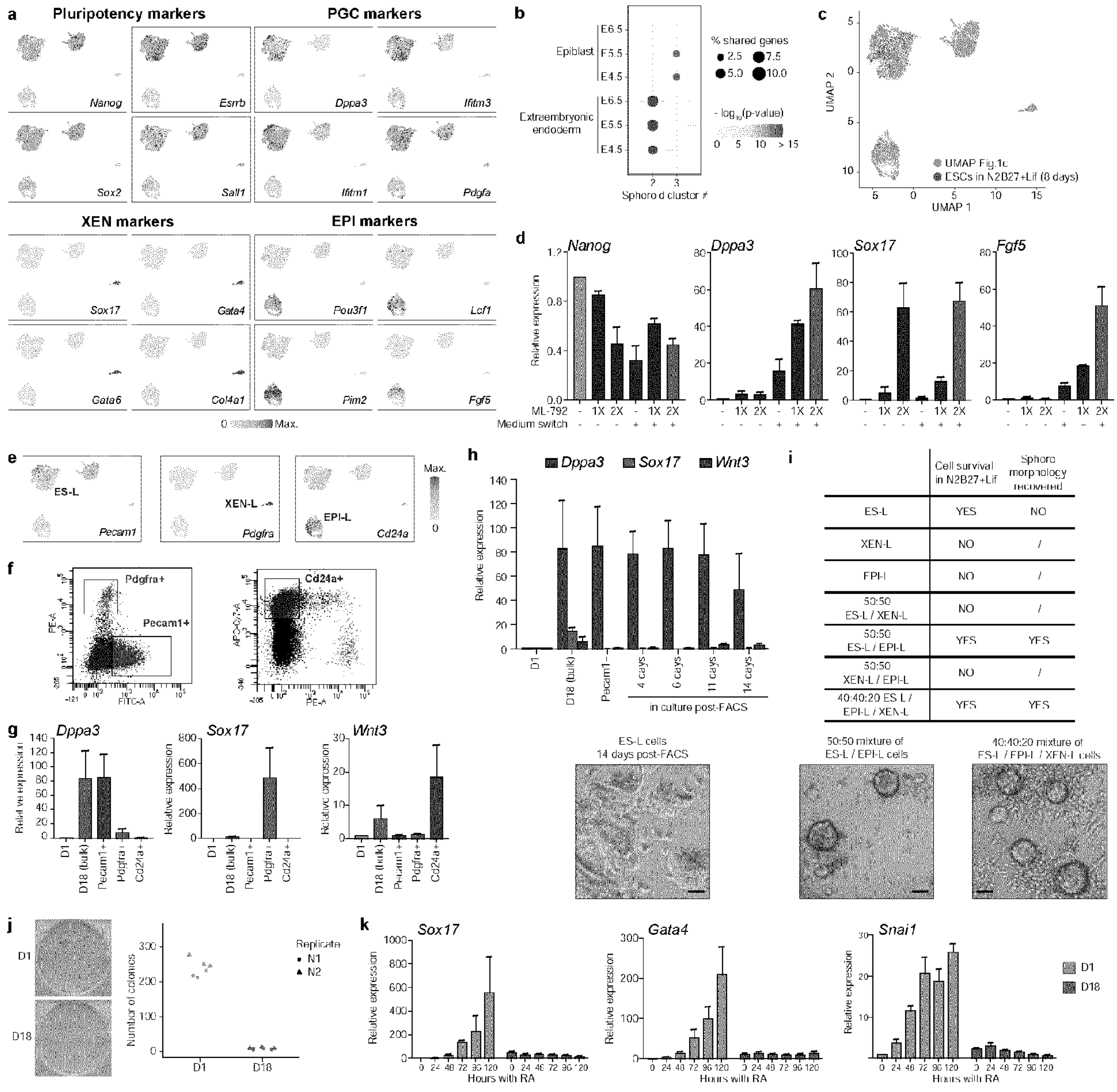
a
Pluripotency markers
PGC markers
Nanog
Esrrb
Dppa3
Ifitm3
Sox2
Sall1
Ifitm1
Pdgfa
XEN markers
EPI markers
Sox17
Gata4
Pou3f1
Lef1
Gata6
Col4a1
Pim2
Fgf5
0 Max.
b
Epiblast
Extraembryonic endoderm
E6.5
E5.5
E4.5
% shared genes
2.5
5.0
7.5
10.0
- log10(p-value)
0 5 10 > 15
Spheroid cluster #
c
UMAP 1
UMAP 2
UMAP Fig.1c
ESCs in N2B27+Lif (8 days)
d
Nanog
Dppa3
Sox17
Fgf5
Relative expression
ML-792
Medium switch
e
ES-L
XEN-L
EPI-L
Pecam1
Pdgfra
Cd24a
Max.
0
f
Pdgfra+
Pecam1+
Cd24a+
FITC-A
PE-A
g
Dppa3
Sox17
Wnt3
Relative expression
D1
D18 (bulk)
Pecam1+
Pdgfra+
Cd24a+
h
Dppa3
Sox17
Wnt3
Relative expression
D1
D18 (bulk)
Pecam1+
4 days
6 days
11 days
14 days
in culture post-FACS
i
Cell survival in N2B27+Lif
Spheroid morphology recovered
ES-L YES NO
XEN-L NO /
EPI-L NO /
50:50 ES-L / XEN-L NO /
50:50 ES-L / EPI-L YES YES
50:50 XEN-L / EPI-L NO /
40:40:20 ES-L / EPI-L / XEN-L YES YES
ES-L cells 14 days post-FACS
50:50 mixture of ES-L / EPI-L cells
40:40:20 mixture of ES-L / EPI-L / XEN-L cells
j
D1
D18
Number of colonies
Replicate
N1
N2
k
Sox17
Gata4
Snai1
Relative expression
Hours with RA
D1
D18

Fig. 2

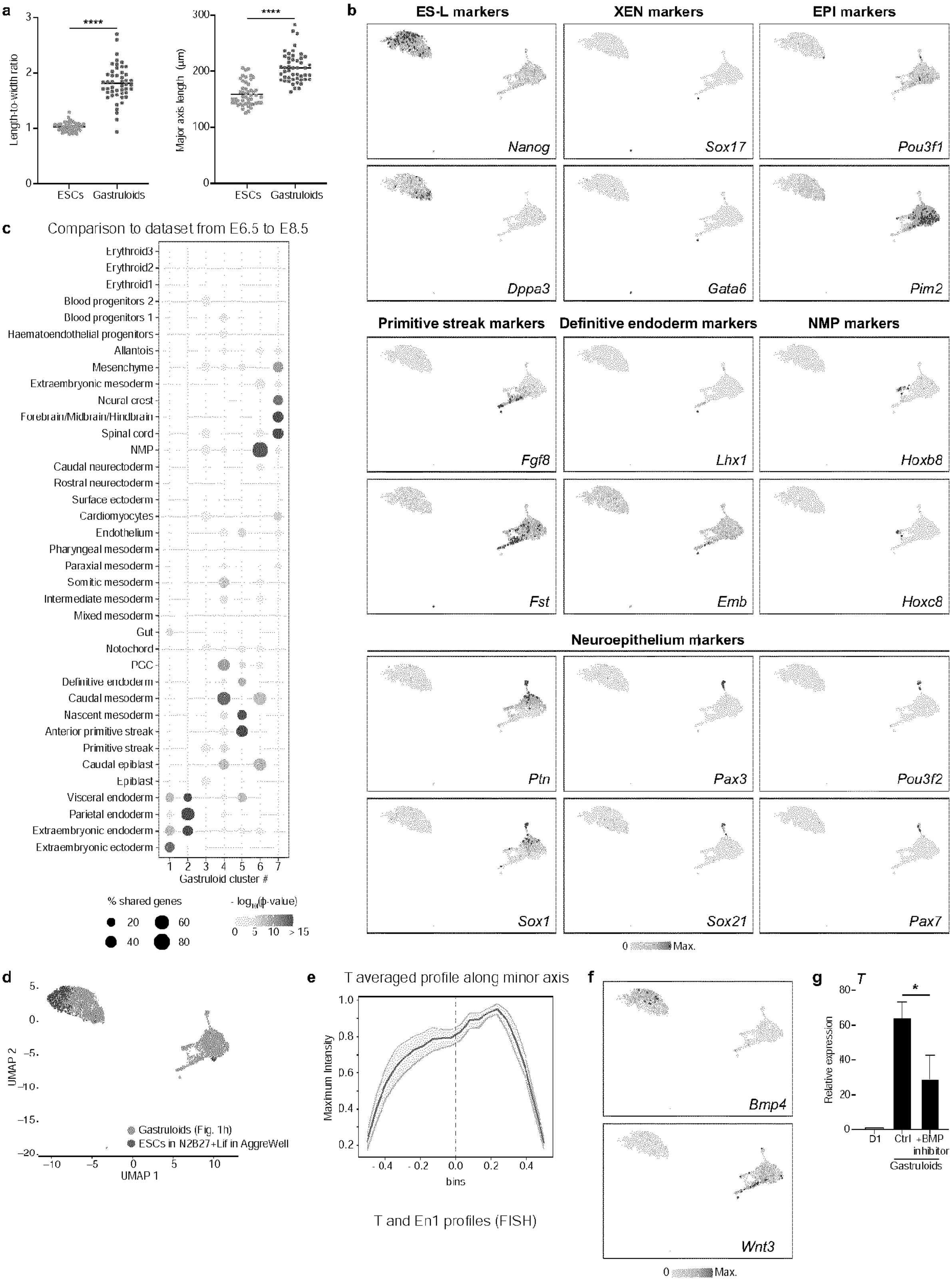
a
Length-to-width ratio
Major axis length (µm)
ESCs Gastruloids
b
ES-L markers
XEN markers
EPI markers
Nanog
Sox17
Pou3f1
Dppa3
Gata6
Pim2
Primitive streak markers
Definitive endoderm markers
NMP markers
Fgf8
Lhx1
Hoxb8
Fst
Emb
Hoxc8
Neuroepithelium markers
Ptn
Pax3
Pou3f2
Sox1
Sox21
Pax7
c
Comparison to dataset from E6.5 to E8.5
Gastruloid cluster #
% shared genes
d
UMAP 1
UMAP 2
Gastruloids (Fig. 1h)
ESCs in N2B27+Lif in AggreWell
e
T averaged profile along minor axis
Maximum Intensity
bins
T and En1 profiles (FISH)
f
Bmp4
Wnt3
g
T
Relative expression
D1
Ctrl
+BMP inhibitor
Gastruloids

Fig. 3

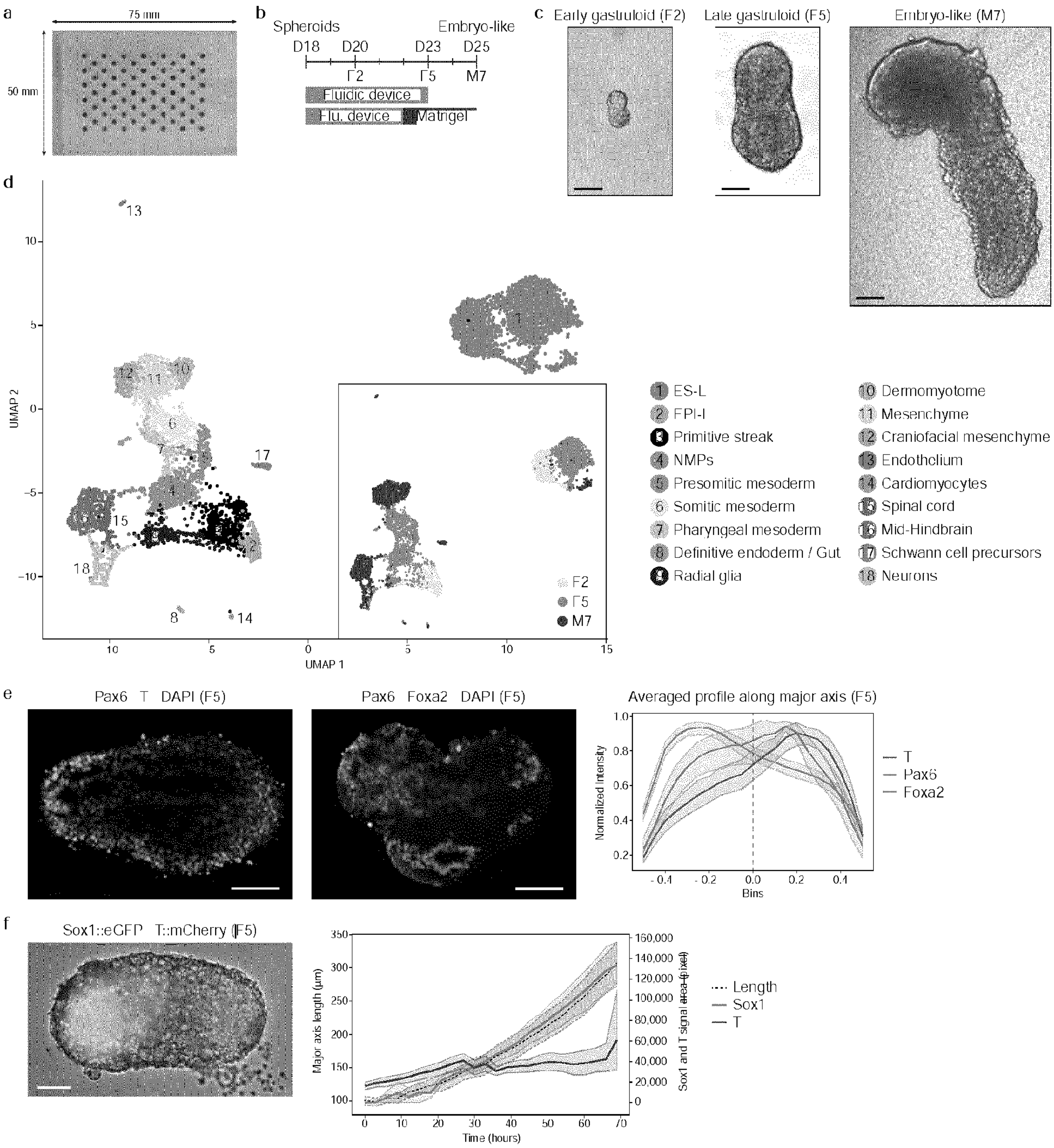
a
75 mm
50 mm
b
Spheroids
Embryo-like
D18 D20 D23 D25
F2 F5 M7
Fluidic device
Flu. device
Matrigel
c
Early gastruloid (F2)
Late gastruloid (F5)
Embryo-like (M7)
d
UMAP 2
UMAP 1
F2
F5
M7
1 ES-L
2 EPI-l
3 Primitive streak
4 NMPs
5 Presomitic mesoderm
6 Somitic mesoderm
7 Pharyngeal mesoderm
8 Definitive endoderm / Gut
9 Radial glia
10 Dermomyotome
11 Mesenchyme
12 Craniofacial mesenchyme
13 Endothelium
14 Cardiomyocytes
15 Spinal cord
16 Mid-Hindbrain
17 Schwann cell precursors
18 Neurons
e
Pax6 T DAPI (F5)
Pax6 Foxa2 DAPI (F5)
Averaged profile along major axis (F5)
Normalized Intensity
Bins
T
Pax6
Foxa2
f
Sox1::eGFP T::mCherry (F5)
Major axis length (µm)
Sox1 and T signal area (pixel)
Time (hours)
Length
Sox1
T

Fig. 4

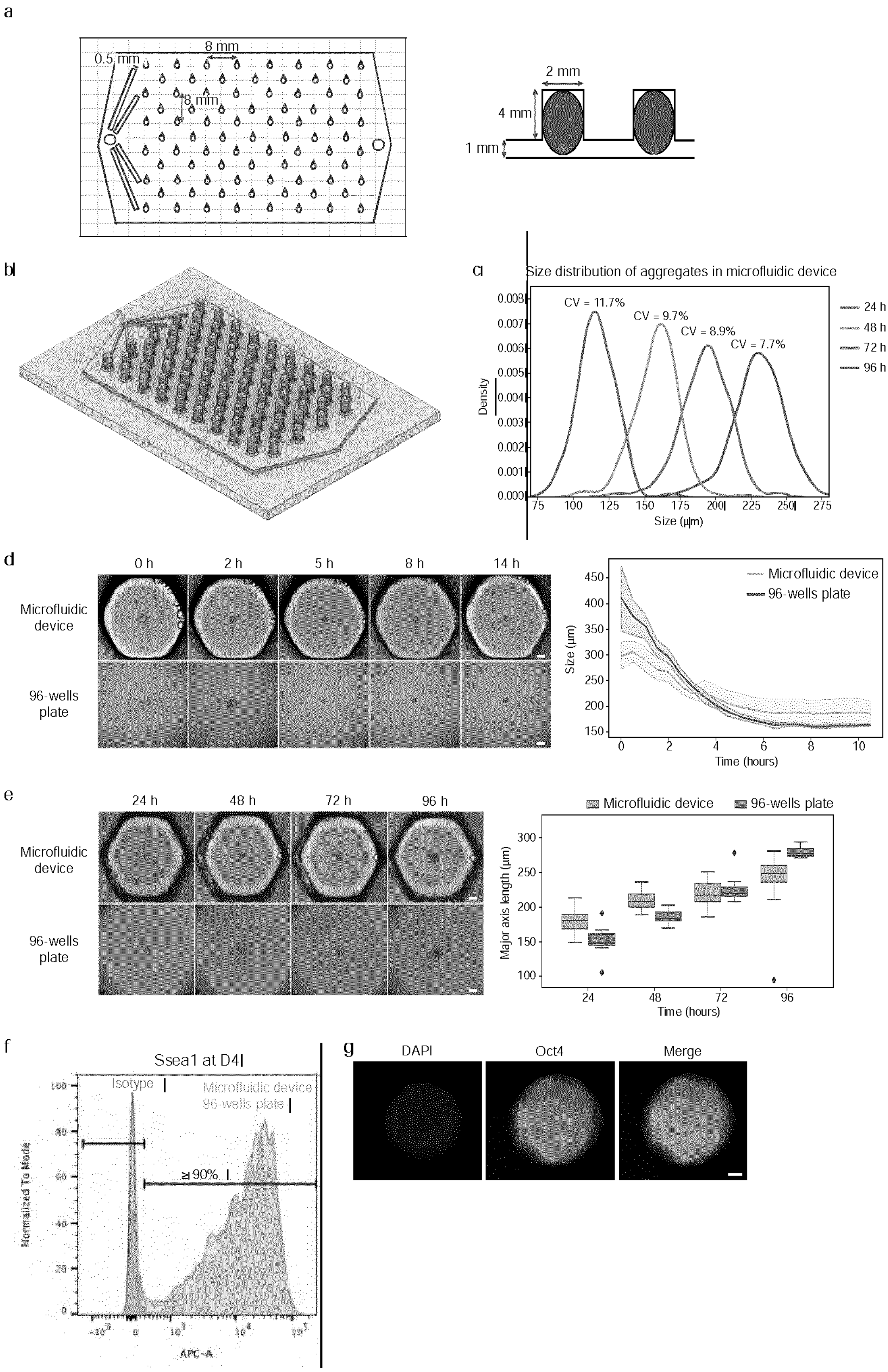
a
8 mm
0.5 mm
8 mm
2 mm
4 mm
1 mm
b
c
Size distribution of aggregates in microfluidic device
CV = 11.7%
CV = 9.7%
CV = 8.9%
CV = 7.7%
24 h
48 h
72 h
96 h
Density
Size (μm)
d
0 h
2 h
5 h
8 h
14 h
Microfluidic device
96-wells plate
Size (μm)
Time (hours)
e
24 h
48 h
72 h
96 h
Microfluidic device
96-wells plate
Major axis length (μm)
Time (hours)
f
Ssea1 at D4
Isotype
Microfluidic device
96-wells plate
≥ 90%
Normalized To Mode
APC-A
g
DAPI
Oct4
Merge

Fig. 5

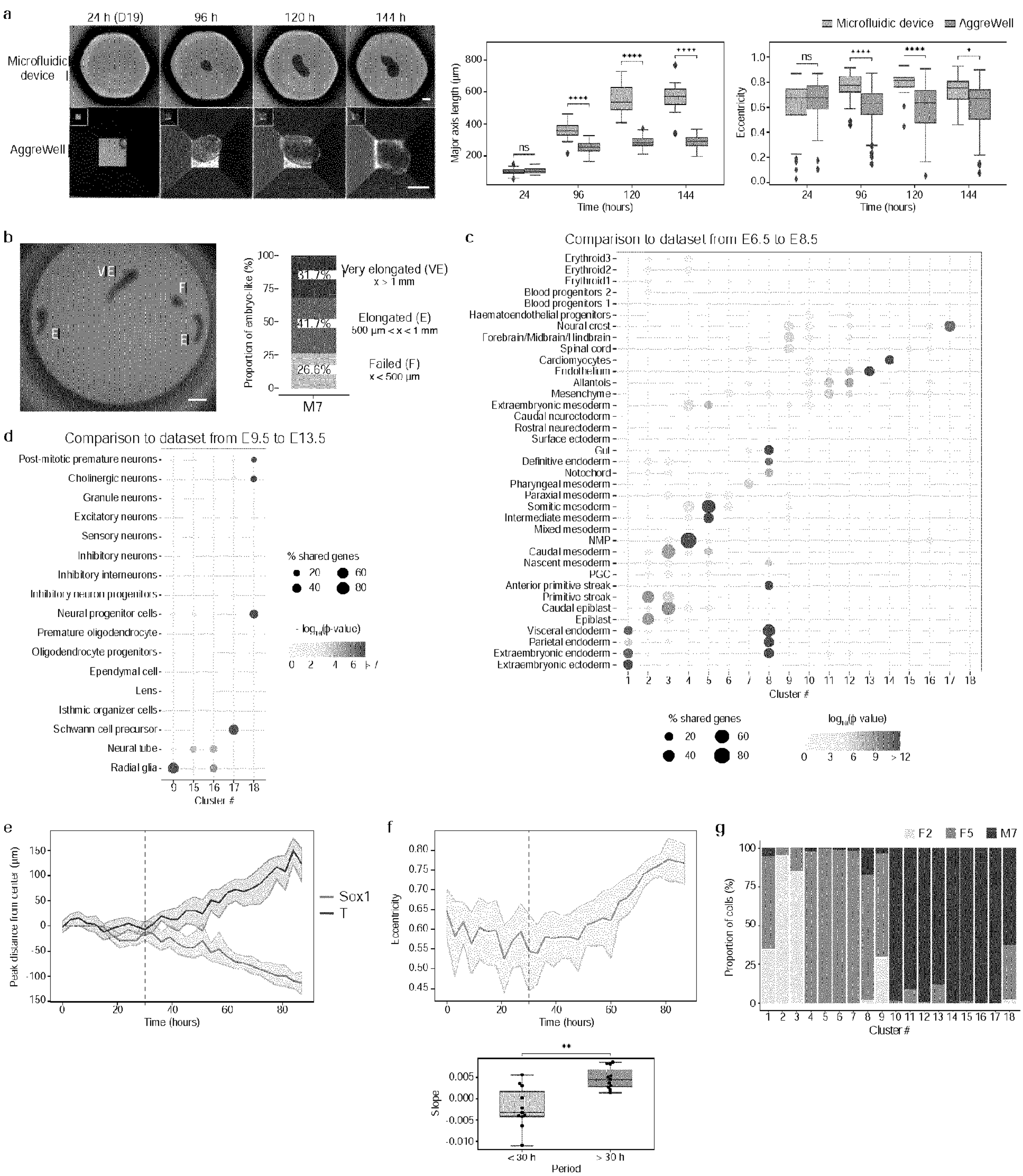
a
24 h (D19)
96 h
120 h
144 h
Microfluidic device
AggreWell
Microfluidic device
AggreWell
Major axis length (μm)
Time (hours)
Eccentricity
Time (hours)
ns
****
b
VE
F
E
E
Proportion of embryo-like (%)
31.7%
41.7%
26.6%
Very elongated (VE)
x > 1 mm
Elongated (E)
500 μm < x < 1 mm
Failed (F)
x < 500 μm
M7
c
Comparison to dataset from E6.5 to E8.5
Erythroid3
Erythroid2
Erythroid1
Blood progenitors 2
Blood progenitors 1
Haematoendothelial progenitors
Neural crest
Forebrain/Midbrain/Hindbrain
Spinal cord
Cardiomyocytes
Endothelium
Allantois
Mesenchyme
Extraembryonic mesoderm
Caudal neurectoderm
Rostral neurectoderm
Surface ectoderm
Gut
Definitive endoderm
Notochord
Pharyngeal mesoderm
Paraxial mesoderm
Somitic mesoderm
Intermediate mesoderm
Mixed mesoderm
NMP
Caudal mesoderm
Nascent mesoderm
PGC
Anterior primitive streak
Primitive streak
Caudal epiblast
Epiblast
Visceral endoderm
Parietal endoderm
Extraembryonic endoderm
Extraembryonic ectoderm
Cluster #
% shared genes
log10(p value)
d
Comparison to dataset from E9.5 to E13.5
Post-mitotic premature neurons
Cholinergic neurons
Granule neurons
Excitatory neurons
Sensory neurons
Inhibitory neurons
Inhibitory interneurons
Inhibitory neuron progenitors
Neural progenitor cells
Premature oligodendrocyte
Oligodendrocyte progenitors
Ependymal cell
Lens
Isthmic organizer cells
Schwann cell precursor
Neural tube
Radial glia
Cluster #
% shared genes
-log10(p-value)
e
Peak distance from center (μm)
Time (hours)
Sox1
T
f
Eccentricity
Time (hours)
Slope
< 30 h
> 30 h
Period
**
g
F2
F5
M7
Proportion of cells (%)
Cluster #

Fig. 6

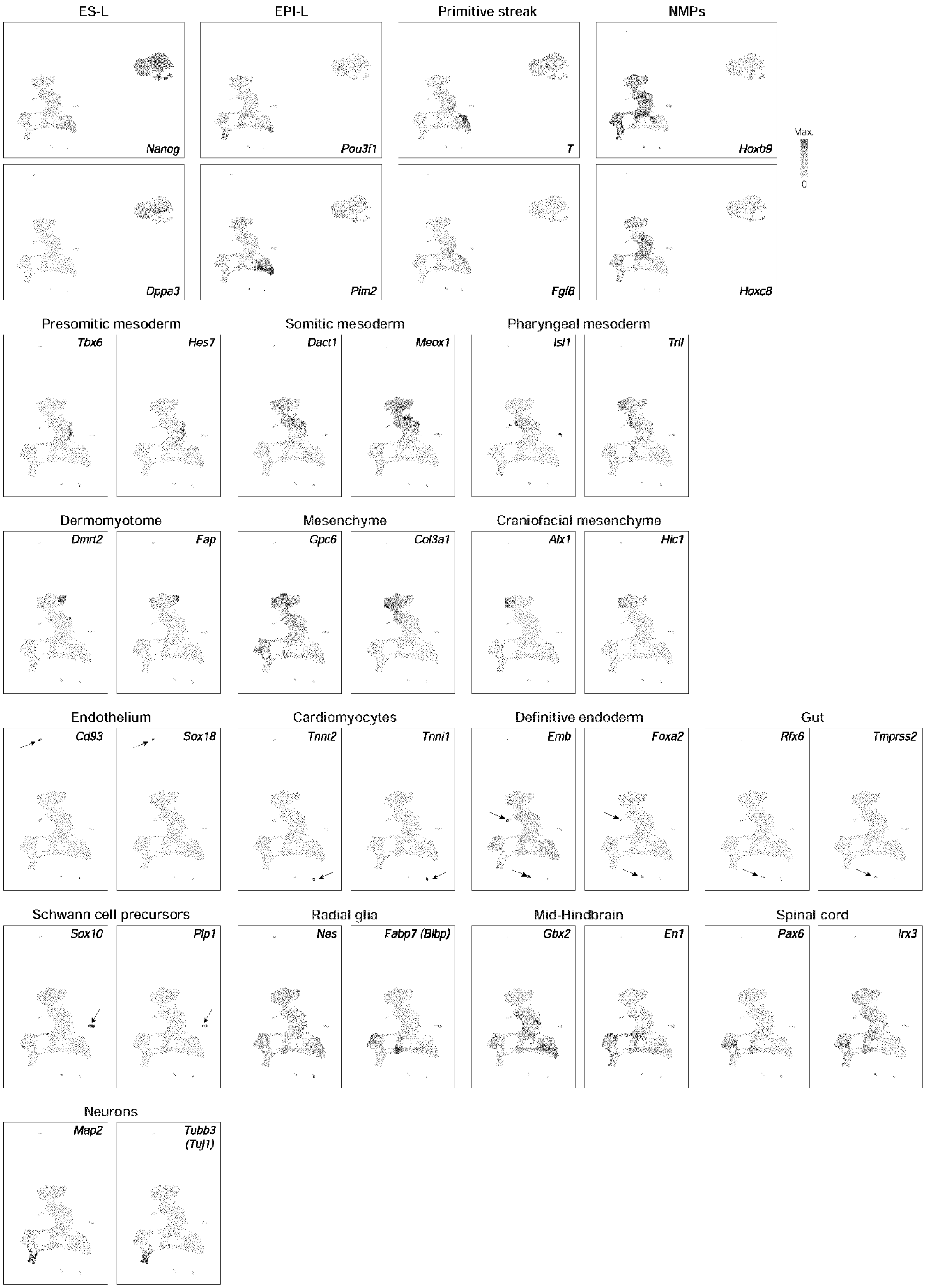
ES-L
EPI-L
Primitive streak
NMPs
Nanog
Pou3f1
T
Hoxb9
Max.
0
Dppa3
Pim2
Fgf8
Hoxc8
Presomitic mesoderm
Somitic mesoderm
Pharyngeal mesoderm
Tbx6
Hes7
Dact1
Meox1
Isl1
Tril
Dermomyotome
Mesenchyme
Craniofacial mesenchyme
Dmrt2
Fap
Gpc6
Col3a1
Alx1
Hic1
Endothelium
Cardiomyocytes
Definitive endoderm
Gut
Cd93
Sox18
Tnnt2
Tnni1
Emb
Foxa2
Rfx6
Tmprss2
Schwann cell precursors
Radial glia
Mid-Hindbrain
Spinal cord
Sox10
Plp1
Nes
Fabp7 (Blbp)
Gbx2
En1
Pax6
Irx3
Neurons
Map2
Tubb3 (Tuj1)

Fig. 7

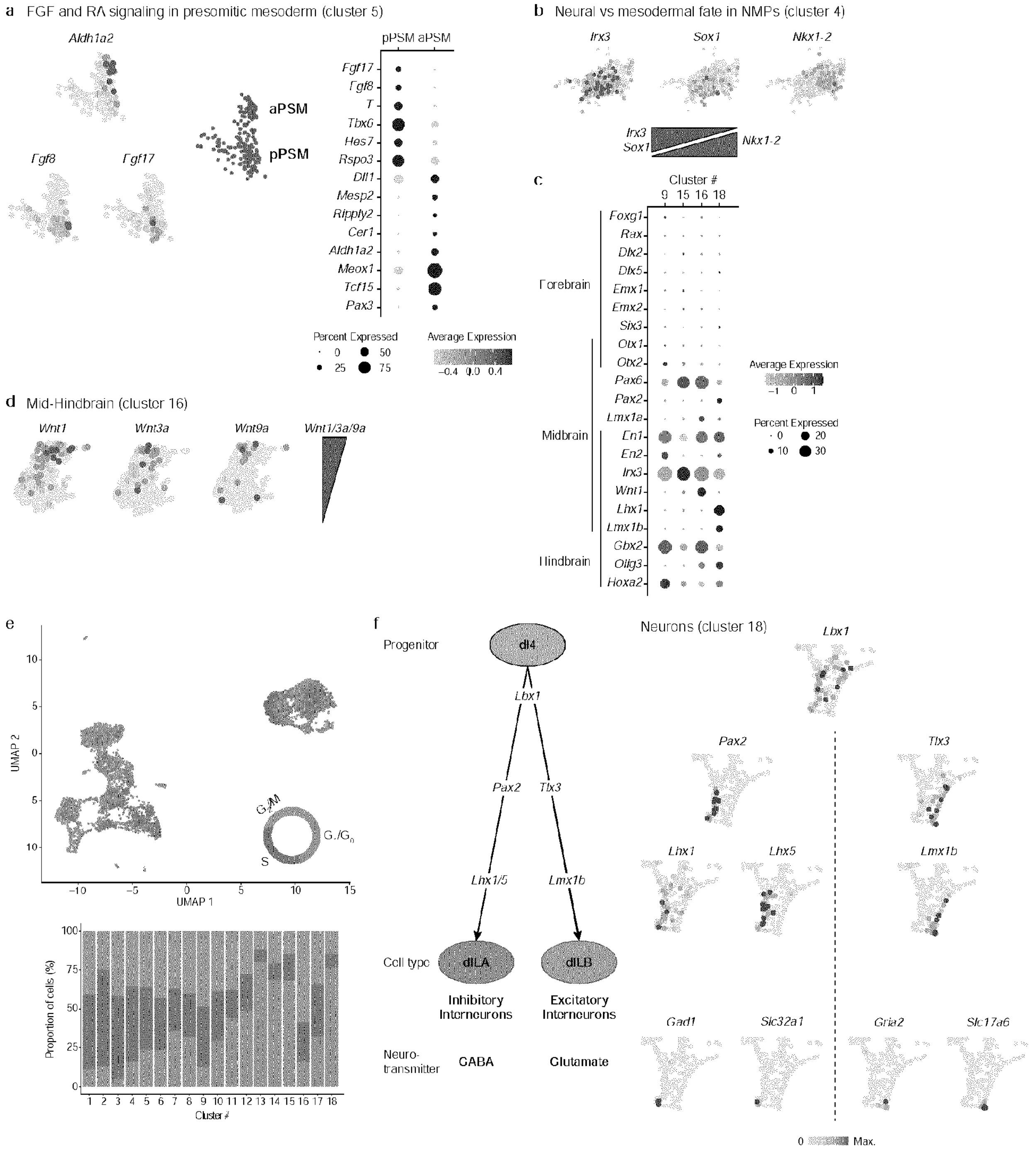
a FGF and RA signaling in presomitic mesoderm (cluster 5)
Aldh1a2
Fgf8
Fgf17
aPSM
pPSM
pPSM aPSM
Fgf17
Fgf8
T
Tbx6
Hes7
Rspo3
Dll1
Mesp2
Ripply2
Cer1
Aldh1a2
Meox1
Tcf15
Pax3
Percent Expressed
0 25 50 75
Average Expression
-0.4 0.0 0.4
b Neural vs mesodermal fate in NMPs (cluster 4)
Irx3
Sox1
Nkx1-2
c
Cluster #
9 15 16 18
Forebrain
Foxg1
Rax
Dlx2
Dlx5
Emx1
Emx2
Six3
Otx1
Otx2
Midbrain
Pax6
Pax2
Lmx1a
En1
En2
Irx3
Wnt1
Lhx1
Lmx1b
Hindbrain
Gbx2
Olig3
Hoxa2
Average Expression
-1 0 1
Percent Expressed
0 10 20 30
d Mid-Hindbrain (cluster 16)
Wnt1
Wnt3a
Wnt9a
Wnt1/3a/9a
e
UMAP 2
UMAP 1
G2/M
G1/G0
S
Proportion of cells (%)
Cluster #
f
Progenitor
dI4
Lbx1
Pax2
Tlx3
Lhx1/5
Lmx1b
Cell type
dILA
dILB
Inhibitory Interneurons
Excitatory Interneurons
Neuro-transmitter
GABA
Glutamate
Neurons (cluster 18)
Lbx1
Pax2
Tlx3
Lhx1
Lhx5
Lmx1b
Gad1
Slc32a1
Gria2
Slc17a6
0 Max.

Fig. 8

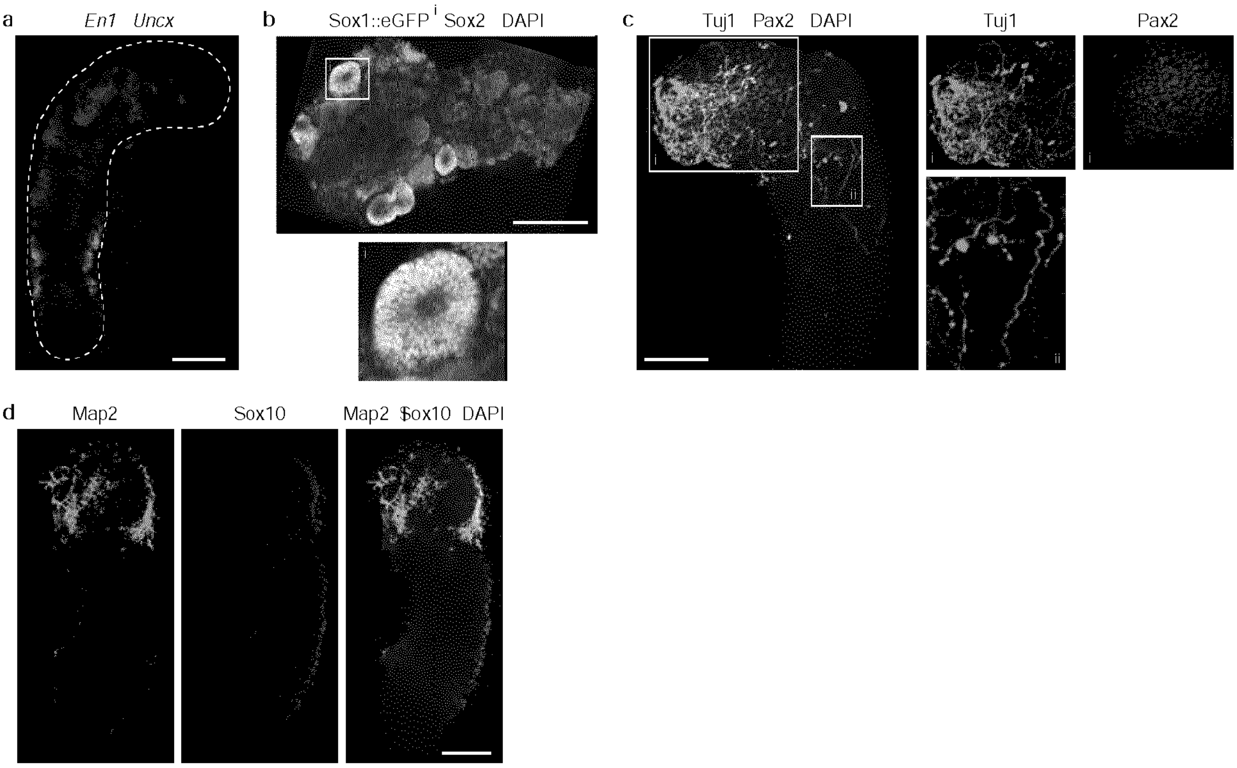
a
En1 Uncx
b
Sox1::eGFP Sox2 DAPI
i
c
Tuj1 Pax2 DAPI
Tuj1
Pax2
i
ii
d
Map2
Sox10
Map2 Sox10 DAPI

Fig. 9

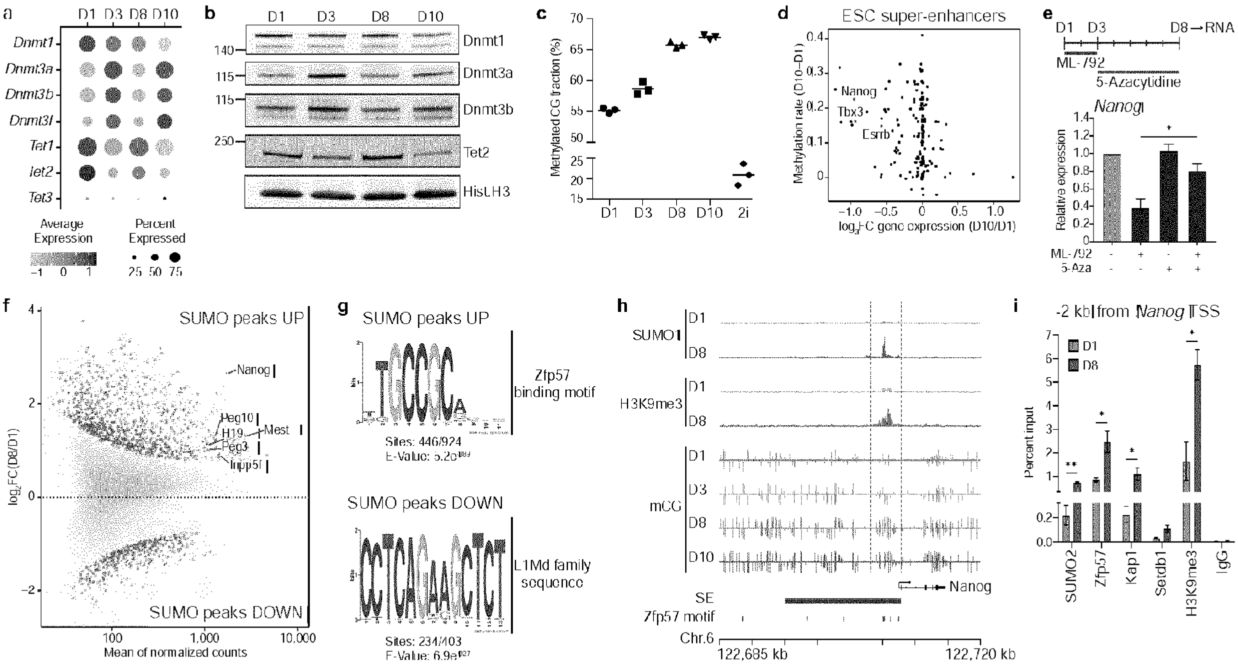
a
D1 D3 D8 D10
Dnmt1
Dnmt3a
Dnmt3b
Dnmt3l
Tet1
Tet2
Tet3
Average Expression
Percent Expressed
b
D1 D3 D8 D10
Dnmt1
Dnmt3a
Dnmt3b
Tet2
Hist H3
c
Methylated CG fraction (%)
D1 D3 D8 D10 2i
d
ESC super-enhancers
Methylation rate (D10–D1)
Nanog
Tbx3
Esrrb
log2FC gene expression (D10/D1)
e
D1 D3 D8→RNA
ML-792
5-Azacytidine
Nanog
Relative expression
ML-792
5-Aza
f
SUMO peaks UP
Nanog
Peg10
H19
Mest
Peg3
Inpp5f
SUMO peaks DOWN
log2FC (D8/D1)
Mean of normalized counts
g
SUMO peaks UP
Zfp57 binding motif
Sites: 446/924
SUMO peaks DOWN
L1Md family sequence
Sites: 234/403
h
SUMO1
H3K9me3
mCG
SE
Zfp57 motif
Nanog
Chr.6
122,685 kb
122,720 kb
i
-2 kb from Nanog TSS
D1
D8
Percent input
SUMO2
Zfp57
Kap1
Setdb1
H3K9me3
IgG

Fig. 10

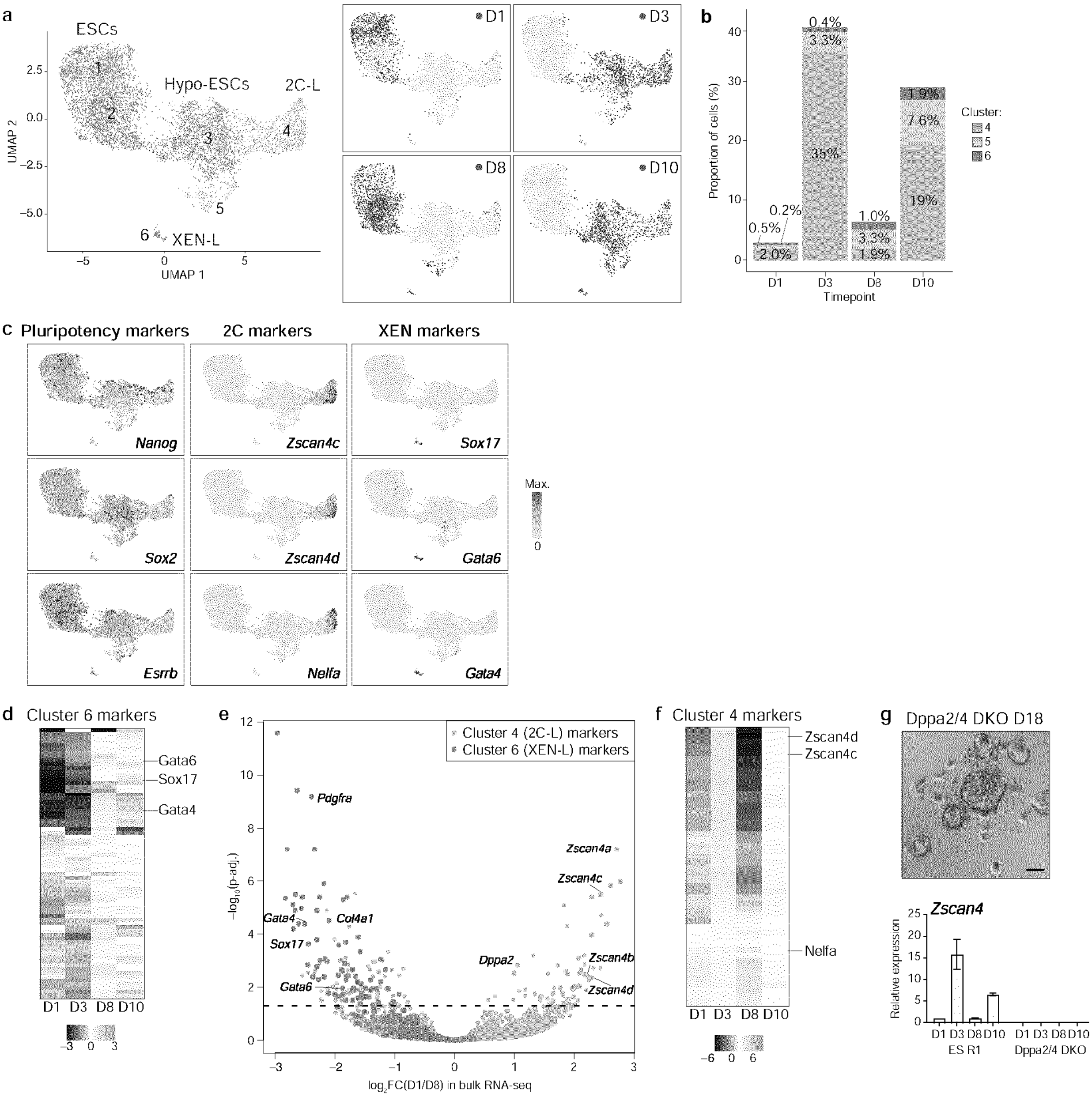
a
ESCs
Hypo-ESCs
2C-L
XEN-L
UMAP 1
UMAP 2
D1
D3
D8
D10
b
Proportion of cells (%)
Timepoint
Cluster:
4
5
6
c Pluripotency markers
2C markers
XEN markers
Nanog
Sox2
Esrrb
Zscan4c
Zscan4d
Nelfa
Sox17
Gata6
Gata4
Max.
d Cluster 6 markers
e
Cluster 4 (2C-L) markers
Cluster 6 (XEN-L) markers
Pdgfra
Col4a1
Dppa2
Zscan4a
Zscan4b
log2FC(D1/D8) in bulk RNA-seq
f Cluster 4 markers
g Dppa2/4 DKO D18
Zscan4
Relative expression
ES R1
Dppa2/4 DKO

Fig. 11

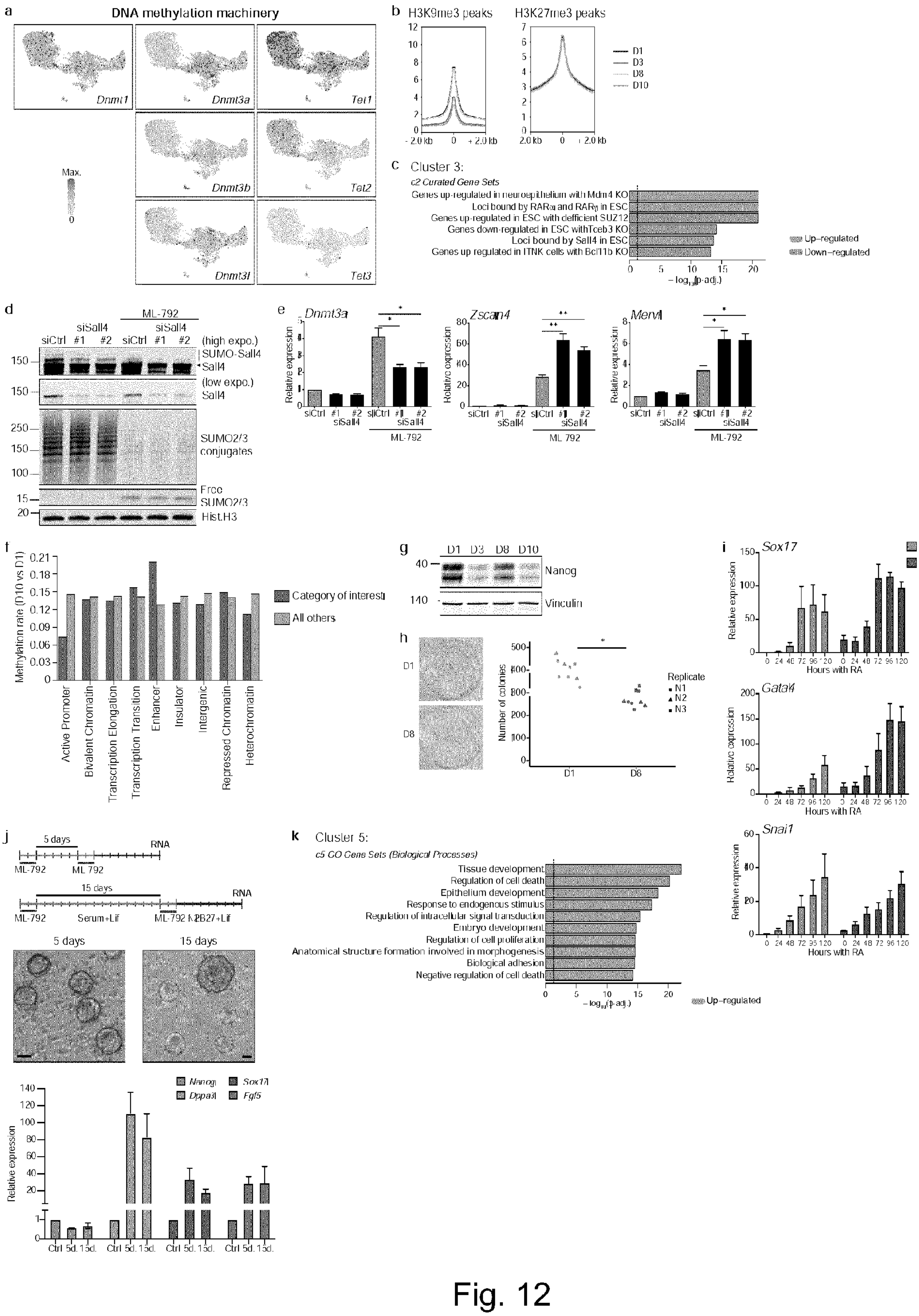
a
DNA methylation machinery
Dnmt1
Dnmt3a
Tet1
Dnmt3b
Tet2
Dnmt3l
Tet3
Max.
0
b
H3K9me3 peaks
H3K27me3 peaks
D1
D3
D8
D10
c
Cluster 3:
c2 Curated Gene Sets
Genes up-regulated in neuroepithelium with Mdm4 KO
Loci bound by RARα and RARγ in ESC
Genes up-regulated in ESC with defficient SUZ12
Genes down-regulated in ESC with Tceb3 KO
Loci bound by Sall4 in ESC
Genes up regulated in ITNK cells with Bcl11b KO
Up-regulated
Down-regulated
d
ML-792
siSall4
siCtrl #1 #2 siCtrl #1 #2
(high expo.)
SUMO-Sall4
Sall4
(low expo.)
Sall4
SUMO2/3 conjugates
Free SUMO2/3
Hist.H3
e
Dnmt3a
Zscan4
Merv
Relative expression
siCtrl
siSall4
ML-792
f
Methylation rate (D10 vs D1)
Category of interest
All others
Active Promoter
Bivalent Chromatin
Transcription Elongation
Transcription Transition
Enhancer
Insulator
Intergenic
Repressed Chromatin
Heterochromatin
g
D1 D3 D8 D10
Nanog
Vinculin
h
D1
D8
Number of colonies
Replicate
N1
N2
N3
i
Sox17
Gata4
Snai1
Relative expression
Hours with RA
D1
D8
j
5 days
RNA
ML-792
15 days
Serum+Lif
ML-792 N2B27+Lif
5 days
15 days
Nanog
Dppa3
Sox17
Fgf5
Relative expression
Ctrl 5d. 15d.
k
Cluster 5:
c5 GO Gene Sets (Biological Processes)
Tissue development
Regulation of cell death
Epithelium development
Response to endogenous stimulus
Regulation of intracellular signal transduction
Embryo development
Regulation of cell proliferation
Anatomical structure formation involved in morphogenesis
Biological adhesion
Negative regulation of cell death
Up-regulated

Fig. 12

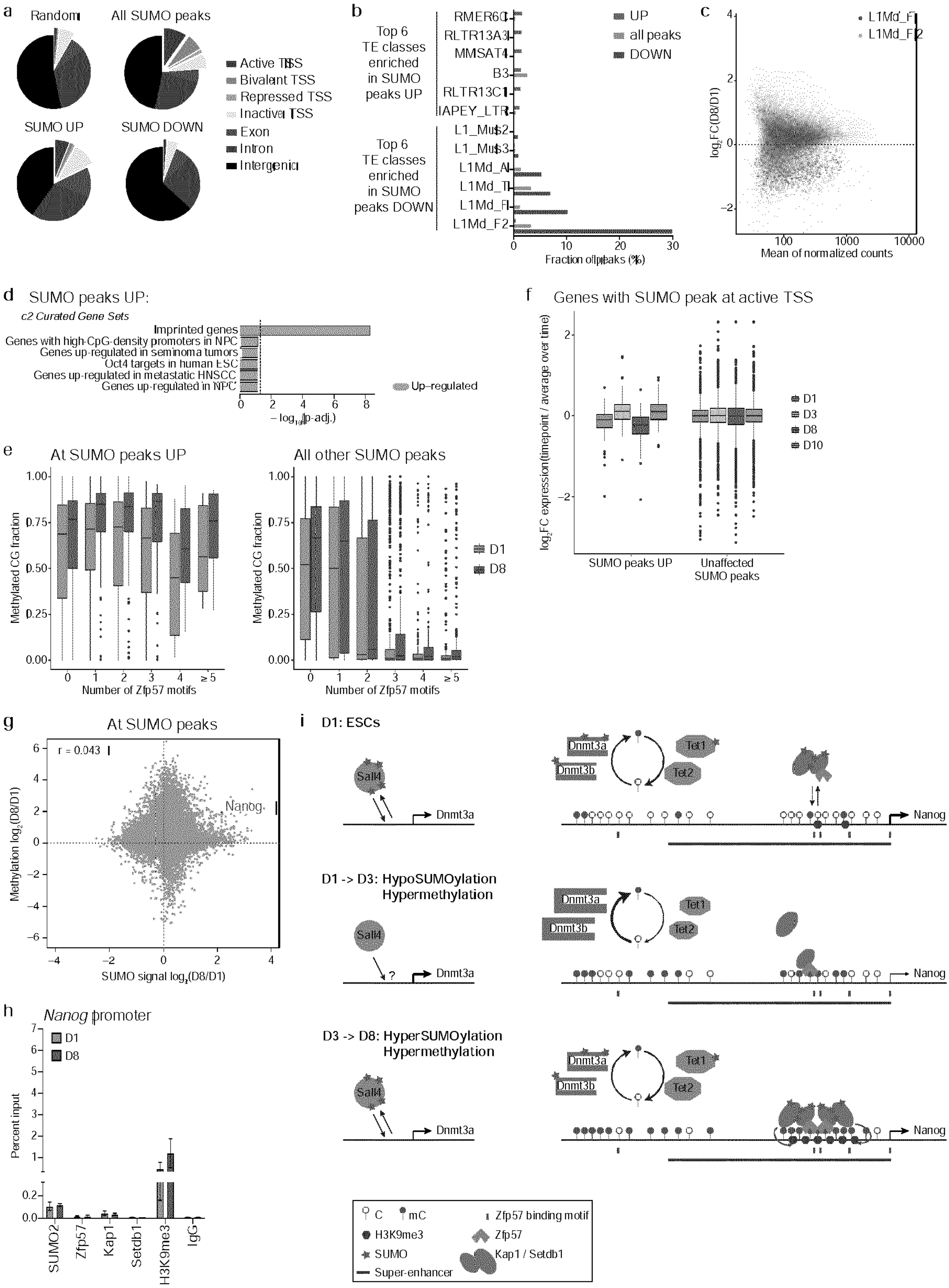

a
Random
All SUMO peaks
SUMO UP
SUMO DOWN
Active TSS
Bivalent TSS
Repressed TSS
Inactive TSS
Exon
Intron
Intergenic
b
Top 6 TE classes enriched in SUMO peaks UP
Top 6 TE classes enriched in SUMO peaks DOWN
RMER6C
RLTR13A3
MMSAT4
B3
RLTR13C1
IAPEY_LTR
L1_Mus2
L1_Mus3
L1Md_A
L1Md_T
L1Md_F
L1Md_F2
UP
all peaks
DOWN
Fraction of peaks (%)
c
L1Md_F
L1Md_F2
Mean of normalized counts
d
SUMO peaks UP:
c2 Curated Gene Sets
Imprinted genes
Genes with high-CpG-density promoters in NPC
Genes up-regulated in seminoma tumors
Oct4 targets in human ESC
Genes up-regulated in metastatic HNSCC
Genes up-regulated in NPC
Up-regulated
e
At SUMO peaks UP
All other SUMO peaks
Methylated CG fraction
Number of Zfp57 motifs
D1
D8
f
Genes with SUMO peak at active TSS
SUMO peaks UP
Unaffected SUMO peaks
D1
D3
D8
D10
g
At SUMO peaks
r = 0.043
Nanog
h
Nanog promoter
D1
D8
Percent input
SUMO2
Zfp57
Kap1
Setdb1
H3K9me3
IgG
i
D1: ESCs
D1 -> D3: HypoSUMOylation Hypermethylation
D3 -> D8: HyperSUMOylation Hypermethylation
Sall4
Dnmt3a
Dnmt3b
Tet1
Tet2
Nanog
C
mC
Zfp57 binding motif
H3K9me3
Zfp57
SUMO
Kap1 / Setdb1
Super-enhancer


Fig. 13

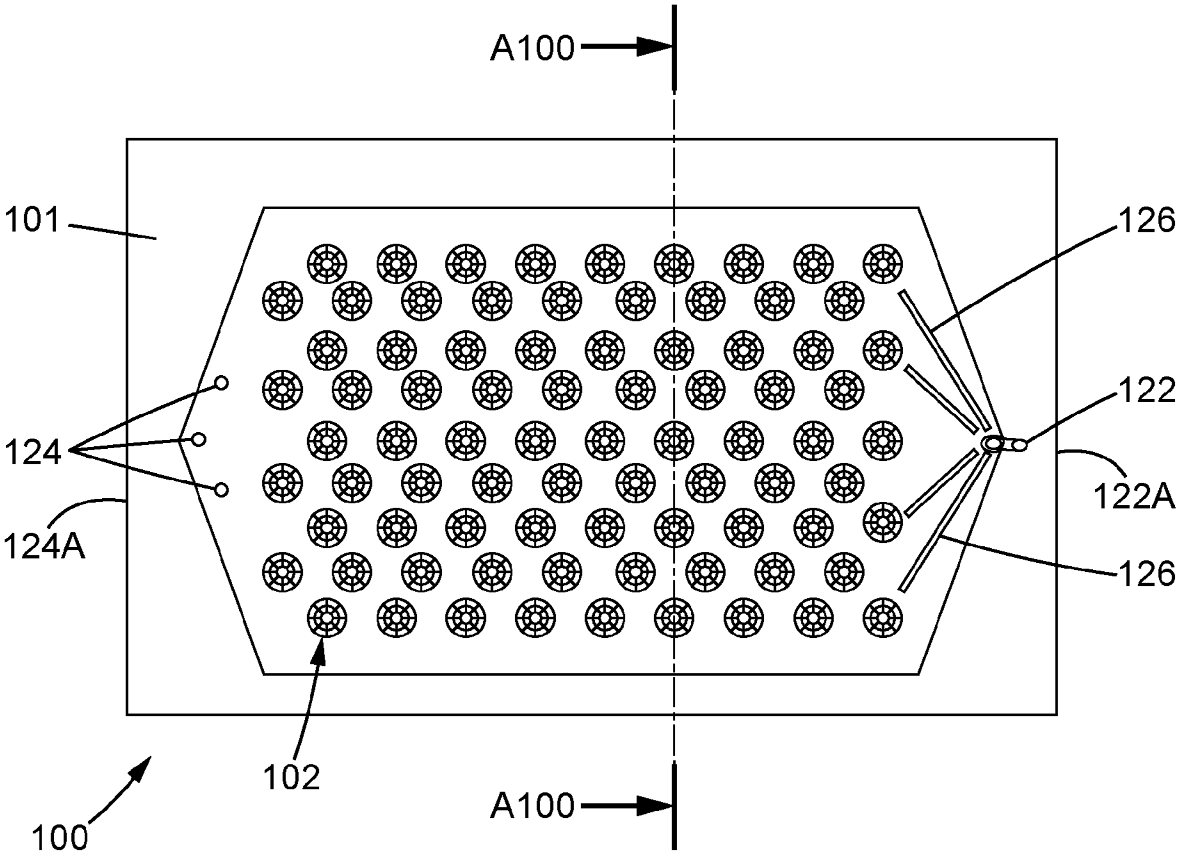
A100
101
126
122
124
122A
124A
126
102
A100
100

FIG. 14

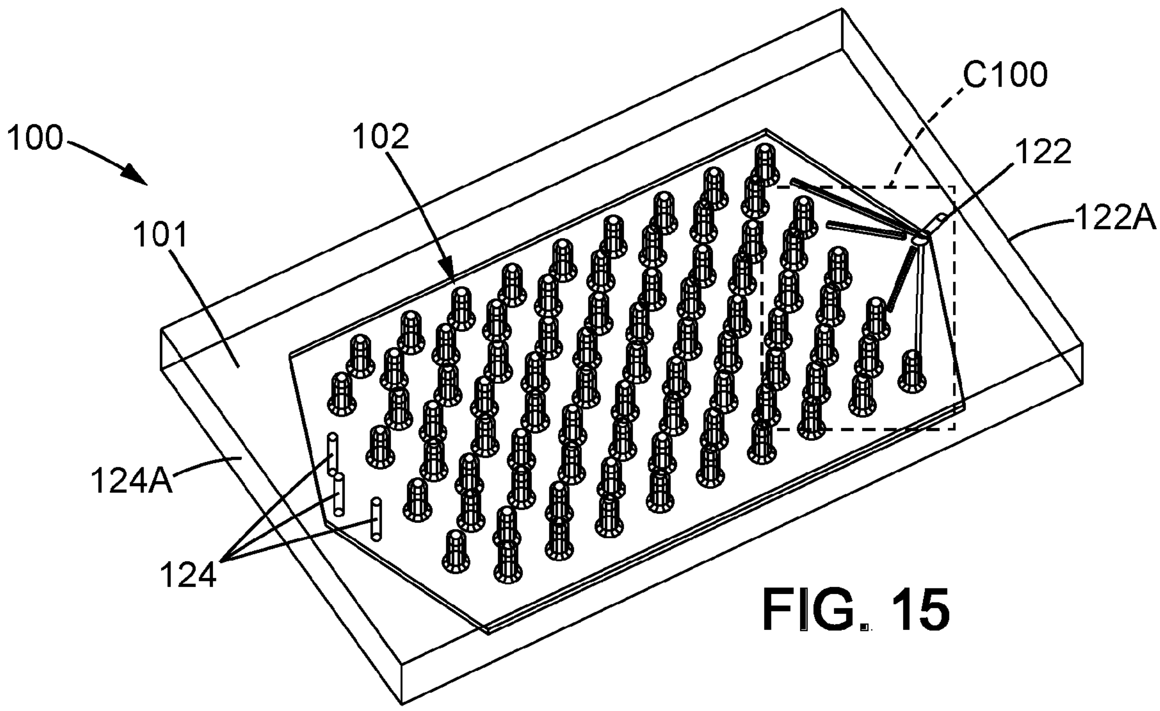
C100
100
102
122
101
122A
124A
124

FIG. 15

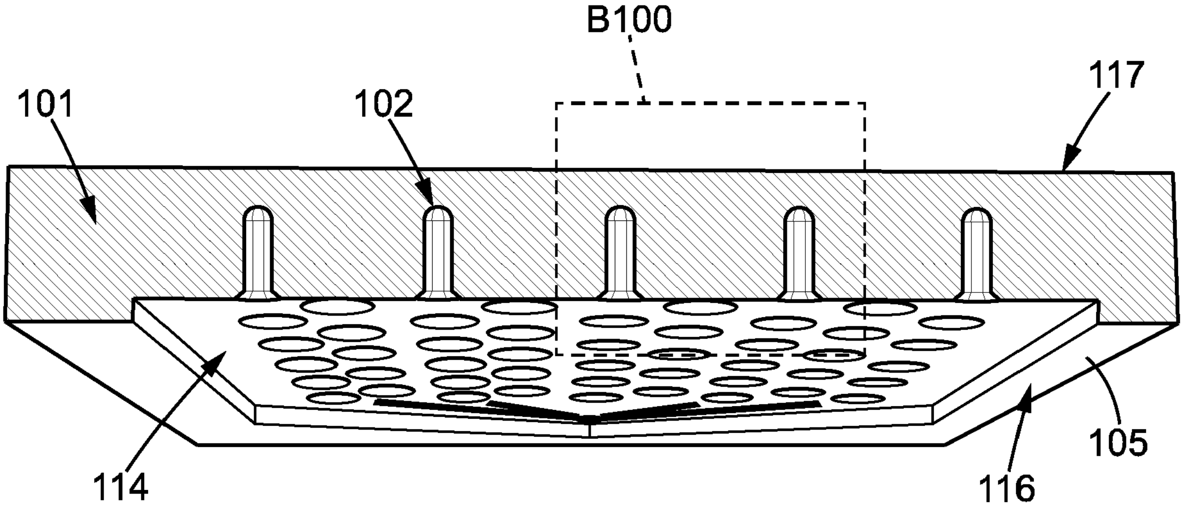
B100
101
102
117
114
116
105

FIG. 16

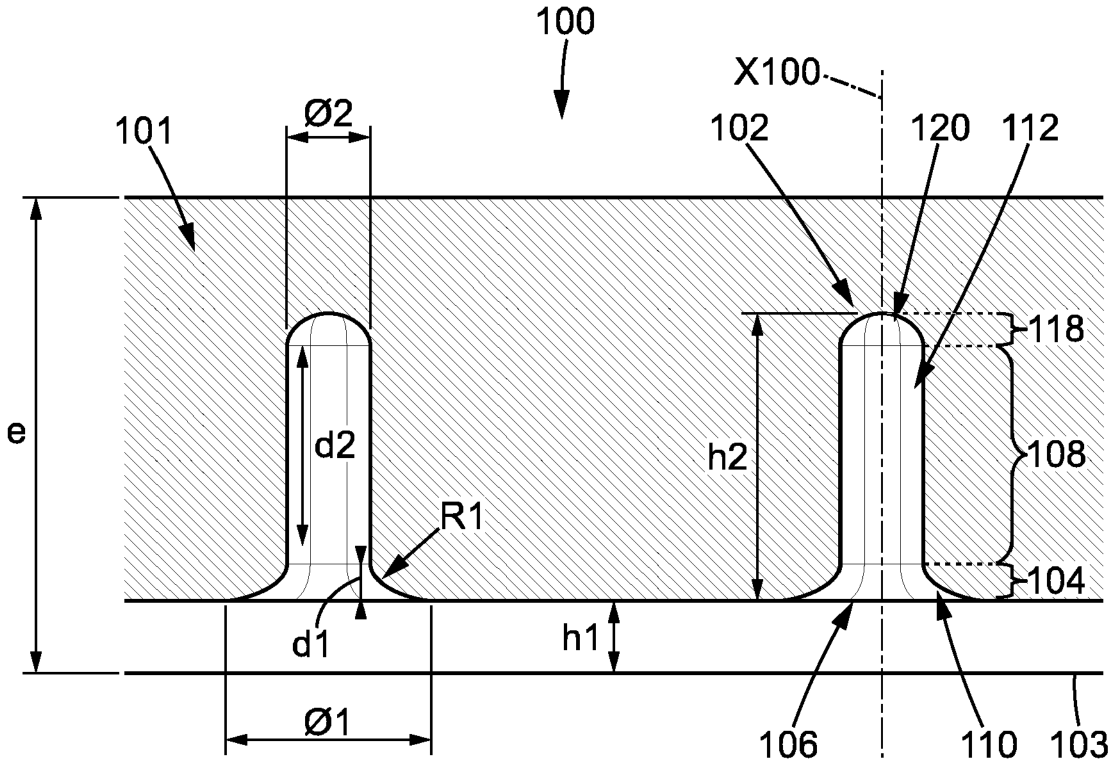
100
X100
101
Ø2
102
120
112
118
e
d2
h2
108
R1
104
d1
h1
Ø1
106
110
103

FIG. 17

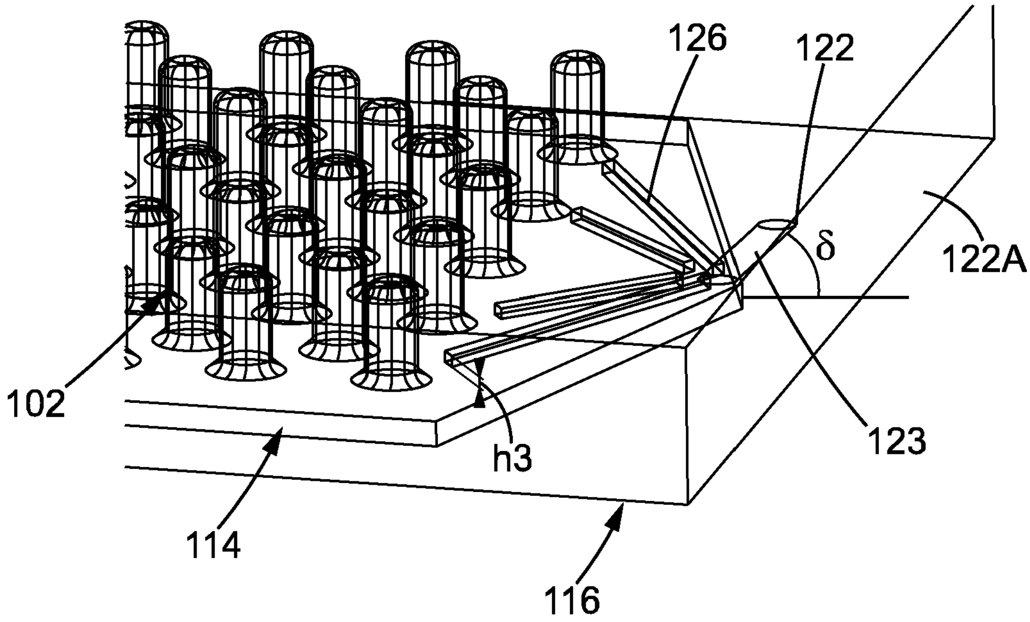
126
122
δ
122A
102
h3
123
114
116

FIG. 18

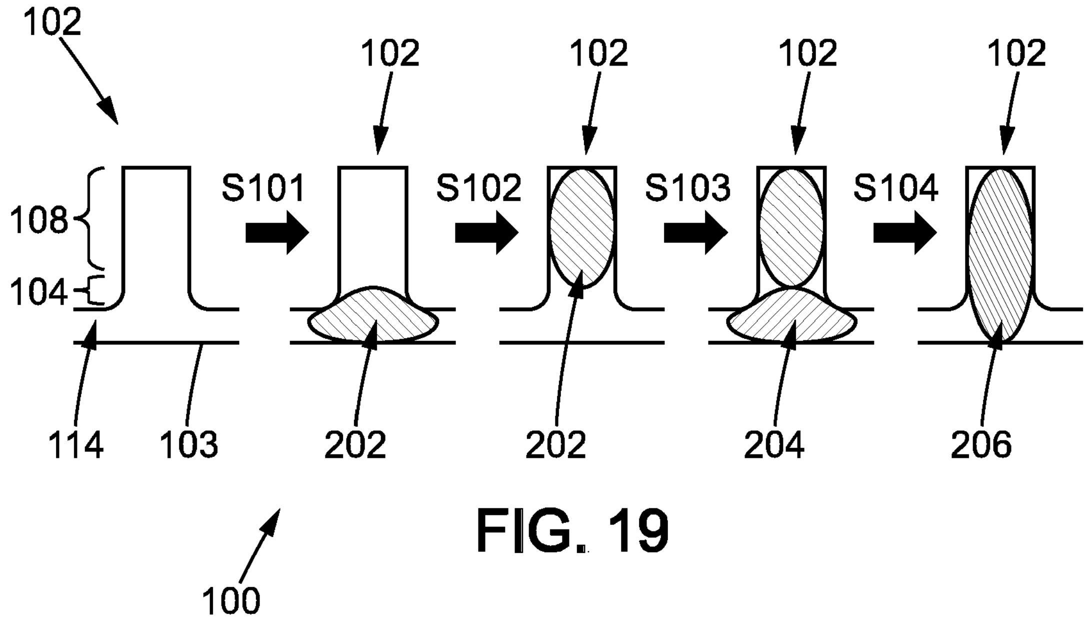
102
102
102
102
102
108
104
S101
S102
S103
S104
114
103
202
202
204
206
100

FIG. 19

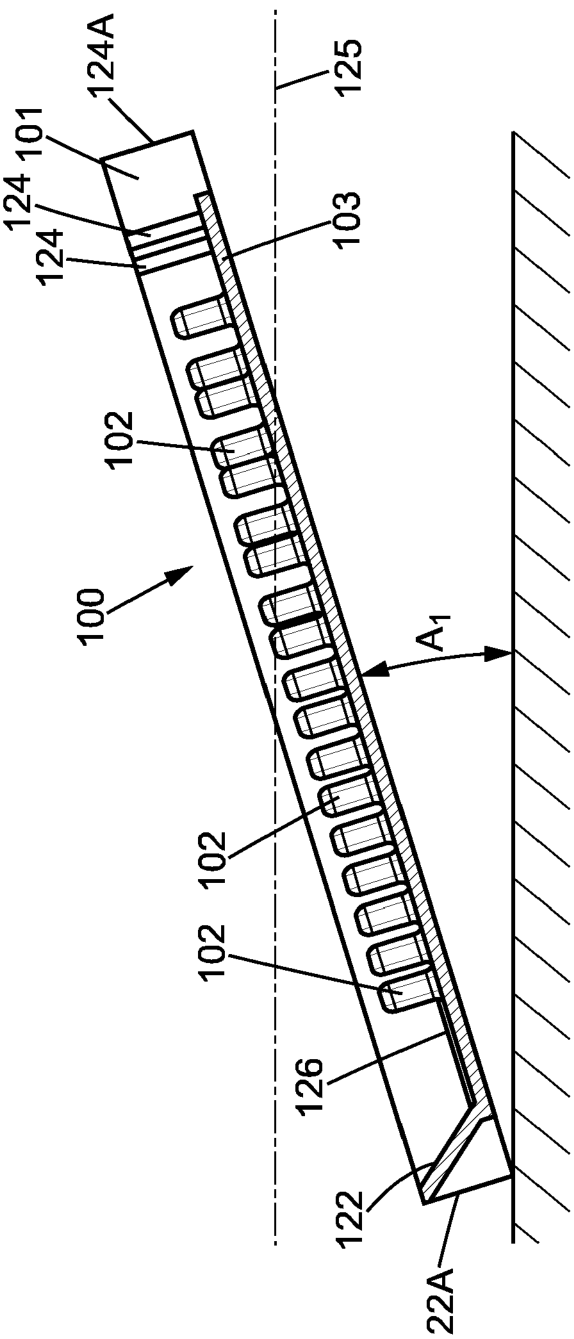


FIG. 20

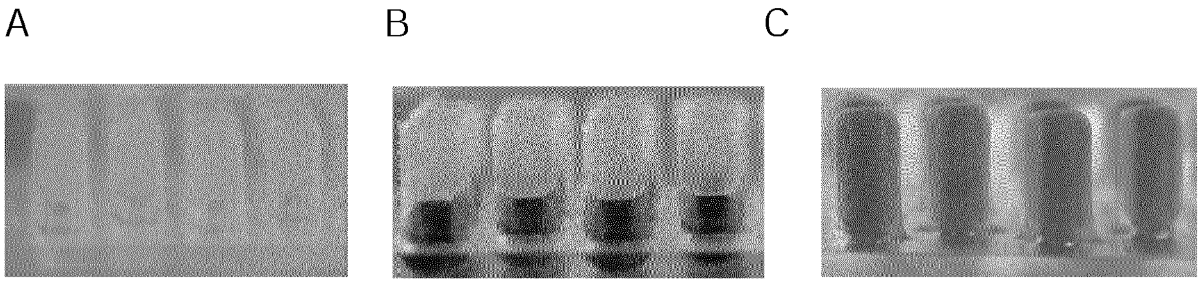


FIG. 21

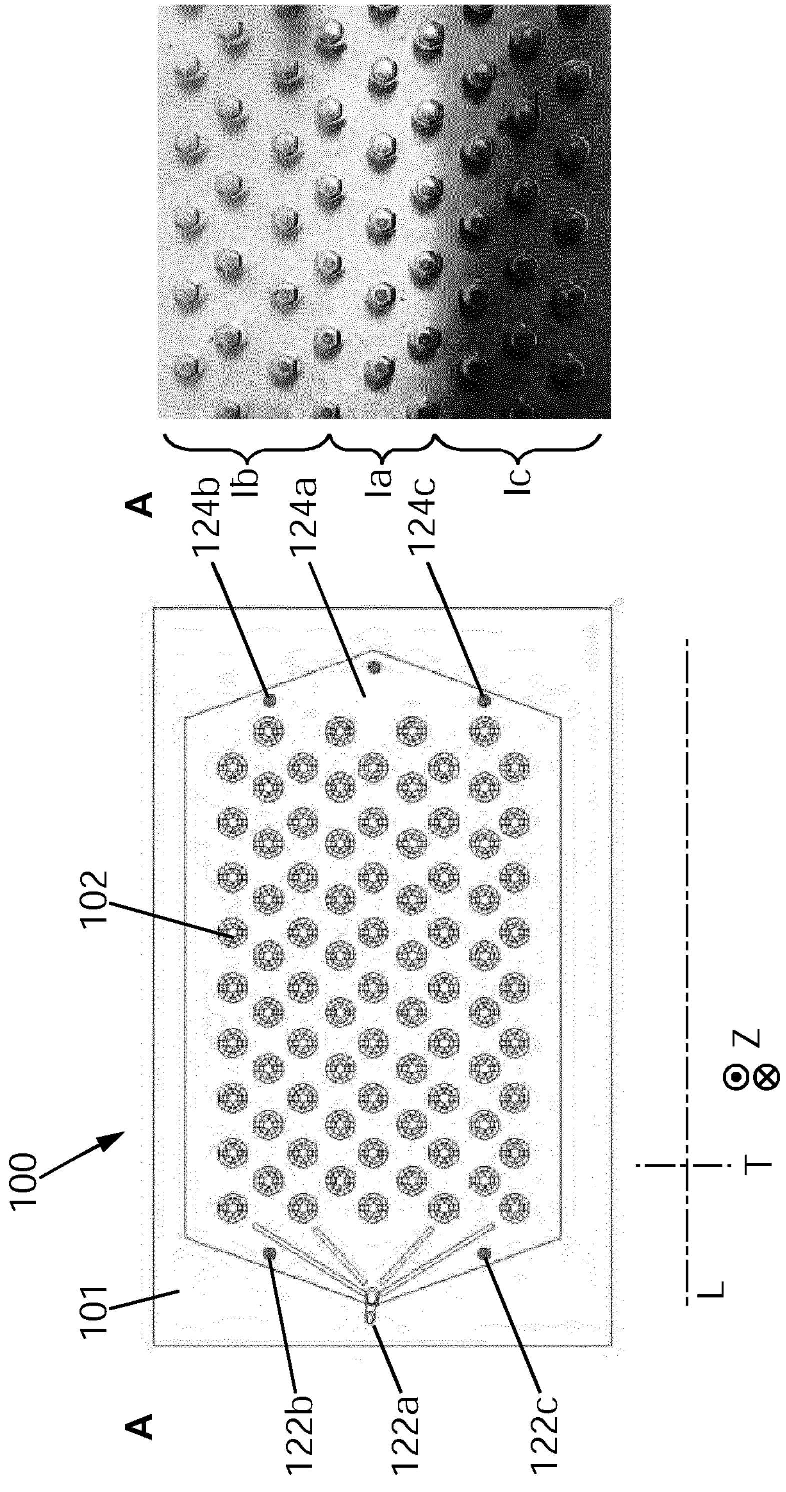


FIG. 22

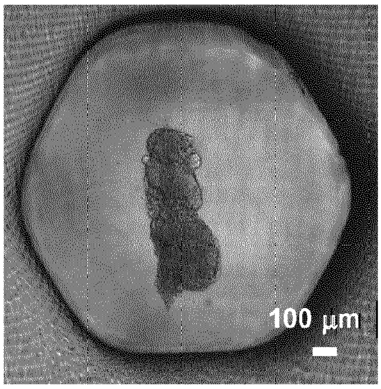


FIG. 23

Europäisches Patentamt
European Patent Office
Office européen des brevets

# EUROPEAN SEARCH REPORT

Application Number
EP 22 30 5110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/348120 A1 (MARTINEZ ARIAS ALFONSO [GB] ET AL) 11 November 2021 (2021-11-11) * paragraph [[0003]] - paragraph [[0005]]; claim 1; example 5 * ----- | 12,13 | INV. C12N5/0735 ADD. C12M3/00 |
| X,D | BECCARI LEONARDO ET AL: "Multi-axial self-organization properties of mouse embryonic stem cells into gastruloids", NATURE, NATURE PUBLISHING GROUP UK, LONDON, vol. 562, no. 7726, 1 October 2018 (2018-10-01), pages 272-276, XP036902738, ISSN: 0028-0836, DOI: 10.1038/S41586-018-0578-0 [retrieved on 2018-10-03] * page 272; figure 1 * ----- | 12,13 | |
| A | WO 2021/050430 A1 (UNIV MICHIGAN REGENTS [US]) 18 March 2021 (2021-03-18) * claims 1-25; figure 4 * ----- | 1-16,24 | |
| | | | TECHNICAL FIELDS SEARCHED (IPC) |
| X | YANG YING ET AL: "SUMO2, a small ubiquitin-like modifier, is essential for development of murine preimplantation embryos", THERIOGENOLOGY, LOS ALTOS, CA, US, vol. 166, 27 February 2021 (2021-02-27), pages 29-37, XP086528500, ISSN: 0093-691X, DOI: 10.1016/J.THERIOGENOLOGY.2021.01.019 [retrieved on 2021-02-27] * page 29; figure 1 * ----- -/-- | 12,13 | C12N C12M |

The present search report has been drawn up for all claims

| Place of search | Date of completion of the search | Examiner |
|---|---|---|
| Munich | 4 October 2022 | Paresce, Donata |

4

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 1 of 4

Europäisches Patentamt
European Patent Office
Office européen des brevets

# EUROPEAN SEARCH REPORT

Application Number
EP 22 30 5110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | COSSEC JACK-CHRISTOPHE ET AL: "SUMO Safeguards Somatic and Pluripotent Cell Identities by Enforcing Distinct Chromatin States", CELL STEM CELL, ELSEVIER, CELL PRESS, AMSTERDAM, NL, vol. 23, no. 5, 25 October 2018 (2018-10-25), page 742, XP085522340, ISSN: 1934-5909, DOI: 10.1016/J.STEM.2018.10.001 * page 75155 - page 755 * ----- | 1-15,24 | |
| A | ANTONIO URRUTIA GUSTAVO ET AL: "ZFP451-mediated SUMOylation of SATB2 drives embryonic stem cell differentiation", GENES & DEVELOPMENT, vol. 35, no. 15-16, 1 August 2021 (2021-08-01), pages 1142-1160, XP055943406, US ISSN: 0890-9369, DOI: 10.1101/gad.345843.120 Retrieved from the Internet: URL:http://genesdev.cshlp.org/content/35/15-16/1142.full.pdf#page=1&view=FitH> * the whole document * ----- | 1-15,24 | TECHNICAL FIELDS SEARCHED (IPC) |

-/--

The present search report has been drawn up for all claims

| Place of search | Date of completion of the search | Examiner |
|---|---|---|
| Munich | 4 October 2022 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01) 4

Europäisches Patentamt
European Patent Office
Office européen des brevets

# EUROPEAN SEARCH REPORT

Application Number
EP 22 30 5110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TALAMILLO ANA ET AL: "The role of SUMOylation during development", BIOCHEMICAL SOCIETY TRANSACTIONS, vol. 48, no. 2, 29 April 2020 (2020-04-29), pages 463-478, XP055943405, GB ISSN: 0300-5127, DOI: 10.1042/BST20190390 Retrieved from the Internet: URL:https://watermark.silverchair.com/bst-2019-0390c.pdf?token=AQECAHi208BE49Ooan9kkhW_Ercy7Dm3ZL_9Cf3qfKAc485ysgAAA-kwggPlBgkqhkiG9w0BBwagggPWMIID0gIBADCCA8sGCSqGSIb3DQEHATAeBglghkgBZQMEAS4wEQQMbxyBH3nOwnNAn6rsAgEQgIIDnAaon_qvhOKsmZ9Qh-icOmsiVZYqQdcibRDNOc8qnL7qQmeXPCuzUXNfXL518MuyWaUgYM4w_XueGrlIBZ7uD> * the whole document * | 1-15,24 | |
| X | WO 2021/004953 A1 (COMMISSARIAT ENERGIE ATOMIQUE [FR]; ECOLE POLYTECH [FR] ET AL.) 14 January 2021 (2021-01-14) * figures 1-2 * | 16-22 | TECHNICAL FIELDS SEARCHED (IPC) |
| X | US 2017/252744 A1 (BAROUD CHARLES [FR] ET AL) 7 September 2017 (2017-09-07) * figures 7-15 * | 16-24 | |
| X | US 2020/038867 A1 (BAROUD CHARLES [FR] ET AL) 6 February 2020 (2020-02-06) * figures 1-5, 22 * | 16-24 | |
| X | US 2017/199173 A1 (KONRY TANIA [US] ET AL) 13 July 2017 (2017-07-13) * claim 1; figure 1A * | 16 | |
| X | US 2020/224137 A1 (KUMACHEVA EUGENIA [CA] ET AL) 16 July 2020 (2020-07-16) * claims 36-42; figures 1-3 * | 16 | |

-/--

The present search report has been drawn up for all claims

| Place of search | Date of completion of the search | Examiner |
|---|---|---|
| Munich | 4 October 2022 | Paresce, Donata |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 4

Europäisches Patentamt
European Patent Office
Office européen des brevets

# EUROPEAN SEARCH REPORT

Application Number
EP 22 30 5110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LAURENT JÉRÉMIE ET AL: "Convergence of microengineering and cellular self-organization towards functional tissue manufacturing", NATURE BIOMEDICAL ENGINEERING, NATURE PUBLISHING GROUP UK, LONDON, vol. 1, no. 12, 1 December 2017 (2017-12-01), pages 939-956, XP036927861, DOI: 10.1038/S41551-017-0166-X [retrieved on 2017-12-12] * the whole document * ----- | 16-24 | |
| | | | TECHNICAL FIELDS SEARCHED (IPC) |

The present search report has been drawn up for all claims

| Place of search | Date of completion of the search | Examiner |
|---|---|---|
| Munich | 4 October 2022 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

4

Europäisches Patentamt
European Patent Office
Office européen des brevets

Application Number
EP 22 30 5110

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 22 30 5110

The Search Division considers that the present European patent application does not comply with the requirements of unity of invention and relates to several inventions or groups of inventions, namely:

1. claims: 1-15(completely); 24(partially)

   A method of in vitro preparing organized 3D cell structures of mammalian cells comprising culturing a population of pluripotent or multipotent vertebrate cells and performing two steps of hypoSUMOylation treatment.

   ---

2. claims: 16-23(completely); 24(partially)

   A microfluidic device and method of manipulating droplets

   ---

## ANNEX TO THE EUROPEAN SEARCH REPORT ON EUROPEAN PATENT APPLICATION NO. EP 22 30 5110

This annex lists the patent family members relating to the patent documents cited in the above-mentioned European search report. The members are as contained in the European Patent Office EDP file on
The European Patent Office is in no way liable for these particulars which are merely given for the purpose of information.

04-10-2022

| Patent document cited in search report | Publication date | Patent family member(s) | Publication date |
|---|---|---|---|
| US 2021348120 A1 | 11-11-2021 | EP 3853344 A1 | 28-07-2021 |
| | | US 2021348120 A1 | 11-11-2021 |
| | | WO 2020058732 A1 | 26-03-2020 |
| WO 2021050430 A1 | 18-03-2021 | EP 4041252 A1 | 17-08-2022 |
| | | WO 2021050430 A1 | 18-03-2021 |
| WO 2021004953 A1 | 14-01-2021 | CN 114375227 A | 19-04-2022 |
| | | EP 3993906 A1 | 11-05-2022 |
| | | FR 3098128 A1 | 08-01-2021 |
| | | JP 2022540813 A | 20-09-2022 |
| | | US 2022252519 A1 | 11-08-2022 |
| | | WO 2021004953 A1 | 14-01-2021 |
| US 2017252744 A1 | 07-09-2017 | CN 107109319 A | 29-08-2017 |
| | | EP 3206791 A1 | 23-08-2017 |
| | | ES 2856733 T3 | 28-09-2021 |
| | | JP 2017537772 A | 21-12-2017 |
| | | US 2017252744 A1 | 07-09-2017 |
| | | WO 2016059302 A1 | 21-04-2016 |
| US 2020038867 A1 | 06-02-2020 | CN 110035825 A | 19-07-2019 |
| | | EP 3519092 A1 | 07-08-2019 |
| | | FR 3056927 A1 | 06-04-2018 |
| | | JP 2019532805 A | 14-11-2019 |
| | | JP 2022136156 A | 15-09-2022 |
| | | US 2020038867 A1 | 06-02-2020 |
| | | WO 2018060471 A1 | 05-04-2018 |
| US 2017199173 A1 | 13-07-2017 | EP 3161052 A1 | 03-05-2017 |
| | | US 2017199173 A1 | 13-07-2017 |
| | | WO 2015200832 A1 | 30-12-2015 |
| US 2020224137 A1 | 16-07-2020 | CA 3069870 A1 | 17-01-2019 |
| | | CN 110997900 A | 10-04-2020 |
| | | EP 3652303 A1 | 20-05-2020 |
| | | US 2020224137 A1 | 16-07-2020 |
| | | WO 2019010587 A1 | 17-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS,* 1644342-14-2
- *CHEMICAL ABSTRACTS,* 144707-18-6
- **VENKATACHALAM et al.** *Ceramic International,* 2019
- **RIVRON, N. C. et al.** Blastocyst-like structures generated solely from stem cells. *Nature,* 2018, vol. 557, 106-111
- **SOZEN, B et al.** Self-assembly of embryonic and two extra-embryonic stem cell types into gastrulating embryo-like structures. *Nat Cell Biol,* 2018, vol. 20, 979-989
- **ZHANG, S. et al.** Implantation initiation of self-assembled embryo-like structures generated using three types of mouse blastocyst-derived stem cells. *Nat Commun,* 2019, vol. 10, 496
- **TURNER, D. A. et al.** Anteroposterior polarity and elongation in the absence of extraembryonic tissues and spatially localised signalling in Gastruloids , mammalian embryonic organoids. *Development dev.,* 2017, 150391
- **BECCARI, L. et al.** Multi-axial self-organization properties of mouse embryonic stem cells into gastruloids. *Nature,* 2018, vol. 562, 272-276
- **MORIS, N. et al.** An in vitro model of early anteroposterior organization during human development. *Nature,* 2020, vol. 582, 410-415
- **VAN DEN BRINK, S. C. et al.** Single-cell and spatial transcriptomics reveal somitogenesis in gastruloids. *Nature,* 2020, vol. 582, 405-409
- **VEENVLIET, J. V. et al.** Mouse embryonic stem cells self-organize into trunk-like structures with neural tube and somites. *Science,* 2020, vol. 370, eaba4937
- **LI, R. et al.** Generation of Blastocyst-like Structures from Mouse Embryonic and Adult Cell Cultures. *Cell,* 2019, vol. 179, 687-702.e18
- **YU, L. et al.** Blastocyst-like structures generated from human pluripotent stem cells. *Nature,* 2021, vol. 591, 620-626
- **LIU, X. et al.** Modelling human blastocysts by reprogramming fibroblasts into iBlastoids. *Nature,* 2021, vol. 591, 627-632
- **GIRGIN, M. U. ; BROGUIERE, N. ; MATTOLINI, L. ; LUTOLF, M. P.** Gastruloids generated without exogenous Wnt activation develop anterior neural tissues. *Stem Cell Reports,* 2021, vol. 16, 1143-1155
- **ZHENG, Y. et al.** Controlled modelling of human epiblast and amnion development using stem cells. *Nature,* 2019, vol. 573, 421-425
- **CUBENAS-POTTS, C. ; MATUNIS, M. J.** SUMO: a multifaceted modifier of chromatin structure and function. *Dev Cell,* 2013, vol. 24, 1-12
- **HENDRIKS, I. A. ; VERTEGAAL, A. C.** A comprehensive compilation of SUMO proteomics. *Nat Rev Mol Cell Biol,* 2016
- **THEURILLAT, I. et al.** Extensive SUMO Modification of Repressive Chromatin Factors Distinguishes Pluripotent from Somatic Cells. *Cell Reports,* 2020, vol. 32, 108146
- **LIU, H. W. et al.** Chromatin modification by SUMO-1 stimulates the promoters of translation machinery genes. *Nucleic Acids Res,* 2012, vol. 40, 10172-86
- **NEYRET-KAHN, H. et al.** Sumoylation at chromatin governs coordinated repression of a transcriptional program essential for cell growth and proliferation. *Genome Res,* 2013, vol. 23, 1563-1579
- **NISKANEN, E. A. et al.** Global SUMOylation on active chromatin is an acute heat stress response restricting transcription. *Genome Biol,* 2015, vol. 16, 153
- **SEIFERT, A. ; SCHOFIELD, P. ; BARTON, G. J. ; HAY, R. T.** Proteotoxic stress reprograms the chromatin landscape of SUMO modification. *Sci Signal,* 2015, vol. 8, rs7
- **CHELOUFI, S. et al.** The histone chaperone CAF-1 safeguards somatic cell identity. *Nature,* 2015, vol. 528, 218-24
- **BORKENT, M. et al.** A Serial shRNA Screen for Roadblocks to Reprogramming Identifies the Protein Modifier SUMO2. *Stem Cell Reports,* 2016, vol. 6, 704-16
- **COSSEC, J.-C. et al.** SUMO Safeguards Somatic and Pluripotent Cell Identities by Enforcing Distinct Chromatin States. *Cell Stem Cell,* 2018, vol. 23, 742-757.e8
- **HIGUCHI, C. ; YAMAMOTO, M. ; SHIN, S.-W. ; MIYAMOTO, K. ; MATSUMOTO, K.** Perturbation of maternal PIASy abundance disrupts zygotic genome activation and embryonic development via SUMOylation pathway. *Biology Open,* 2019
- **YAN, Y.-L. et al.** DPPA2/4 and SUMO E3 ligase PIAS4 opposingly regulate zygotic transcriptional program. *PLoS Biol,* 2019, vol. 17, e3000324
- **HE, X. et al.** Probing the roles of SUMOylation in cancer cell biology by using a selective SAE inhibitor. *Nat Chem Biol,* 2017, vol. 13, 1164-1171

- **MOHAMMED, H. et al.** Single-Cell Landscape of Transcriptional Heterogeneity and Cell Fate Decisions during Mouse Early Gastrulation. *Cell Rep,* 2017, vol. 20, 1215-1228
- **PIJUAN-SALA, B. et al.** A single-cell molecular map of mouse gastrulation and early organogenesis. *Nature,* 2019, vol. 566, 490-495
- The Acquisition of Cell Fate in Mouse Development. **GRAHAM, S. J. L. ; ZERNICKA-GOETZ, M.** Current Topics in Developmental Biology. Elsevier, 2016, vol. 117, 671-695
- **BEN-HAIM, N. et al.** The Nodal Precursor Acting via Activin Receptors Induces Mesoderm by Maintaining a Source of Its Convertases and BMP4. *Developmental Cell,* 2006, vol. 11, 313-323
- **SART, S. ; TOMASI, R. F.-X. ; AMSELEM, G. ; BAROUD, C. N.** Multiscale cytometry and regulation of 3D cell cultures on a chip. *Nat Commun,* 2017, vol. 8, 469
- **SART, S. et al.** Mapping the structure and biological functions within mesenchymal bodies using microfluidics. *Sci. Adv.,* 2020, vol. 6, eaaw7853
- **TOMASI, R. F.-X. ; SART, S. ; CHAMPETIER, T. ; BAROUD, C. N.** Individual Control and Quantification of 3D Spheroids in a High-Density Microfluidic Droplet Array. *Cell Reports,* 2020, vol. 31, 107670
- **CAO, J. et al.** The single-cell transcriptional landscape of mammalian organogenesis. *Nature,* 2019, vol. 566, 496-502
- **CHAL, J. ; POURQUIÉ, O.** Making muscle: skeletal myogenesis in vivo and in vitro. *Development,* 2017, vol. 144, 2104-2122
- **GOUTI, M. et al.** A Gene Regulatory Network Balances Neural and Mesoderm Specification during Vertebrate Trunk Development. *Developmental Cell,* 2017, vol. 41, 243-261.e7
- **LA MANNO, G. et al.** *Molecular architecture of the developing mouse brain,* 2020, http://biorxiv.org/lookup/doi/10.1101/2020.07.02.184051
- **SHI, Y. ; KIRWAN, P. ; SMITH, J. ; ROBINSON, H. P. C. ; LIVESEY, F. J.** Human cerebral cortex development from pluripotent stem cells to functional excitatory synapses. *Nat Neurosci,* 2012, vol. 15, 477-486
- **PENISSON, M. ; LADEWIG, J. ; BELVINDRAH, R. ; FRANCIS, F.** Genes and Mechanisms Involved in the Generation and Amplification of Basal Radial Glial Cells. *Front. Cell. Neurosci.,* 2019, vol. 13, 381
- **HERNANDEZ-MIRANDA, L. R. ; MÜLLER, T. ; BIRCHMEIER, C.** The dorsal spinal cord and hindbrain: From developmental mechanisms to functional circuits. *Developmental Biology,* 2017, vol. 432, 34-42
- **BRITSCH, S.** The transcription factor Sox10 is a key regulator of peripheral glial development. *Genes & Development,* 2001, vol. 15, 66-78
- **ECKERSLEY-MASLIN, M. et al.** Dppa2 and Dppa4 directly regulate the Dux-driven zygotic transcriptional program. *Genes Dev.,* 2019, vol. 33, 194-208
- **ZHANG, J. et al.** Sall4 modulates embryonic stem cell pluripotency and early embryonic development by the transcriptional regulation of Pou5f1. *Nat Cell Biol,* 2006, vol. 8, 1114-1123
- **LI, X. et al.** A Maternal-Zygotic Effect Gene, Zfp57, Maintains Both Maternal and Paternal Imprints. *Developmental Cell,* 2008, vol. 15, 547-557
- **IVANOV, A. V. et al.** PHD domain-mediated E3 ligase activity directs intramolecular sumoylation of an adjacent bromodomain required for gene silencing. *Mol Cell,* 2007, vol. 28, 823-37
- **HAYASHI, K. ; LOPES, S. M. C. DE S. ; TANG, F. ; SURANI, M. A.** Dynamic Equilibrium and Heterogeneity of Mouse Pluripotent Stem Cells with Distinct Functional and Epigenetic States. *Cell Stem Cell,* 2008, vol. 3, 391-401
- **TORRES-PADILLA, M.-E. ; CHAMBERS, I.** Transcription factor heterogeneity in pluripotent stem cells: a stochastic advantage. *Development,* 2014, vol. 141, 2173-2181
- **AGUILERA-CASTREJON, A. et al.** Ex utero mouse embryogenesis from pre-gastrulation to late organogenesis. *Nature,* 2021, vol. 593, 119-124
- **OGINUMA, M. et al.** Intracellular pH controls WNT downstream of glycolysis in amniote embryos. *Nature,* 2020, vol. 584, 98-101
- **CHAMBERS, I. et al.** Functional Expression Cloning of Nanog, a Pluripotency Sustaining Factor in Embryonic Stem Cells. *Cell,* 2003, vol. 113, 643-655
- **ZHANG, Y. et al.** Dynamic epigenomic landscapes during early lineage specification in mouse embryos. *Nat Genet,* 2018, vol. 50, 96-105
- **KLIMANSKAYA, I. et al.** Human embryonic stem cell lines derived from single blastomeres. *Nature,* 2006, vol. 444, 481-485
- **DELUZ, C. ; FRIMAN, E. T. ; STREBINGER, D. ; BENKE, A. ; RACCAUD, M. ; CALLEGARI, A. ; LELEU, M. ; MANLEY, S. ; SUTER, D. M.** A Role for Mitotic Bookmarking of SOX2 in Pluripotency and Differentiation. *Genes Dev,* 2016, vol. 30 (22), 2538-2550, https://doi.org/10.1101/gad.289256.116
- **ECKERSLEY-MASLIN, M. ; ALDA-CATALINAS, C. ; BLOTENBURG, M. ; KREIBICH, E. ; KRUEGER, C. ; REIK, W.** Dppa2 and Dppa4 Directly Regulate the Dux-Driven Zygotic Transcriptional Program. *Genes Dev.,* 2019, vol. 33 (3-4), 194-208, https://doi.org/10.1101/gad.321174.118

- **SEMRAU, S. ; GOLDMANN, J. E. ; SOUMILLON, M. ; MIKKELSEN, T. S. ; JAENISCH, R. ; VAN OUDENAARDEN, A.** Dynamics of Lineage Commitment Revealed by Single-Cell Transcriptomics of Differentiating Embryonic Stem Cells. *Nat Commun,* 2017, vol. 8 (1), 1096, https://doi.org/10.1038/s41467-017-01076-4
- **JAITIN, D. A. ; KENIGSBERG, E. ; KEREN-SHAUL, H. ; ELEFANT, N. ; PAUL, F. ; ZARETSKY, I. ; MILDNER, A. ; COHEN, N. ; JUNG, S. ; TANAY, A.** Massively Parallel Single-Cell RNA-Seq for Marker-Free Decomposition of Tissues into Cell Types. *Science,* 2014, vol. 343 (6172), 776-779, https://doi.org/10.1126/science.1247651
- **KEREN-SHAUL, H. ; KENIGSBERG, E. ; JAITIN, D. A. ; DAVID, E. ; PAUL, F. ; TANAY, A. ; AMIT, I.** MARS-Seq2.0: An Experimental and Analytical Pipeline for Indexed Sorting Combined with Single-Cell RNA Sequencing. *Nat Protoc,* 2019, vol. 14 (6), 1841-1862, https://doi.org/10.1038/s41596-019-0164-4
- **HAO, Y. ; HAO, S. ; ANDERSEN-NISSEN, E. ; MAUCK, W. M. ; ZHENG, S. ; BUTLER, A. ; LEE, M. J. ; WILK, A. J. ; DARBY, C. ; ZAGER, M.** Integrated Analysis of Multimodal Single-Cell Data. *Cell,* 2021, https://doi.org/10.1016/j.cell.2021.04.048
- **KORSUNSKY, I. ; MILLARD, N. ; FAN, J. ; SLOWIKOWSKI, K. ; ZHANG, F. ; WEI, K. ; BAGLAENKO, Y. ; BRENNER, M. ; LOH, P. ; RAYCHAUDHURI, S.** Fast, Sensitive and Accurate Integration of Single-Cell Data with Harmony. *Nat Methods,* 2019, vol. 16 (12), 1289-1296, https://doi.org/10.1038/s41592-019-0619-0
- **KRUEGER, F. ; ANDREWS, S. R.** Bismark: A Flexible Aligner and Methylation Caller for Bisulfite-Seq Applications. *Bioinformatics,* 2011, vol. 27 (11), 1571-1572, https://doi.org/10.1093/bioinformatics/btr167
- **LANGMEAD, B. ; SALZBERG, S. L.** Fast Gapped-Read Alignment with Bowtie 2. *Nat Methods,* 2012, vol. 9, 357-359, https://doi.org/10.1038/nmeth.1923
- **WHYTE, W. A. ; ORLANDO, D. A. ; HNISZ, D. ; ABRAHAM, B. J. ; LIN, C. Y. ; KAGEY, M. H. ; RAHL, P. B. ; LEE, T. I. ; YOUNG, R. A.** Master Transcription Factors and Mediator Establish Super-Enhancers at Key Cell Identity Genes. *Cell,* 2013, vol. 153 (2), 307-319, https://doi.org/10.1016/j.cell.2013.03.035
- **PINTACUDA, G. ; WEI, G. ; ROUSTAN, C. ; KIRMIZITAS, B. A. ; SOLCAN, N. ; CERASE, A. ; CASTELLO, A. ; MOHAMMED, S. ; MOINDROT, B. ; NESTEROVA, T. B.** HnRNPK Recruits PCGF3/5-PRC1 to the Xist RNA B-Repeat to Establish Polycomb-Mediated Chromosomal Silencing. *Molecular Cell,* 2017, vol. 68 (5), 955-969.e10, https://doi.org/10.1016/j.molcel.2017.11.013
- **LI, H. ; HANDSAKER, B. ; WYSOKER, A. ; FENNELL, T. ; RUAN, J. ; HOMER, N. ; MARTH, G. ; ABECASIS, G. ; DURBIN, R.** 1000 Genome Project Data Processing Subgroup. The Sequence Alignment/Map Format and SAMtools. *Bioinformatics,* 2009, vol. 25 (16), 2078-2079, https://doi.org/10.1093/bioinformatics/btp352
- **ORLANDO, D. A. ; CHEN, M. W. ; BROWN, V. E. ; SOLANKI, S. ; CHOI, Y. J. ; OLSON, E. R. ; FRITZ, C. C. ; BRADNER, J. E. ; GUENTHER, M. G.** Quantitative ChIP-Seq Normalization Reveals Global Modulation of the Epigenome. *Cell Reports,* 2014, vol. 9 (3), 1163-1170, https://doi.org/10.1016/j.celrep.2014.10.018
- **ZHANG, Y. ; LIU, T. ; MEYER, C. A. ; EECKHOUTE, J. ; JOHNSON, D. S. ; BERNSTEIN, B. E. ; NUSBAUM, C. ; MYERS, R. M. ; BROWN, M. ; LI, W.** Model-Based Analysis of ChIP-Seq (MACS). *Genome Biol,* 2008, vol. 9, R137, https://doi.org/10.1186/gb-2008-9-9-r137
- **LI, Q. ; BROWN, J. B. ; HUANG, H. ; BICKEL, P. J.** Measuring Reproducibility of High-Throughput Experiments. *The Annals of Applied Statistics,* 2011, vol. 5 (3), 1752-1779, https://doi.org/10.1214/11-AOAS466
- **QUINLAN, A. R. ; HALL, I. M.** BEDTools: A Flexible Suite of Utilities for Comparing Genomic Features. *Bioinformatics,* 2010, vol. 26 (6), 841-842, https://doi.org/10.1093/bioinformatics/btq033
- **LIAO, Y. ; SMYTH, G. K. ; SHI, W.** FeatureCounts: An Efficient General Purpose Program for Assigning Sequence Reads to Genomic Features. *Bioinformatics,* 2014, vol. 30 (7), 923-930, https://doi.org/10.1093/bioinformatics/btt656
- **LOVE, M. I. ; HUBER, W. ; ANDERS, S.** Moderated Estimation of Fold Change and Dispersion for RNA-Seq Data with DESeq2. *Genome Biol,* 2014, vol. 15, 550, https://doi.org/10.1186/s13059-014-0550-8
- **MACHANICK, P. ; BAILEY, T. L.** MEME-ChIP: Motif Analysis of Large DNA Datasets. *Bioinformatics,* 2011, vol. 27, 1696-1697, https://doi.org/10.1093/bioinformatics/btr189
- **ALHAMDOOSH, M. ; NG, M. ; WILSON, N. J. ; SHERIDAN, J. M. ; HUYNH, H. ; WILSON, M. J. ; RITCHIE, M. E.** Combining Multiple Tools Outperforms Individual Methods in Gene Set Enrichment Analyses. *Bioinformatics,* 2017, vol. 33 (3), 414-424, https://doi.org/10.1093/bioinformatics/btw623
- **LOVE, M. I. ; HUBER, W.; ; ANDERS, S.** Moderated Estimation of Fold Change and Dispersion for RNA-Seq Data with DESeq2. *Genome Biology,* 2014, vol. 15 (12), 550, https://doi.org/10.1186/s13059-014-0550-8